# EUROPEAN PATENT SPECIFICATION

(11) **EP 2 376 525 B1**
(45) Date of publication and mention of the grant of the patent: **03.05.2017**
(21) Application number: 09795419.2
(22) Date of filing: 17.12.2009
(51) Int. Cl.: C07K 14/025

(54) **IMMUNOGENIC POLYPEPTIDES COMPRISING A SCAFFOLD POLYPEPTIDE AND A L2 POLYPEPTIDE OR A FRAGMENT THEREOF**
EIN GERÜST-POLYPEPTID UND EIN L2-POLYPEPTID ODER EIN FRAGMENT DAVON UMFASSENDE IMMUNOGENE POLYPEPTIDE
POLYPEPTIDES IMMUNOGÉNÉTIQUES COMPORTANT UN POLYPEPTIDE RECOMBINANT ET POLYPEPTIDE L2 OU SON FRAGMENT

(30) Priority: 19.12.2008 EP 08172349
(43) Date of publication of application: 19.10.2011
(73) Proprietor: Deutsches Krebsforschungszentrum, 69120 Heidelberg (DE)
(72) Inventor: MUELLER, Martin, 69151 Neckargemuend (DE); RUBIO, Ivonne, 69117 Heidelberg (DE); TOMMASINO, Massimo, I-69390 Charly-Lyon (IT); OTTONELLO, Simone, I-43100 Parma (IT); BOLCHI, Angelo, I-43037 Lesignano de Bagni Parma (IT)
(74) Representative: Herzog, Fiesser & Partner Patentanwälte PartG mbB
(86) International application number: PCT/EP2009/067422
(87) International publication number: WO 2010/070052

(56) References cited:
- WO-A-02/070004
- WO-A-2008/140474
- WO-A-2009/001867
- GAMBHIRA RATISH ET AL: "A protective and broadly cross-neutralizing epitope of human papillomavirus L2" JOURNAL OF VIROLOGY, vol. 81, no. 24, December 2007 (2007-12), pages 13927-13931, XP002519253 ISSN: 0022-538X
- KONDO ET AL: "Neutralization of HPV16, 18, 31, and 58 pseudovirions with antisera induced by immunizing rabbits with synthetic peptides representing segments of the HPV16 minor capsid protein L2 surface region" VIROLOGY, ACADEMIC PRESS,ORLANDO, US, vol. 358, no. 2, 25 January 2007 (2007-01-25), pages 266-272, XP005853978 ISSN: 0042-6822
- DE JONG A ET AL: "Enhancement of human papillomavirus (HPV) type 16 E6 and E7-specific T-cell immunity in healthy volunteers through vaccination with TA-CIN, an HPV16 L2E7E6 fusion protein vaccine" VACCINE, BUTTERWORTH SCIENTIFIC. GUILDFORD, GB, vol. 20, no. 29-30, 4 October 2002 (2002-10-04), pages 3456-3464, XP004381809 ISSN: 0264-410X
- SKERRA ET AL: "Alternative non-antibody scaffolds for molecular recognition" CURRENT OPINION IN BIOTECHNOLOGY, LONDON, GB, vol. 18, no. 4, 14 September 2007 (2007-09-14), pages 295-304, XP022244962 ISSN: 0958-1669
- MAMBETISAEVA E T ET AL: "Expression of Three Functional Domains of Connexin 32 as Thioredoxin Fusion Proteins inEscherichia coliand Generation of Antibodies" PROTEIN EXPRESSION AND PURIFICATION, ACADEMIC PRESS, SAN DIEGO, CA, vol. 11, no. 1, 1 October 1997 (1997-10-01), pages 26-34, XP004451747 ISSN: 1046-5928
- MORETTO NADIA ET AL: "Conformation-sensitive antibodies against Alzheimer amyloid-beta by immunization with a thioredoxin-constrained B-cell epitope peptide" JOURNAL OF BIOLOGICAL CHEMISTRY, AMERICAN SOCIETY OF BIOLOCHEMICAL BIOLOGISTS, BIRMINGHAM,; US, vol. 282, no. 15, 1 April 2007 (2007-04-01), pages 11436-11445, XP002451806 ISSN: 0021-9258
- WOODMAN ET AL: "Design and Validation of a Neutral Protein Scaffold for the Presentation of Peptide Aptamers" JOURNAL OF MOLECULAR BIOLOGY, LONDON, GB, vol. 352, no. 5, 7 October 2005 (2005-10-07), pages 1118-1133, XP005074554 ISSN: 0022-2836
- RUBIO ET AL.: "Potent anti-HPV immune responses induced by tandem repeats of the HPV16 L2 (20-38) peptide displayed on bacterial thioredoxin" VACCINE, vol. 27, 31 January 2009 (2009-01-31), pages 1949-1956, XP002519254

### SEQUENCE LISTING

<110> DKFZ Deutsches Krebsforschungszentrum, Stiftung des öffentlichen Rechts
<120> Immunogenic polypeptides comprising a scaffold polypeptide and a L2 polypeptide or fragment
<130> DK65846PC
<160> 498
<170> PatentIn version 3.5
<210> 1
   <211> 120
   <212> PRT
   <213> Human papillomavirus type 16
<400> 1
   Met Arg His Lys Arg Ser Ala Lys Arg Thr Lys Arg Ala Ser Ala Thr
   1 5 10 15
   Gln Leu Tyr Lys Thr Cys Lys Gln Ala Gly Thr Cys Pro Pro Asp Ile
   20 25 30
   Ile Pro Lys Val Glu Gly Lys Thr Ile Ala Glu Gln Ile Leu Gln Tyr
   35 40 45
   Gly Ser Met Gly Val Phe Phe Gly Gly Leu Gly Ile Gly Thr Gly Ser
   50 55 60
   Gly Thr Gly Gly Arg Thr Gly Tyr Ile Pro Leu Gly Thr Arg Pro Pro
   65 70 75 80
   Thr Ala Thr Asp Thr Leu Ala Pro Val Arg Pro Pro Leu Thr Val Asp
   85 90 95
   Pro Val Gly Pro Ser Asp Pro Ser Ile Val Ser Leu Val Glu Glu Thr
   100 105 110
   Ser Phe Ile Asp Ala Gly Ala Pro
   115 120
<210> 2
   <211> 19
   <212> PRT
   <213> Human papillomavirus type 16
<400> 2
   Lys Thr Cys Lys Gln Ala Gly Thr Cys Pro Pro Asp Ile Ile Pro Lys
   1 5 10 15
   Val Glu Gly
<210> 3
   <211> 12
   <212> PRT
   <213> Human papillomavirus type 16
<400> 3
   Lys Thr Cys Lys Gln Ala Gly Thr Cys Pro Pro Asp
   1 5 10
<210> 4
   <211> 11
   <212> PRT
   <213> Human papillomavirus type 16
<400> 4
   Thr Cys Lys Gln Ala Gly Thr Cys Pro Pro Asp
   1 5 10
<210> 5
   <211> 10
   <212> PRT
   <213> Human papillomavirus type 16
<400> 5
   Cys Lys Gln Ala Gly Thr Cys Pro Pro Asp
   1 5 10
<210> 6
   <211> 10
   <212> PRT
   <213> Human papillomavirus type 16
<400> 6
   Thr Cys Lys Gln Ala Gly Thr Cys Pro Pro
   1 5 10
<210> 7
   <211> 9
   <212> PRT
   <213> Human papillomavirus type 16
<400> 7
   Cys Lys Gln Ala Gly Thr Cys Pro Pro
   1 5
<210> 8
   <211> 10
   <212> PRT
   <213> Human papillomavirus type 16
<400> 8
   Asp Ile Ile Pro Lys Val Glu Gly Lys Thr
   1 5 10
<210> 9
   <211> 9
   <212> PRT
   <213> Human papillomavirus type 16
<400> 9
   Thr Gly Tyr Ile Pro Leu Gly Thr Arg
   1 5
<210> 10
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 10
   Lys Thr Cys Lys Gln Xaa Gly Thr Cys Pro Pro Asp Ile Ile Pro Lys
   1               5                   10                  15
   Val Glu Gly
<210> 11
   <211> 12
   <212> PRT
   <213> artificial
<220>
   <223> L2 fragment
<220>
   <221> MISC_FEATURE
   <222> (6)..(6)
   <223> Xaa represents Gly or Ala
<400> 11
   Lys Thr Cys Lys Gln Xaa Gly Thr Cys Pro Pro Asp
   1               5                   10
<210> 12
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 12
   Thr Cys Lys Gln Xaa Gly Thr Cys Pro Pro Asp
   1               5                   10
<210> 13
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 13
   Cys Lys Gln Xaa Gly Thr Cys Pro Pro Asp
   1               5                   10
<210> 14
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 14
   Thr Cys Lys Gln Xaa Gly Thr Cys Pro Pro
   1               5                   10
<210> 15
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 15
   Cys Lys Gln Xaa Gly Thr Cys Pro Pro
   1               5
<210> 16
   <211> 3
   <212> PRT
   <213> artificial
<220>
   <223> linker peptide
<400> 16
   Gly Gly Pro
   1
<210> 17
   <211> 4
   <212> PRT
   <213> artificial
<220>
   <223> linker peptide
<400> 17
   Gly Pro Gly Pro
   1
<210> 18
   <211> 5
   <212> PRT
   <213> artificial
<220>
   <223> linker peptide
<400> 18
   Gly Pro Gly Pro Gly
   1 5
<210> 19
   <211> 4
   <212> PRT
   <213> artificial
<220>
   <223> linker peptide
<400> 19
   Ser Gly Ser Gly
   1
<210> 20
   <211> 63
   <212> PRT
   <213> artificial
<220>
   <223> Multimers with (GGP) linker
<400> 20
   Lys Thr Cys Lys Gln Ala Gly Thr Cys Pro Pro Asp Ile Ile Pro Lys
   1               5                   10                  15
   Val Glu Gly Gly Gly Pro Lys Thr Cys Lys Gln Ala Gly Thr Cys Pro
               20                  25                  30
   Pro Asp Ile Ile Pro Lys Val Glu Gly Gly Gly Pro Lys Thr Cys Lys
           35                  40                  45
   Gln Ala Gly Thr Cys Pro Pro Asp Ile Ile Pro Lys Val Glu Gly
       50                  55                  60
<210> 21
   <211> 195
   <212> PRT
   <213> artificial
<220>
   <223> Multimers with (GGP) linker
<400> 21
   Lys Thr Cys Lys Gln Ala Gly Thr Cys Pro Pro Asp Ile Ile Pro Lys
   1               5                   10                  15
   Val Glu Gly Gly Gly Pro Lys Thr Cys Lys Gln Ala Gly Thr Cys Pro
               20                  25                  30
   Pro Asp Ile Ile Pro Lys Val Glu Gly Gly Gly Pro Lys Thr Cys Lys
           35                  40                  45
   Gln Ala Gly Thr Cys Pro Pro Asp Ile Ile Pro Lys Val Glu Gly Gly
       50                  55                  60
   Gly Pro Lys Thr Cys Lys Gln Ala Gly Thr Cys Pro Pro Asp Ile Ile
   65 70 75 80
   Pro Lys Val Glu Gly Gly Gly Pro Lys Thr Cys Lys Gln Ala Gly Thr
   85 90 95
   Cys Pro Pro Asp Ile Ile Pro Lys Val Glu Gly Gly Gly Pro Lys Thr
   100 105 110
   Cys Lys Gln Ala Gly Thr Cys Pro Pro Asp Ile Ile Pro Lys Val Glu
   115 120 125
   Gly Gly Gly Pro Lys Thr Cys Lys Gln Ala Gly Thr Cys Pro Pro Asp
   130 135 140
   Ile Ile Pro Lys Val Glu Gly Gly Gly Pro Lys Thr Cys Lys Gln Ala
   145 150 155 160
   Gly Thr Cys Pro Pro Asp Ile Ile Pro Lys Val Glu Gly Gly Gly Pro
   165 170 175
   Lys Thr Cys Lys Gln Ala Gly Thr Cys Pro Pro Asp Ile Ile Pro Lys
   180 185 190
   Val Glu Gly
   195
<210> 22
   <211> 72
   <212> PRT
   <213> artificial
<220>
   <223> Multimers with (GGP) linker
<400> 22
   Lys Thr Cys Lys Gln Ala Gly Thr Cys Pro Pro Asp Gly Gly Pro Lys
   1 5 10 15
   Thr Cys Lys Gln Ala Gly Thr Cys Pro Pro Asp Gly Gly Pro Lys Thr
   20 25 30
   Cys Lys Gln Ala Gly Thr Cys Pro Pro Asp Gly Gly Pro Lys Thr Cys
   35 40 45
   Lys Gln Ala Gly Thr Cys Pro Pro Asp Gly Gly Pro Lys Thr Cys Lys
   50 55 60
   Gln Ala Gly Thr Cys Pro Pro Asp
   65 70
<210> 23
   <211> 4
   <212> PRT
   <213> artificial
<220>
   <223> Thioredoxin display site
<400> 23
   Cys Gly Pro Cys
   1
<210> 24
   <211> 109
   <212> PRT
   <213> Escherichia coli
<400> 24
   Met Ser Asp Lys Ile Ile His Leu Thr Asp Asp Ser Phe Asp Thr Asp
   1               5                   10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala
               100                 105
<210> 25
   <211> 109
   <212> PRT
   <213> Salmonella enterica
<400> 25
   Met Ser Asp Lys Ile Ile His Leu Thr Asp Asp Ser Phe Gly Thr Asp
   1               5                   10                  15
   Val Leu Lys Ala Asp Gly Ala Ile Leu Val Asp Phe Trp Ala Glu Trp
                   20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala
               100                 105
<210> 26
   <211> 105
   <212> PRT
   <213> Mus musculus
<400> 26
   Met Val Lys Leu Ile Glu Ser Lys Glu Ala Phe Gln Glu Ala Leu Ala
   1               5                   10                  15
   Ala Ala Gly Asp Lys Leu Val Val Val Asp Phe Ser Ala Thr Trp Cys
               20                  25                  30
   Gly Pro Cys Lys Met Ile Lys Pro Phe Phe His Ser Leu Cys Asp Lys
           35                  40                  45
   Tyr Ser Asn Val Val Phe Leu Glu Val Asp Val Asp Asp Cys Gln Asp
       50                  55                  60
   Val Ala Ala Asp Cys Glu Val Lys Cys Met Pro Thr Phe Gln Phe Tyr
   65                  70                  75                  80
   Lys Lys Gly Gln Lys Val Gly Glu Phe Ser Gly Ala Asn Lys Glu Lys
                   85                  90                  95
   Leu Glu Ala Ser Ile Thr Glu Tyr Ala
               100                 105
<210> 27
   <211> 105
   <212> PRT
   <213> Rattus norvegicus
<400> 27
   Met Val Lys Gln Ile Glu Ser Lys Ser Ala Phe Gln Glu Val Leu Asp
   1 5 10 15
   Ser Ala Gly Asp Lys Leu Val Val Val Asp Phe Ser Ala Thr Trp Cys
   20 25 30
   Gly Pro Cys Lys Met Ile Lys Pro Phe Phe His Ala Leu Ser Glu Lys
   35 40 45
   Phe Asn Asn Val Val Phe Ile Glu Val Asp Val Asp Asp Cys Lys Asp
   50 55 60
   Ile Ala Ala Glu Cys Glu Val Lys Cys Met Pro Thr Phe Gln Phe Phe
   65 70 75 80
   Lys Lys Gly Gln Lys Val Gly Glu Phe Ser Gly Ala Asn Lys Glu Lys
   85 90 95
   Leu Glu Ala Thr Ile Asn Glu Leu Leu
   100 105
<210> 28
   <211> 105
   <212> PRT
   <213> Homo sapiens
<400> 28
   Met Val Lys Gln Ile Glu Ser Lys Thr Ala Phe Gln Glu Ala Leu Asp
   1               5                   10                  15
   Ala Ala Gly Asp Lys Leu Val Val Val Asp Phe Ser Ala Thr Trp Cys
               20                  25                  30
   Gly Pro Cys Lys Met Ile Lys Pro Phe Phe His Ser Leu Ser Glu Lys
           35                  40                  45
   Tyr Ser Asn Val Ile Phe Leu Glu Val Asp Val Asp Asp Cys Gln Asp
       50                  55                  60
   Val Ala Ser Glu Cys Glu Val Lys Cys Met Pro Thr Phe Gln Phe Phe
   65                  70                  75                  80
   Lys Lys Gly Gln Lys Val Gly Glu Phe Ser Gly Ala Asn Lys Glu Lys
                   85                  90                  95
   Leu Glu Ala Thr Ile Asn Glu Leu Val
               100                 105
<210> 29
   <211> 216
   <212> PRT
   <213> artificial
<220>
   <223> immunogenic polypeptide
<400> 29
   Met Gly Ser Ser His His His His His His Ser Ser Gly Leu Val Pro
   1               5                   10                  15
   Arg Gly Ser His Met Gly Asp Lys Ile Ile His Leu Thr Asp Asp Ser
               20                  25                  30
   Phe Asp Thr Asp Val Leu Lys Ala Asp Gly Ala Ile Leu Val Asp Phe
           35                  40                  45
   Trp Ala Glu Trp Cys Gly Pro Lys Thr Cys Lys Gln Ala Gly Thr Cys
       50                  55                  60
   Pro Pro Asp Ile Ile Pro Lys Val Glu Gly Gly Gly Pro Lys Thr Cys
   65                  70                  75                  80
   Lys Gln Ala Gly Thr Cys Pro Pro Asp Ile Ile Pro Lys Val Glu Gly
                   85                  90                  95
   Gly Gly Pro Lys Thr Cys Lys Gln Ala Gly Thr Cys Pro Pro Asp Ile
               100                 105                 110
   Ile Pro Lys Val Glu Gly Gly Gly Pro Cys Lys Met Ile Ala Pro Ile
           115                 120                 125
   Leu Asp Glu Ile Ala Asp Glu Tyr Gln Gly Lys Leu Thr Val Ala Lys
       130                 135                 140
   Leu Asn Ile Asp Gln Asn Pro Gly Thr Ala Pro Lys Tyr Gly Ile Arg
   145                 150                 155                 160
   Gly Ile Pro Thr Leu Leu Leu Phe Lys Asn Gly Glu Val Ala Ala Thr
                   165                 170                 175
   Lys Val Gly Ala Leu Ser Lys Gly Gln Leu Lys Glu Phe Leu Asp Ala
               180                 185                 190
   Asn Leu Arg Asp Pro Asn Ser Ser Ser Val Asp Lys Leu Ala Ala Ala
           195                 200                 205
   Leu Glu His His His His His His
       210                 215
<210> 30
   <211> 348
   <212> PRT
   <213> artificial
<220>
   <223> immunogenic polypeptide
<400> 30
   Met Gly Ser Ser His His His His His His Ser Ser Gly Leu Val Pro
   1 5 10 15
   Arg Gly Ser His Met Gly Asp Lys Ile Ile His Leu Thr Asp Asp Ser
   20 25 30
   Phe Asp Thr Asp Val Leu Lys Ala Asp Gly Ala Ile Leu Val Asp Phe
   35 40 45
   Trp Ala Glu Trp Cys Gly Pro Lys Thr Cys Lys Gln Ala Gly Thr Cys
   50 55 60
   Pro Pro Asp Ile Ile Pro Lys Val Glu Gly Gly Gly Pro Lys Thr Cys
   65 70 75 80
   Lys Gln Ala Gly Thr Cys Pro Pro Asp Ile Ile Pro Lys Val Glu Gly
   85 90 95
   Gly Gly Pro Lys Thr Cys Lys Gln Ala Gly Thr Cys Pro Pro Asp Ile
   100 105 110
   Ile Pro Lys Val Glu Gly Gly Gly Pro Lys Thr Cys Lys Gln Ala Gly
   115 120 125
   Thr Cys Pro Pro Asp Ile Ile Pro Lys Val Glu Gly Gly Gly Pro Lys
   130 135 140
   Thr Cys Lys Gln Ala Gly Thr Cys Pro Pro Asp Ile Ile Pro Lys Val
   145 150 155 160
   Glu Gly Gly Gly Pro Lys Thr Cys Lys Gln Ala Gly Thr Cys Pro Pro
   165 170 175
   Asp Ile Ile Pro Lys Val Glu Gly Gly Gly Pro Lys Thr Cys Lys Gln
   180 185 190
   Ala Gly Thr Cys Pro Pro Asp Ile Ile Pro Lys Val Glu Gly Gly Gly
   195 200 205
   Pro Lys Thr Cys Lys Gln Ala Gly Thr Cys Pro Pro Asp Ile Ile Pro
   210 215 220
   Lys Val Glu Gly Gly Gly Pro Lys Thr Cys Lys Gln Ala Gly Thr Cys
   225 230 235 240
   Pro Pro Asp Ile Ile Pro Lys Val Glu Gly Gly Gly Pro Cys Lys Met
   245 250 255
   Ile Ala Pro Ile Leu Asp Glu Ile Ala Asp Glu Tyr Gln Gly Lys Leu
   260 265 270
   Thr Val Ala Lys Leu Asn Ile Asp Gln Asn Pro Gly Thr Ala Pro Lys
   275 280 285
   Tyr Gly Ile Arg Gly Ile Pro Thr Leu Leu Leu Phe Lys Asn Gly Glu
   290 295 300
   Val Ala Ala Thr Lys Val Gly Ala Leu Ser Lys Gly Gln Leu Lys Glu
   305 310 315 320
   Phe Leu Asp Ala Asn Leu Arg Asp Pro Asn Ser Ser Ser Val Asp Lys
   325 330 335
   Leu Ala Ala Ala Leu Glu His His His His His His
   340 345
<210> 31
   <211> 9
   <212> PRT
   <213> Human papillomavirus type 16
<400> 31
   Ile Ile Pro Lys Val Glu Gly Lys Thr
   1 5
<210> 32
   <211> 8
   <212> PRT
   <213> Human papillomavirus type 16
<400> 32
   Ile Pro Lys Val Glu Gly Lys Thr
   1 5
<210> 33
   <211> 19
   <212> PRT
   <213> artificial
<220>
   <223> L2 fragment
<400> 33
   Arg Gly Cys Lys Gln Ala Gly Thr Cys Pro Pro Asp Val Ile Asn Lys
   1               5                   10                  15
   Val Glu Gln
<210> 34
   <211> 19
   <212> PRT
   <213> artificial
<220>
   <223> L2 fragment
<400> 34
   Arg Gly Cys Lys Ala Ser Asn Thr Cys Pro Pro Asp Val Ile Asn Lys
   1               5                   10                  15
   Val Glu Gln
<210> 35
   <211> 19
   <212> PRT
   <213> artificial
<220>
   <223> L2 fragment
<400> 35
   Arg Gly Cys Lys Ala Ala Gly Thr Cys Pro Pro Asp Val Ile Asn Lys
   1               5                   10                  15
   Val Glu Gln
<210> 36
   <211> 19
   <212> PRT
   <213> artificial
<220>
   <223> L2 fragment
<400> 36
   Gln Ser Cys Lys Ala Ala Gly Thr Cys Pro Pro Asp Val Leu Asn Lys
   1 5 10 15
   Val Glu Gln
<210> 37
   <211> 19
   <212> PRT
   <213> artificial
<220>
   <223> L2 fragment
<400> 37
   Gln Ser Cys Lys Ala Ala Gly Thr Cys Pro Pro Asp Val Val Asn Lys
   1 5 10 15
   Val Glu Gln
<210> 38
   <211> 19
   <212> PRT
   <213> artificial
<220>
   <223> L2 fragment
<400> 38
   Gln Thr Cys Lys Gln Ala Gly Thr Cys Pro Pro Asp Val Ile Asn Lys
   1 5 10 15
   Val Glu Gln
<210> 39
   <211> 19
   <212> PRT
   <213> artificial
<220>
   <223> L2 fragment
<400> 39
   Gln Thr Cys Lys Gln Ala Gly Thr Cys Pro Pro Asp Val Ile Asn Lys
   1 5 10 15
   Val Glu Gln
<210> 40
   <211> 19
   <212> PRT
   <213> artificial
<220>
   <223> L2 fragment
<400> 40
   Arg Thr Cys Lys Gln Ala Gly Thr Cys Pro Pro Asp Val Ile Asn Lys
   1               5                   10                  15
   Val Glu Ser
<210> 41
   <211> 19
   <212> PRT
   <213> artificial
<220>
   <223> L2 fragment
<400> 41
   Arg Thr Cys Lys Gln Ala Gly Thr Cys Pro Pro Asp Val Ile Asn Lys
   1               5                   10                  15
   Val Glu Gln
<210> 42
   <211> 19
   <212> PRT
   <213> artificial
<220>
   <223> L2 fragment
<400> 42
   Lys Gly Cys Lys Ala Ser Gly Thr Cys Pro Pro Asp Val Ile Asn Lys
   1               5                   10                  15
   Val Glu Gln
<210> 43
   <211> 19
   <212> PRT
   <213> artificial
<220>
   <223> L2 fragment
<400> 43
   Arg Thr Cys Lys Gln Ser Gly Thr Cys Pro Pro Asp Val Val Pro Lys
   1               5                   10                  15
   Val Glu Gly
<210> 44
   <211> 19
   <212> PRT
   <213> artificial
<220>
   <223> L2 fragment
<400> 44
   Arg Thr Cys Lys Gln Ala Gly Thr Cys Pro Pro Asp Val Ile Pro Lys
   1               5                   10                  15
   Val Glu Gly
<210> 45
   <211> 19
   <212> PRT
   <213> artificial
<220>
   <223> L2 fragment
<400> 45
   Arg Thr Cys Lys Val Thr Gly Thr Cys Pro Ala Asp Val Val Pro Lys
   1               5                   10                  15
   Val Glu Gly
<210> 46
   <211> 19
   <212> PRT
   <213> artificial
<220>
   <223> L2 fragment
<400> 46
   Arg Thr Cys Lys Ala Thr Gly Thr Arg Pro Ala Asp Val Ile Pro Lys
   1               5                   10                  15
   Val Glu Gly
<210> 47
   <211> 19
   <212> PRT
   <213> artificial
<220>
   <223> L2 fragment
<400> 47
   Arg Thr Cys Lys Gln Ser Gly Thr Cys Pro Pro Asp Ile Ile Pro Arg
   1               5                   10                  15
   Val Glu Gln
<210> 48
   <211> 19
   <212> PRT
   <213> artificial
<220>
   <223> L2 fragment
<400> 48
   Arg Thr Cys Lys Gln Ala Gly Thr Cys Pro Pro Asp Ile Ile Pro Arg
   1               5                   10                  15
   Leu Glu Gln
<210> 49
   <211> 19
   <212> PRT
   <213> artificial
<220>
   <223> L2 fragment
<400> 49
   Arg Thr Cys Lys Gln Ala Gly Thr Cys Pro Pro Asp Ile Ile Pro Arg
   1               5                   10                  15
   Val Glu Gln
<210> 50
   <211> 19
   <212> PRT
   <213> artificial
<220>
   <223> L2 fragment
<400> 50
   Lys Thr Cys Lys Val Ala Gly Thr Cys Pro Pro Asp Val Ile Pro Lys
   1               5                   10                  15
   Val Glu Gly
<210> 51
   <211> 19
   <212> PRT
   <213> artificial
<220>
   <223> L2 fragment
<400> 51
   Lys Thr Cys Lys Ala Ala Gly Thr Cys Pro Pro Asp Val Ile Pro Lys
   1               5                   10                  15
   Val Glu Gly
<210> 52
   <211> 19
   <212> PRT
   <213> artificial
<220>
   <223> L2 fragment
<400> 52
   Arg Thr Cys Lys Ala Ala Gly Thr Cys Pro Pro Asp Val Ile Pro Lys
   1               5                   10                  15
   Val Glu Gly
<210> 53
   <211> 19
   <212> PRT
   <213> artificial
<220>
   <223> L2 fragment
<400> 53
   Arg Thr Cys Lys Ala Ser Gly Thr Cys Pro Pro Asp Val Ile Pro Lys
   1               5                   10                  15
   Val Glu Gly
<210> 54
   <211> 19
   <212> PRT
   <213> artificial
<220>
   <223> L2 fragment
<400> 54
   Ser Thr Cys Lys Ala Ala Gly Thr Cys Pro Ala Asp Val Ile Pro Lys
   1               5                   10                  15
   Val Glu Gly
<210> 55
   <211> 19
   <212> PRT
   <213> artificial
<220>
   <223> L2 fragment
<400> 55
   Lys Thr Cys Lys Leu Ser Gly Thr Cys Pro Glu Asp Val Ile Asn Lys
   1               5                   10                  15
   Val Glu Gln
<210> 56
   <211> 19
   <212> PRT
   <213> artificial
<220>
   <223> L2 fragment
<400> 56
   Lys Thr Cys Lys Gln Ser Gly Thr Cys Pro Pro Asp Ile Ile Pro Lys
   1               5                   10                  15
   Val Glu Gly
<210> 57
   <211> 19
   <212> PRT
   <213> artificial
<220>
   <223> L2 fragment
<400> 57
   Lys Thr Cys Lys Gln Ala Gly Thr Cys Pro Pro Asp Ile Val Pro Lys
   1               5                   10                  15
   Val Glu Gly
<210> 58
   <211> 19
   <212> PRT
   <213> artificial
<220>
   <223> L2 fragment
<400> 58
   Gln Thr Cys Lys Ala Ser Gly Thr Cys Pro Pro Asp Val Ile Pro Lys
   1               5                   10                  15
   Val Glu Gly
<210> 59
   <211> 19
   <212> PRT
   <213> artificial
<220>
   <223> L2 fragment
<400> 59
   Lys Thr Cys Lys Gln Ala Gly Thr Cys Pro Pro Asp Val Ile Pro Lys
   1               5                   10                  15
   Val Glu Gly
<210> 60
   <211> 19
   <212> PRT
   <213> artificial
<220>
   <223> L2 fragment
<400> 60
   Gln Thr Cys Lys Ala Ala Gly Thr Cys Pro Ser Asp Ile Ile Pro Lys
   1               5                   10                  15
   Val Glu His
<210> 61
   <211> 19
   <212> PRT
   <213> artificial
<220>
   <223> L2 fragment
<400> 61
   Gln Thr Cys Lys Ala Ser Gly Thr Cys Pro Pro Asp Val Ile Pro Lys
   1               5                   10                  15
   Val Glu Gln
<210> 62
   <211> 19
   <212> PRT
   <213> artificial
<220>
   <223> L2 fragment
<400> 62
   Gln Thr Cys Lys Leu Thr Gly Thr Cys Pro Pro Asp Val Ile Pro Lys
   1 5 10 15
   Val Glu His
<210> 63
   <211> 19
   <212> PRT
   <213> artificial
<220>
   <223> L2 fragment
<400> 63
   Gln Thr Cys Lys Ala Ala Gly Thr Cys Pro Ser Asp Val Ile Asn Lys
   1 5 10 15
   Val Glu His
<210> 64
   <211> 19
   <212> PRT
   <213> artificial
<220>
   <223> L2 fragment
<400> 64
   Lys Gln Cys Gln Leu Gly Ala Asp Cys Pro Pro Asp Val Arg Asn Lys
   1 5 10 15
   Val Glu Gly
<210> 65
   <211> 19
   <212> PRT
   <213> artificial
<220>
   <223> L2 fragment
<400> 65
   Ala Lys Cys Gln Leu Ser Gly Asn Cys Leu Pro Asp Val Lys Asn Lys
   1 5 10 15
   Val Glu Ala
<210> 66
   <211> 19
   <212> PRT
   <213> artificial
<220>
   <223> L2 fragment
<400> 66
   Ala Lys Cys Gln Leu Ser Gly Asp Cys Leu Pro Asp Val Lys Asn Lys
   1               5                   10                  15
   Val Glu Ala
<210> 67
   <211> 19
   <212> PRT
   <213> artificial
<220>
   <223> L2 fragment
<400> 67
   Arg His Cys Ala Leu Ser Gly Thr Cys Pro Asp Asp Val Lys Asn Lys
   1               5                   10                  15
   Val Glu Asn
<210> 68
   <211> 19
   <212> PRT
   <213> artificial
<220>
   <223> L2 fragment
<400> 68
   Lys His Cys Ala Gly Ser Gly Thr Cys Pro Glu Asp Val Lys Asn Lys
   1               5                   10                  15
   Val Glu Gln
<210> 69
   <211> 19
   <212> PRT
   <213> artificial
<220>
   <223> L2 fragment
<400> 69
   Lys Thr Cys Leu Gln Gly Gly Asp Cys Ile Pro Asp Val Lys Asn Lys
   1 5 10 15
   Phe Glu Asn
<210> 70
   <211> 19
   <212> PRT
   <213> artificial
<220>
   <223> L2 fragment
<400> 70
   Arg Ser Cys Leu Gln Gly Gly Asp Cys Ile Pro Asp Val Gln Asn Lys
   1 5 10 15
   Phe Glu Gly
<210> 71
   <211> 19
   <212> PRT
   <213> artificial
<220>
   <223> L2 fragment
<400> 71
   Gln Thr Cys Lys Ala Thr Gly Thr Cys Pro Pro Asp Val Ile Pro Lys
   1 5 10 15
   Val Glu Gly
<210> 72
   <211> 19
   <212> PRT
   <213> artificial
<220>
   <223> L2 fragment
<400> 72
   Lys Thr Cys Lys Gln Ser Gly Thr Cys Pro Pro Asp Val Val Pro Lys
   1 5 10 15
   Val Glu Gly
<210> 73
   <211> 19
   <212> PRT
   <213> artificial
<220>
   <223> L2 fragment
<400> 73
   Arg Thr Cys Lys Gln Ser Gly Thr Cys Pro Pro Asp Val Ile Asn Lys
   1               5                   10                  15
   Val Glu Gly
<210> 74
   <211> 19
   <212> PRT
   <213> artificial
<220>
   <223> L2 fragment
<400> 74
   Lys Thr Cys Lys Gln Ala Gly Thr Cys Pro Ser Asp Val Ile Asn Lys
   1               5                   10                  15
   Val Glu Gly
<210> 75
   <211> 19
   <212> PRT
   <213> artificial
<220>
   <223> L2 fragment
<400> 75
   Lys Thr Cys Lys Leu Ser Gly Thr Cys Pro Glu Asp Val Val Asn Lys
   1               5                   10                  15
   Ile Glu Gln
<210> 76
   <211> 19
   <212> PRT
   <213> artificial
<220>
   <223> L2 fragment
<400> 76
   Arg Thr Cys Lys Gln Ser Gly Thr Cys Pro Pro Asp Val Val Asp Lys
   1               5                   10                  15
   Val Glu Gly
<210> 77
   <211> 19
   <212> PRT
   <213> artificial
<220>
   <223> L2 fragment
<400> 77
   Ser Thr Cys Lys Ala Ala Gly Thr Cys Pro Pro Asp Val Val Asn Lys
   1               5                   10                  15
   Val Glu Gly
<210> 78
   <211> 19
   <212> PRT
   <213> artificial
<220>
   <223> L2 fragment
<400> 78
   Pro Thr Cys Lys Ile Ala Gly Asn Cys Pro Ala Asp Ile Gln Asn Lys
   1               5                   10                  15
   Phe Glu Asn
<210> 79
   <211> 19
   <212> PRT
   <213> artificial
<220>
   <223> L2 fragment
<400> 79
   Pro Ala Cys Lys Ile Ser Asn Thr Cys Pro Pro Asp Ile Ile Asn Lys
   1               5                   10                  15
   Tyr Glu Asn
<210> 80
   <211> 12
   <212> PRT
   <213> artificial
<220>
   <223> L2 fragment
<400> 80
   Arg Gly Cys Lys Gln Ala Gly Thr Cys Pro Pro Asp
   1 5 10
<210> 81
   <211> 12
   <212> PRT
   <213> artificial
<220>
   <223> L2 fragment
<400> 81
   Arg Gly Cys Lys Ala Ser Asn Thr Cys Pro Pro Asp
   1 5 10
<210> 82
   <211> 12
   <212> PRT
   <213> artificial
<220>
   <223> L2 fragment
<400> 82
   Arg Gly Cys Lys Ala Ala Gly Thr Cys Pro Pro Asp
   1 5 10
<210> 83
   <211> 12
   <212> PRT
   <213> artificial
<220>
   <223> L2 fragment
<400> 83
   Gln Ser Cys Lys Ala Ala Gly Thr Cys Pro Pro Asp
   1 5 10
<210> 84
   <211> 12
   <212> PRT
   <213> artificial
<220>
   <223> L2 fragment
<400> 84
   Gln Thr Cys Lys Gln Ala Gly Thr Cys Pro Pro Asp
   1 5 10
<210> 85
   <211> 12
   <212> PRT
   <213> artificial
<220>
   <223> L2 fragment
<400> 85
   Arg Thr Cys Lys Gln Ala Gly Thr Cys Pro Pro Asp
   1 5 10
<210> 86
   <211> 12
   <212> PRT
   <213> artificial
<220>
   <223> L2 fragment
<400> 86
   Lys Gly Cys Lys Ala Ser Gly Thr Cys Pro Pro Asp
   1 5 10
<210> 87
   <211> 12
   <212> PRT
   <213> artificial
<220>
   <223> L2 fragment
<400> 87
   Arg Thr Cys Lys Gln Ser Gly Thr Cys Pro Pro Asp
   1 5 10
<210> 88
   <211> 12
   <212> PRT
   <213> artificial
<220>
   <223> L2 fragment
<400> 88
   Arg Thr Cys Lys Val Thr Gly Thr Cys Pro Ala Asp
   1 5 10
<210> 89
   <211> 12
   <212> PRT
   <213> artificial
<220>
   <223> L2 fragment
<400> 89
   Arg Thr Cys Lys Ala Thr Gly Thr Arg Pro Ala Asp
   1 5 10
<210> 90
   <211> 12
   <212> PRT
   <213> artificial
<220>
   <223> L2 fragment
<400> 90
   Lys Thr Cys Lys Val Ala Gly Thr Cys Pro Pro Asp
   1 5 10
<210> 91
   <211> 12
   <212> PRT
   <213> artificial
<220>
   <223> L2 fragment
<400> 91
   Lys Thr Cys Lys Ala Ala Gly Thr Cys Pro Pro Asp
   1 5 10
<210> 92
   <211> 12
   <212> PRT
   <213> artificial
<220>
   <223> L2 fragment
<400> 92
   Arg Thr Cys Lys Ala Ala Gly Thr Cys Pro Pro Asp
   1 5 10
<210> 93
   <211> 12
   <212> PRT
   <213> artificial
<220>
   <223> L2 fragment
<400> 93
   Arg Thr Cys Lys Ala Ser Gly Thr Cys Pro Pro Asp
   1 5 10
<210> 94
   <211> 12
   <212> PRT
   <213> artificial
<220>
   <223> L2 fragment
<400> 94
   Ser Thr Cys Lys Ala Ala Gly Thr Cys Pro Ala Asp
   1               5                   10
<210> 95
   <211> 12
   <212> PRT
   <213> artificial
<220>
   <223> L2 fragment
<400> 95
   Lys Thr Cys Lys Leu Ser Gly Thr Cys Pro Glu Asp
   1               5                   10
<210> 96
   <211> 12
   <212> PRT
   <213> artificial
<220>
   <223> L2 fragment
<400> 96
   Lys Thr Cys Lys Gln Ala Gly Thr Cys Pro Glu Asp
   1               5                   10
<210> 97
   <211> 12
   <212> PRT
   <213> artificial
<220>
   <223> L2 fragment
<400> 97
   Gln Thr Cys Lys Ala Ser Gly Thr Cys Pro Pro Asp
   1               5                   10
<210> 98
   <211> 12
   <212> PRT
   <213> artificial
<220>
   <223> L2 fragment
<400> 98
   Gln Thr Cys Lys Ala Ala Gly Thr Cys Pro Ser Asp
   1 5 10
<210> 99
   <211> 12
   <212> PRT
   <213> artificial
<220>
   <223> L2 fragment
<400> 99
   Gln Thr Cys Lys Leu Thr Gly Thr Cys Pro Pro Asp
   1 5 10
<210> 100
   <211> 12
   <212> PRT
   <213> artificial
<220>
   <223> L2 fragment
<400> 100
   Lys Gln Cys Gln Leu Gly Ala Asp Cys Pro Pro Asp
   1 5 10
<210> 101
   <211> 12
   <212> PRT
   <213> artificial
<220>
   <223> L2 fragment
<400> 101
   Ala Lys Cys Gln Leu Ser Gly Asn Cys Leu Pro Asp
   1 5 10
<210> 102
   <211> 12
   <212> PRT
   <213> artificial
<220>
   <223> L2 fragment
<400> 102
   Ala Lys Cys Gln Leu Ser Gly Asp Cys Leu Pro Asp
   1 5 10
<210> 103
   <211> 12
   <212> PRT
   <213> artificial
<220>
   <223> L2 fragment
<400> 103
   Arg His Cys Ala Leu Ser Gly Thr Cys Pro Asp Asp
   1 5 10
<210> 104
   <211> 12
   <212> PRT
   <213> artificial
<220>
   <223> L2 fragment
<400> 104
   Lys His Cys Ala Gly Ser Gly Thr Cys Pro Glu Asp
   1 5 10
<210> 105
   <211> 12
   <212> PRT
   <213> artificial
<220>
   <223> L2 fragment
<400> 105
   Lys Thr Cys Leu Gln Gly Gly Asp Cys Ile Pro Asp
   1 5 10
<210> 106
   <211> 12
   <212> PRT
   <213> artificial
<220>
   <223> L2 fragment
<400> 106
   Arg Ser Cys Leu Gln Gly Gly Asp Cys Ile Pro Asp
   1 5 10
<210> 107
   <211> 12
   <212> PRT
   <213> artificial
<220>
   <223> L2 fragment
<400> 107
   Gln Thr Cys Lys Ala Thr Gly Thr Cys Pro Pro Asp
   1 5 10
<210> 108
   <211> 12
   <212> PRT
   <213> artificial
<220>
   <223> L2 fragment
<400> 108
   Lys Thr Cys Lys Gln Ser Gly Thr Cys Pro Pro Asp
   1 5 10
<210> 109
   <211> 12
   <212> PRT
   <213> artificial
<220>
   <223> L2 fragment
<400> 109
   Lys Thr Cys Lys Gln Ala Gly Thr Cys Pro Ser Asp
   1 5 10
<210> 110
   <211> 12
   <212> PRT
   <213> artificial
<220>
   <223> L2 fragment
<400> 110
   Ser Thr Cys Lys Ala Ala Gly Thr Cys Pro Pro Asp
   1 5 10
<210> 111
   <211> 12
   <212> PRT
   <213> artificial
<220>
   <223> L2 fragment
<400> 111
   Pro Thr Cys Lys Ile Ala Gly Asn Cys Pro Ala Asp
   1 5 10
<210> 112
   <211> 12
   <212> PRT
   <213> artificial
<220>
   <223> L2 fragment
<400> 112
   Pro Ala Cys Lys Ile Ser Asn Thr Cys Pro Pro Asp
   1               5                   10
<210> 113
   <211> 11
   <212> PRT
   <213> artificial
<220>
   <223> L2 fragment
<400> 113
   Gly Cys Lys Gln Ala Gly Thr Cys Pro Pro Asp
   1               5                   10
<210> 114
   <211> 11
   <212> PRT
   <213> artificial
<220>
   <223> L2 fragment
<400> 114
   Gly Cys Lys Ala Ser Asn Thr Cys Pro Pro Asp
   1               5                   10
<210> 115
   <211> 11
   <212> PRT
   <213> artificial
<220>
   <223> L2 fragment
<400> 115
   Gly Cys Lys Ala Ala Gly Thr Cys Pro Pro Asp
   1               5                   10
<210> 116
   <211> 11
   <212> PRT
   <213> artificial
<220>
   <223> L2 fragment
<400> 116
   Ser Cys Lys Ala Ala Gly Thr Cys Pro Pro Asp
   1 5 10
<210> 117
   <211> 11
   <212> PRT
   <213> artificial
<220>
   <223> L2 fragment
<400> 117
   Thr Cys Lys Gln Ser Gly Thr Cys Pro Ser Asp
   1 5 10
<210> 118
   <211> 11
   <212> PRT
   <213> artificial
<220>
   <223> L2 fragment
<400> 118
   Gly Cys Lys Ala Ser Gly Thr Cys Pro Pro Asp
   1 5 10
<210> 119
   <211> 11
   <212> PRT
   <213> artificial
<220>
   <223> L2 fragment
<400> 119
   Thr Cys Lys Gln Ser Gly Thr Cys Pro Pro Asp
   1 5 10
<210> 120
   <211> 11
   <212> PRT
   <213> artificial
<220>
   <223> L2 fragment
<400> 120
   Thr Cys Lys Val Thr Gly Thr Cys Pro Ala Asp
   1 5 10
<210> 121
   <211> 11
   <212> PRT
   <213> artificial
<220>
   <223> L2 fragment
<400> 121
   Thr Cys Lys Ala Thr Gly Thr Arg Pro Ala Asp
   1 5 10
<210> 122
   <211> 11
   <212> PRT
   <213> artificial
<220>
   <223> L2 fragment
<400> 122
   Thr Cys Lys Val Ala Gly Thr Cys Pro Pro Asp
   1 5 10
<210> 123
   <211> 11
   <212> PRT
   <213> artificial
<220>
   <223> L2 fragment
<400> 123
   Thr Cys Lys Ala Ala Gly Thr Cys Pro Pro Asp
   1 5 10
<210> 124
   <211> 11
   <212> PRT
   <213> artificial
<220>
   <223> L2 fragment
<400> 124
   Thr Cys Lys Ala Ser Gly Thr Cys Pro Pro Asp
   1 5 10
<210> 125
   <211> 11
   <212> PRT
   <213> artificial
<220>
   <223> L2 fragment
<400> 125
   Thr Cys Lys Ala Ala Gly Thr Cys Pro Ala Asp
   1 5 10
<210> 126
   <211> 11
   <212> PRT
   <213> artificial
<220>
   <223> L2 fragment
<400> 126
   Thr Cys Lys Leu Ser Gly Thr Cys Pro Glu Asp
   1 5 10
<210> 127
   <211> 11
   <212> PRT
   <213> artificial
<220>
   <223> L2 fragment
<400> 127
   Thr Cys Lys Ala Ala Gly Thr Cys Pro Ser Asp
   1 5 10
<210> 128
   <211> 11
   <212> PRT
   <213> artificial
<220>
   <223> L2 fragment
<400> 128
   Thr Cys Lys Leu Thr Gly Thr Cys Pro Pro Asp
   1 5 10
<210> 129
   <211> 11
   <212> PRT
   <213> artificial
<220>
   <223> L2 fragment
<400> 129
   Gln Cys Gln Leu Gly Ala Asp Cys Pro Pro Asp
   1 5 10
<210> 130
   <211> 11
   <212> PRT
   <213> artificial
<220>
   <223> L2 fragment
<400> 130
   Lys Cys Gln Leu Ser Gly Asn Cys Leu Pro Asp
   1 5 10
<210> 131
   <211> 11
   <212> PRT
   <213> artificial
<220>
   <223> L2 fragment
<400> 131
   Lys Cys Gln Leu Ser Gly Asp Cys Leu Pro Asp
   1 5 10
<210> 132
   <211> 11
   <212> PRT
   <213> artificial
<220>
   <223> L2 fragment
<400> 132
   His Cys Ala Leu Ser Gly Thr Cys Pro Asp Asp
   1 5 10
<210> 133
   <211> 11
   <212> PRT
   <213> artificial
<220>
   <223> L2 fragment
<400> 133
   His Cys Ala Gly Ser Gly Thr Cys Pro Glu Asp
   1 5 10
<210> 134
   <211> 11
   <212> PRT
   <213> artificial
<220>
   <223> L2 fragment
<400> 134
   Thr Cys Leu Gln Gly Gly Asp Cys Ile Pro Asp
   1 5 10
<210> 135
   <211> 11
   <212> PRT
   <213> artificial
<220>
   <223> L2 fragment
<400> 135
   Ser Cys Leu Gln Gly Gly Asp Cys Ile Pro Asp
   1 5 10
<210> 136
   <211> 11
   <212> PRT
   <213> artificial
<220>
   <223> L2 fragment
<400> 136
   Thr Cys Lys Ala Thr Gly Thr Cys Pro Pro Asp
   1 5 10
<210> 137
   <211> 11
   <212> PRT
   <213> artificial
<220>
   <223> L2 fragment
<400> 137
   Thr Cys Lys Gln Ala Gly Thr Cys Pro Ser Asp
   1 5 10
<210> 138
   <211> 11
   <212> PRT
   <213> artificial
<220>
   <223> L2 fragment
<400> 138
   Thr Cys Lys Ile Ala Gly Asn Cys Pro Ala Asp
   1 5 10
<210> 139
   <211> 11
   <212> PRT
   <213> artificial
<220>
   <223> L2 fragment
<400> 139
   Ala Cys Lys Ile Ser Asn Thr Cys Pro Pro Asp
   1               5                   10
<210> 140
   <211> 11
   <212> PRT
   <213> artificial
<220>
   <223> L2 fragment
<400> 140
   Thr Cys Lys Gln Ser Gly Thr Cys Pro Ser Asp
   1               5                   10
<210> 141
   <211> 10
   <212> PRT
   <213> artificial
<220>
   <223> L2 fragment
<400> 141
   Cys Lys Ala Ser Asn Thr Cys Pro Pro Asp
   1               5                   10
<210> 142
   <211> 10
   <212> PRT
   <213> artificial
<220>
   <223> L2 fragment
<400> 142
   Cys Lys Ala Ser Asn Thr Cys Pro Pro Asp
   1               5                   10
<210> 143
   <211> 10
   <212> PRT
   <213> artificial
<220>
   <223> L2 fragment
<400> 143
   Cys Lys Ala Ser Gly Thr Cys Pro Pro Asp
   1 5 10
<210> 144
   <211> 10
   <212> PRT
   <213> artificial
<220>
   <223> L2 fragment
<400> 144
   Cys Lys Gln Ser Gly Thr Cys Pro Pro Asp
   1 5 10
<210> 145
   <211> 10
   <212> PRT
   <213> artificial
<220>
   <223> L2 fragment
<400> 145
   Cys Lys Val Thr Gly Thr Cys Pro Ala Asp
   1 5 10
<210> 146
   <211> 10
   <212> PRT
   <213> artificial
<220>
   <223> L2 fragment
<400> 146
   Cys Lys Ala Thr Gly Thr Arg Pro Ala Asp
   1 5 10
<210> 147
   <211> 10
   <212> PRT
   <213> artificial
<220>
   <223> L2 fragment
<400> 147
   Cys Lys Val Ala Gly Thr Cys Pro Pro Asp
   1 5 10
<210> 148
   <211> 10
   <212> PRT
   <213> artificial
<220>
   <223> L2 fragment
<400> 148
   Cys Lys Val Ala Gly Thr Cys Pro Pro Asp
   1               5                   10
<210> 149
   <211> 10
   <212> PRT
   <213> artificial
<220>
   <223> L2 fragment
<400> 149
   Cys Lys Leu Ser Gly Thr Cys Pro Glu Asp
   1               5                   10
<210> 150
   <211> 10
   <212> PRT
   <213> artificial
<220>
   <223> L2 fragment
<400> 150
   Cys Lys Ala Ala Gly Thr Cys Pro Ser Asp
   1               5                   10
<210> 151
   <211> 10
   <212> PRT
   <213> artificial
<220>
   <223> L2 fragment
<400> 151
   Cys Lys Leu Thr Gly Thr Cys Pro Pro Asp
   1               5                   10
<210> 152
   <211> 10
   <212> PRT
   <213> artificial
<220>
   <223> L2 fragment
<400> 152
   Cys Gln Leu Gly Ala Asp Cys Pro Pro Asp
   1 5 10
<210> 153
   <211> 10
   <212> PRT
   <213> artificial
<220>
   <223> L2 fragment
<400> 153
   Cys Gln Leu Ser Gly Asn Cys Leu Pro Asp
   1 5 10
<210> 154
   <211> 10
   <212> PRT
   <213> artificial
<220>
   <223> L2 fragment
<400> 154
   Cys Gln Leu Ser Gly Asn Cys Leu Pro Asp
   1 5 10
<210> 155
   <211> 10
   <212> PRT
   <213> artificial
<220>
   <223> L2 fragment
<400> 155
   Cys Ala Leu Ser Gly Thr Cys Pro Asp Asp
   1 5 10
<210> 156
   <211> 10
   <212> PRT
   <213> artificial
<220>
   <223> L2 fragment
<400> 156
   Cys Ala Gly Ser Gly Thr Cys Pro Glu Asp
   1 5 10
<210> 157
   <211> 10
   <212> PRT
   <213> artificial
<220>
   <223> L2 fragment
<400> 157
   Cys Leu Gln Gly Gly Asp Cys Ile Pro Asp
   1               5                   10
<210> 158
   <211> 10
   <212> PRT
   <213> artificial
<220>
   <223> L2 fragment
<400> 158
   Cys Lys Ala Thr Gly Thr Cys Pro Pro Asp
   1               5                   10
<210> 159
   <211> 10
   <212> PRT
   <213> artificial
<220>
   <223> L2 fragment
<400> 159
   Cys Lys Gln Ala Gly Thr Cys Pro Ser Asp
   1               5                   10
<210> 160
   <211> 10
   <212> PRT
   <213> artificial
<220>
   <223> L2 fragment
<400> 160
   Cys Lys Ile Ala Gly Asn Cys Pro Ala Asp
   1               5                   10
<210> 161
   <211> 10
   <212> PRT
   <213> artificial
<220>
   <223> L2 fragment
<400> 161
   Cys Lys Ile Ser Asn Thr Cys Pro Pro Asp
   1 5 10
<210> 162
   <211> 10
   <212> PRT
   <213> artificial
<220>
   <223> L2 fragment
<400> 162
   Gly Cys Lys Gln Ala Gly Thr Cys Pro Pro
   1 5 10
<210> 163
   <211> 10
   <212> PRT
   <213> artificial
<220>
   <223> L2 fragment
<400> 163
   Gly Cys Lys Ala Ser Asn Thr Cys Pro Pro
   1 5 10
<210> 164
   <211> 10
   <212> PRT
   <213> artificial
<220>
   <223> L2 fragment
<400> 164
   Gly Cys Lys Ala Ala Gly Thr Cys Pro Pro
   1 5 10
<210> 165
   <211> 10
   <212> PRT
   <213> artificial
<220>
   <223> L2 fragment
<400> 165
   Ser Cys Lys Ala Ala Gly Thr Cys Pro Pro
   1 5 10
<210> 166
   <211> 10
   <212> PRT
   <213> artificial
<220>
   <223> L2 fragment
<400> 166
   Thr Cys Lys Leu Ala Gly Thr Cys Pro Pro
   1               5                   10
<210> 167
   <211> 10
   <212> PRT
   <213> artificial
<220>
   <223> L2 fragment
<400> 167
   Gly Cys Lys Ala Ser Gly Thr Cys Pro Pro
   1               5                   10
<210> 168
   <211> 10
   <212> PRT
   <213> artificial
<220>
   <223> L2 fragment
<400> 168
   Thr Cys Lys Gln Ser Gly Thr Cys Pro Pro
   1               5                   10
<210> 169
   <211> 10
   <212> PRT
   <213> artificial
<220>
   <223> L2 fragment
<400> 169
   Thr Cys Lys Val Thr Gly Thr Cys Pro Ala
   1               5                   10
<210> 170
   <211> 10
   <212> PRT
   <213> artificial
<220>
   <223> L2 fragment
<400> 170
   Thr Cys Lys Ala Thr Gly Thr Arg Pro Ala
   1 5 10
<210> 171
   <211> 10
   <212> PRT
   <213> artificial
<220>
   <223> L2 fragment
<400> 171
   Thr Cys Lys Val Ala Gly Thr Cys Pro Pro
   1 5 10
<210> 172
   <211> 10
   <212> PRT
   <213> artifiicial
<400> 172
   Thr Cys Lys Ala Ala Gly Thr Cys Pro Pro
   1 5 10
<210> 173
   <211> 10
   <212> PRT
   <213> artificial
<220>
   <223> L2 fragment
<400> 173
   Thr Cys Lys Ala Ser Gly Thr Cys Pro Pro
   1 5 10
<210> 174
   <211> 10
   <212> PRT
   <213> artificial
<220>
   <223> L2 fragment
<400> 174
   Thr Cys Lys Ala Ala Gly Thr Cys Pro Ala
   1 5 10
<210> 175
   <211> 10
   <212> PRT
   <213> artificial
<220>
   <223> L2 fragment
<400> 175
   Thr Cys Lys Leu Ser Gly Thr Cys Pro Glu
   1 5 10
<210> 176
   <211> 10
   <212> PRT
   <213> artificial
<220>
   <223> L2 fragment
<400> 176
   Thr Cys Lys Ala Ala Gly Thr Cys Pro Ser
   1 5 10
<210> 177
   <211> 10
   <212> PRT
   <213> artificial
<220>
   <223> L2 fragment
<400> 177
   Thr Cys Lys Leu Thr Gly Thr Cys Pro Pro
   1 5 10
<210> 178
   <211> 10
   <212> PRT
   <213> artificial
<220>
   <223> L2 fragment
<400> 178
   Gln Cys Gln Leu Gly Ala Asp Cys Pro Pro
   1 5 10
<210> 179
   <211> 10
   <212> PRT
   <213> artificial
<220>
   <223> L2 fragment
<400> 179
   Lys Cys Gln Leu Ser Gly Asn Cys Leu Pro
   1 5 10
<210> 180
   <211> 10
   <212> PRT
   <213> artificial
<220>
   <223> L2 fragment
<400> 180
   Lys Cys Gln Leu Ser Gly Asp Cys Leu Pro
   1 5 10
<210> 181
   <211> 10
   <212> PRT
   <213> artificial
<220>
   <223> L2 fragment
<400> 181
   His Cys Ala Leu Ser Gly Thr Cys Pro Asp
   1 5 10
<210> 182
   <211> 10
   <212> PRT
   <213> artificial
<220>
   <223> L2 fragment
<400> 182
   His Cys Ala Gly Ser Gly Thr Cys Pro Glu
   1 5 10
<210> 183
   <211> 10
   <212> PRT
   <213> artificial
<220>
   <223> L2 fragment
<400> 183
   Thr Cys Leu Gln Gly Gly Asp Cys Ile Pro
   1 5 10
<210> 184
   <211> 10
   <212> PRT
   <213> artificial
<220>
   <223> L2 fragment
<400> 184
   Ser Cys Leu Gln Gly Gly Asp Cys Ile Pro
   1               5                   10
<210> 185
   <211> 10
   <212> PRT
   <213> artificial
<220>
   <223> L2 fragment
<400> 185
   Thr Cys Lys Ala Thr Gly Thr Cys Pro Pro
   1               5                   10
<210> 186
   <211> 10
   <212> PRT
   <213> artificial
<220>
   <223> L2 fragment
<400> 186
   Thr Cys Lys Gln Ala Gly Thr Cys Pro Ser
   1               5                   10
<210> 187
   <211> 10
   <212> PRT
   <213> artificial
<220>
   <223> L2 fragment
<400> 187
   Thr Cys Lys Ile Ala Gly Asn Cys Pro Ala
   1               5                   10
<210> 188
   <211> 10
   <212> PRT
   <213> artificial
<220>
   <223> L2 fragment
<400> 188
   Ala Cys Lys Ile Ser Asn Thr Cys Pro Pro
   1               5                   10
<210> 189
   <211> 9
   <212> PRT
   <213> artificial
<220>
   <223> L2 fragment
<400> 189
<210> 190
   <211> 9
   <212> PRT
   <213> artificial
<220>
   <223> L2 fragment
<400> 190
   Cys Lys Ala Ser Asn Thr Cys Pro Pro
   1               5
<210> 191
   <211> 9
   <212> PRT
   <213> artificial
<220>
   <223> L2 fragment
<400> 191
   Cys Lys Ala Ala Gly Thr Cys Pro Pro
   1               5
<210> 192
   <211> 9
   <212> PRT
   <213> artificial
<220>
   <223> L2 fragment
<400> 192
   Cys Lys Ala Ser Gly Thr Cys Pro Pro
   1               5
<210> 193
   <211> 9
   <212> PRT
   <213> artificial
<220>
   <223> L2 fragment
<400> 193
   Cys Lys Gln Ser Gly Thr Cys Pro Pro
   1 5
<210> 194
   <211> 9
   <212> PRT
   <213> artificial
<220>
   <223> L2 fragment
<400> 194
   Cys Lys Val Thr Gly Thr Cys Pro Ala
   1 5
<210> 195
   <211> 9
   <212> PRT
   <213> artificial
<220>
   <223> L2 fragment
<400> 195
   Cys Lys Ala Thr Gly Thr Arg Pro Ala
   1 5
<210> 196
   <211> 9
   <212> PRT
   <213> artificial
<220>
   <223> L2 fragment
<400> 196
   Cys Lys Val Ala Gly Thr Cys Pro Pro
   1 5
<210> 197
   <211> 9
   <212> PRT
   <213> artificial
<220>
   <223> L2 fragment
<400> 197
   Cys Lys Val Ala Gly Thr Cys Pro Pro
   1 5
<210> 198
   <211> 9
   <212> PRT
   <213> artificial
<220>
   <223> L2 fragment
<400> 198
   Cys Lys Leu Ser Gly Thr Cys Pro Glu
   1               5
<210> 199
   <211> 9
   <212> PRT
   <213> artificial
<220>
   <223> L2 fragment
<400> 199
   Cys Lys Ala Ala Gly Thr Cys Pro Ser
   1               5
<210> 200
   <211> 9
   <212> PRT
   <213> artificial
<220>
   <223> L2 fragment
<400> 200
   Cys Lys Leu Thr Gly Thr Cys Pro Pro
   1               5
<210> 201
   <211> 9
   <212> PRT
   <213> artificial
<220>
   <223> L2 fragment
<400> 201
   Cys Gln Leu Gly Ala Asp Cys Pro Pro
   1               5
<210> 202
   <211> 9
   <212> PRT
   <213> artificial
<220>
   <223> L2 fragment
<400> 202
   Cys Gln Leu Ser Gly Asn Cys Leu Pro
   1 5
<210> 203
   <211> 9
   <212> PRT
   <213> artifical
<400> 203
   Cys Gln Leu Ser Gly Asp Cys Leu Pro
   1 5
<210> 204
   <211> 9
   <212> PRT
   <213> artificial
<220>
   <223> L2 fragment
<400> 204
   Cys Ala Leu Ser Gly Thr Cys Pro Asp
   1 5
<210> 205
   <211> 9
   <212> PRT
   <213> artificial
<220>
   <223> L2 fragment
<400> 205
   Cys Ala Gly Ser Gly Thr Cys Pro Glu
   1 5
<210> 206
   <211> 9
   <212> PRT
   <213> artificial
<220>
   <223> L2 fragment
<400> 206
   Cys Leu Gln Gly Gly Asp Cys Ile Pro
   1 5
<210> 207
   <211> 9
   <212> PRT
   <213> artificial
<220>
   <223> L2 fragment
<400> 207
   Cys Lys Ala Thr Gly Thr Cys Pro Pro
   1 5
<210> 208
   <211> 9
   <212> PRT
   <213> artificial
<220>
   <223> L2 fragment
<400> 208
   Cys Lys Gln Ala Gly Thr Cys Pro Ser
   1 5
<210> 209
   <211> 9
   <212> PRT
   <213> artificial
<220>
   <223> L2 fragment
<400> 209
   Cys Lys Gln Ala Gly Thr Cys Pro Ser
   1 5
<210> 210
   <211> 9
   <212> PRT
   <213> artificial
<220>
   <223> L2 fragment
<400> 210
   Cys Lys Ile Ser Asn Thr Cys Pro Pro
   1 5
<210> 211
   <211> 10
   <212> PRT
   <213> artificial
<220>
   <223> L2 fragment
<400> 211
   Asp Val Ile Asn Lys Val Glu Gln Thr Thr
   1 5 10
<210> 212
   <211> 10
   <212> PRT
   <213> artificial
<220>
   <223> L2 fragment
<400> 212
   Asp Val Ile Asn Lys Val Glu Gln Ser Thr
   1               5                   10
<210> 213
   <211> 10
   <212> PRT
   <213> artificial
<220>
   <223> L2 fragment
<400> 213
   Asp Val Ile Asn Lys Val Glu Gln Lys Thr
   1               5                   10
<210> 214
   <211> 10
   <212> PRT
   <213> artificial
<220>
   <223> L2 fragment
<400> 214
   Asp Val Leu Asn Lys Val Glu Gln Thr Thr
   1               5                   10
<210> 215
   <211> 10
   <212> PRT
   <213> artificial
<220>
   <223> L2 fragment
<400> 215
   Asp Val Val Asn Lys Val Glu Gln Thr Thr
   1               5                   10
<210> 216
   <211> 10
   <212> PRT
   <213> artificial
<220>
   <223> L2 fragment
<400> 216
   Asp Val Ile Asn Lys Val Glu Ser Thr Thr
   1 5 10
<210> 217
   <211> 10
   <212> PRT
   <213> artificial
<220>
   <223> L2 fragment
<400> 217
   Asp Val Ile Asn Lys Val Glu Gln Asn Thr
   1 5 10
<210> 218
   <211> 10
   <212> PRT
   <213> artificial
<220>
   <223> L2 fragment
<400> 218
   Asp Val Val Pro Lys Val Glu Gly Asp Thr
   1 5 10
<210> 219
   <211> 10
   <212> PRT
   <213> artificial
<220>
   <223> L2 fragment
<400> 219
   Asp Val Ile Pro Lys Val Glu Gly Asp Thr
   1 5 10
<210> 220
   <211> 10
   <212> PRT
   <213> artificial
<220>
   <223> L2 fragment
<400> 220
   Asp Ile Ile Pro Arg Val Glu Gln Asn Thr
   1 5 10
<210> 221
   <211> 10
   <212> PRT
   <213> artificial
<220>
   <223> L2 fragment
<400> 221
   Asp Ile Ile Pro Arg Leu Glu Gln Asn Thr
   1 5 10
<210> 222
   <211> 10
   <212> PRT
   <213> artificial
<220>
   <223> L2 fragment
<400> 222
<210> 223
   <211> 10
   <212> PRT
   <213> artificial
<220>
   <223> L2 fragment
<400> 223
   Asp Ile Ile Pro Arg Val Glu Gln Asp Thr
   1 5 10
<210> 224
   <211> 10
   <212> PRT
   <213> artificial
<220>
   <223> L2 fragment
<400> 224
   Asp Ile Ile Pro Lys Val Glu Gln Lys Thr
   1 5 10
<210> 225
   <211> 10
   <212> PRT
   <213> artificial
<220>
   <223> L2 fragment
<400> 225
   Asp Val Ile Pro Lys Val Glu Gly Ser Thr
   1 5 10
<210> 226
   <211> 10
   <212> PRT
   <213> artificial
<220>
   <223> L2 fragment
<400> 226
   Asp Ile Ile Pro Lys Val Glu His Asn Thr
   1 5 10
<210> 227
   <211> 10
   <212> PRT
   <213> artificial
<220>
   <223> L2 fragment
<400> 227
   Asp Val Ile Pro Lys Val Glu Gln Asn Thr
   1 5 10
<210> 228
   <211> 10
   <212> PRT
   <213> artificial
<220>
   <223> L2 fragment
<400> 228
   Asp Val Ile Pro Lys Val Glu His Asn Thr
   1 5 10
<210> 229
   <211> 10
   <212> PRT
   <213> artificial
<220>
   <223> L2 fragment
<400> 229
   Asp Val Ile Asn Lys Val Glu His Thr Thr
   1 5 10
<210> 230
   <211> 10
   <212> PRT
   <213> artificial
<220>
   <223> L2 fragment
<400> 230
   Asp Val Arg Asn Lys Val Glu Gly Thr Thr
   1 5 10
<210> 231
   <211> 10
   <212> PRT
   <213> artificial
<220>
   <223> L2 fragment
<400> 231
   Asp Val Lys Asn Lys Val Glu Ala Asp Thr
   1 5 10
<210> 232
   <211> 10
   <212> PRT
   <213> artificial
<220>
   <223> L2 fragment
<400> 232
   Asp Val Lys Asn Lys Val Glu Ala Asn Thr
   1 5 10
<210> 233
   <211> 10
   <212> PRT
   <213> artificial
<220>
   <223> L2 fragment
<400> 233
   Asp Val Lys Asn Lys Val Glu Asn Asn Thr
   1 5 10
<210> 234
   <211> 10
   <212> PRT
   <213> artificial
<220>
   <223> L2 fragment
<400> 234
   Asp Val Lys Asn Lys Val Glu Gln Thr Thr
   1 5 10
<210> 235
   <211> 10
   <212> PRT
   <213> artificial
<220>
   <223> L2 fragment
<400> 235
   Asp Val Lys Asn Lys Phe Glu Asn Ser Thr
   1 5 10
<210> 236
   <211> 10
   <212> PRT
   <213> artificial
<220>
   <223> L2 fragment
<400> 236
   Asp Val Gln Asn Lys Phe Glu Gly Asn Thr
   1 5 10
<210> 237
   <211> 10
   <212> PRT
   <213> artificial
<220>
   <223> L2 fragment
<400> 237
   Asp Ile Gln Asn Lys Ile Glu Gln Thr Thr
   1 5 10
<210> 238
   <211> 10
   <212> PRT
   <213> artificial
<220>
   <223> L2 fragment
<400> 238
   Asp Val Ile Lys Arg Tyr Glu Gln Thr Thr
   1 5 10
<210> 239
   <211> 9
   <212> PRT
   <213> artificial
<220>
   <223> L2 fragment
<400> 239
   Val Ile Asn Lys Val Glu Gln Thr Thr
   1 5
<210> 240
   <211> 9
   <212> PRT
   <213> artificial
<220>
   <223> L2 fragment
<400> 240
   Val Ile Asn Lys Val Glu Gln Ser Thr
   1 5
<210> 241
   <211> 9
   <212> PRT
   <213> artificial
<220>
   <223> L2 fragment
<400> 241
   Val Ile Asn Lys Val Glu Gln Lys Thr
   1 5
<210> 242
   <211> 9
   <212> PRT
   <213> artificial
<220>
   <223> L2 fragment
<400> 242
   Val Leu Asn Lys Val Glu Gln Thr Thr
   1 5
<210> 243
   <211> 9
   <212> PRT
   <213> artificial
<220>
   <223> L2 fragment
<400> 243
   Val Val Asn Lys Val Glu Gln Thr Thr
   1 5
<210> 244
   <211> 9
   <212> PRT
   <213> artificial
<220>
   <223> L2 fragment
<400> 244
   Val Ile Asn Lys Val Glu Ser Thr Thr
   1 5
<210> 245
   <211> 9
   <212> PRT
   <213> artificial
<220>
   <223> L2 fragment
<400> 245
   Val Ile Asn Lys Val Glu Gln Asn Thr
   1 5
<210> 246
   <211> 9
   <212> PRT
   <213> artificial
<220>
   <223> L2 fragment
<400> 246
   Val Val Pro Lys Val Glu Gly Asp Thr
   1 5
<210> 247
   <211> 9
   <212> PRT
   <213> artificial
<220>
   <223> L2 fragment
<400> 247
   Val Ile Pro Lys Val Glu Gly Asp Thr
   1 5
<210> 248
   <211> 9
   <212> PRT
   <213> artificial
<220>
   <223> L2 fragment
<400> 248
   Ile Ile Pro Arg Val Glu Gln Asn Thr
   1 5
<210> 249
   <211> 9
   <212> PRT
   <213> artificial
<220>
   <223> L2 fragment
<400> 249
   Ile Ile Pro Arg Leu Glu Gln Asn Thr
   1 5
<210> 250
   <211> 9
   <212> PRT
   <213> artificial
<220>
   <223> L2 fragment
<400> 250
   Ile Ile Pro Arg Val Glu Gln Asp Thr
   1 5
<210> 251
   <211> 9
   <212> PRT
   <213> artificial
<220>
   <223> L2 fragment
<400> 251
   Val Ile Pro Lys Val Glu Gly Thr Thr
   1 5
<210> 252
   <211> 9
   <212> PRT
   <213> artificial
<220>
   <223> L2 fragment
<400> 252
   Ile Ile Pro Lys Val Glu Gln Lys Thr
   1 5
<210> 253
   <211> 9
   <212> PRT
   <213> artificial
<220>
   <223> L2 fragment
<400> 253
   Val Ile Pro Lys Val Glu Gly Ser Thr
   1 5
<210> 254
   <211> 9
   <212> PRT
   <213> artificial
<220>
   <223> L2 fragment
<400> 254
   Ile Ile Pro Lys Val Glu His Asn Thr
   1 5
<210> 255
   <211> 9
   <212> PRT
   <213> artificial
<220>
   <223> L2 fragment
<400> 255
   Val Ile Pro Lys Val Glu Gln Asn Thr
   1 5
<210> 256
   <211> 9
   <212> PRT
   <213> artificial
<220>
   <223> L2 fragment
<400> 256
   Val Ile Pro Lys Val Glu His Asn Thr
   1 5
<210> 257
   <211> 9
   <212> PRT
   <213> artificial
<220>
   <223> L2 fragment
<400> 257
   Val Ile Asn Lys Val Glu His Thr Thr
   1 5
<210> 258
   <211> 9
   <212> PRT
   <213> artificial
<220>
   <223> L2 fragment
<400> 258
   Val Arg Asn Lys Val Glu Gly Thr Thr
   1 5
<210> 259
   <211> 9
   <212> PRT
   <213> artificial
<220>
   <223> L2 fragment
<400> 259
   Val Lys Asn Lys Val Glu Ala Asp Thr
   1 5
<210> 260
   <211> 9
   <212> PRT
   <213> artificial
<220>
   <223> L2 fragment
<400> 260
   Val Lys Asn Lys Val Glu Ala Asn Thr
   1 5
<210> 261
   <211> 9
   <212> PRT
   <213> artificial
<220>
   <223> L2 fragment
<400> 261
   Val Lys Asn Lys Val Glu Asn Asn Thr
   1               5
<210> 262
   <211> 9
   <212> PRT
   <213> artificial
<220>
   <223> L2 fragment
<400> 262
   Val Lys Asn Lys Val Glu Gln Thr Thr
   1               5
<210> 263
   <211> 9
   <212> PRT
   <213> artificial
<220>
   <223> L2 fragment
<400> 263
   Val Lys Asn Lys Phe Glu Asn Ser Thr
   1               5
<210> 264
   <211> 9
   <212> PRT
   <213> artificial
<220>
   <223> L2 fragment
<400> 264
   Val Gln Asn Lys Phe Glu Gly Asn Thr
   1               5
<210> 265
   <211> 9
   <212> PRT
   <213> artificial
<220>
   <223> L2 fragment
<400> 265
   Ile Gln Asn Lys Ile Glu Gln Thr Thr
   1               5
<210> 266
   <211> 9
   <212> PRT
   <213> artificial
<220>
   <223> L2 fragment
<400> 266
   Val Ile Lys Arg Tyr Glu Gln Thr Thr
   1 5
<210> 267
   <211> 8
   <212> PRT
   <213> artificial
<220>
   <223> L2 fragment
<400> 267
   Ile Asn Lys Val Glu Gln Thr Thr
   1 5
<210> 268
   <211> 8
   <212> PRT
   <213> artificial
<220>
   <223> L2 fragment
<400> 268
   Ile Asn Lys Val Glu Gln Thr Thr
   1 5
<210> 269
   <211> 8
   <212> PRT
   <213> artificial
<220>
   <223> L2 fragment
<400> 269
   Ile Asn Lys Val Glu Gln Lys Thr
   1 5
<210> 270
   <211> 8
   <212> PRT
   <213> artificial
<220>
   <223> L2 fragment
<400> 270
   Leu Asn Lys Val Glu Gln Thr Thr
   1 5
<210> 271
   <211> 8
   <212> PRT
   <213> artificial
<220>
   <223> L2 fragment
<400> 271
   Val Asn Lys Val Glu Gln Thr Thr
   1 5
<210> 272
   <211> 8
   <212> PRT
   <213> artificial
<220>
   <223> L2 fragment
<400> 272
   Ile Asn Lys Val Glu Ser Thr Thr
   1 5
<210> 273
   <211> 8
   <212> PRT
   <213> artificial
<220>
   <223> L2 fragment
<400> 273
   Ile Asn Lys Val Glu Gln Asn Thr
   1 5
<210> 274
   <211> 8
   <212> PRT
   <213> artificial
<220>
   <223> L2 fragment
<400> 274
   Val Pro Lys Val Glu Gly Asp Thr
   1 5
<210> 275
   <211> 8
   <212> PRT
   <213> artificial
<220>
   <223> L2 fragment
<400> 275
   Ile Pro Lys Val Glu Gly Asp Thr
   1               5
<210> 276
   <211> 8
   <212> PRT
   <213> artificial
<220>
   <223> L2 fragment
<400> 276
   Ile Pro Arg Val Glu Gln Asn Thr
   1               5
<210> 277
   <211> 8
   <212> PRT
   <213> artificial
<220>
   <223> L2 fragment
<400> 277
   Ile Pro Arg Leu Glu Gln Asn Thr
   1               5
<210> 278
   <211> 8
   <212> PRT
   <213> artificial
<220>
   <223> L2 fragment
<400> 278
   Ile Pro Arg Val Glu Gln Asp Thr
   1               5
<210> 279
   <211> 8
   <212> PRT
   <213> artificial
<220>
   <223> L2 fragment
<400> 279
   Ile Pro Lys Val Glu Gly Thr Thr
   1 5
<210> 280
   <211> 8
   <212> PRT
   <213> artificial
<220>
   <223> L2 fragment
<400> 280
   Ile Pro Lys Val Glu His Lys Thr
   1 5
<210> 281
   <211> 8
   <212> PRT
   <213> artificial
<220>
   <223> L2 fragment
<400> 281
   Ile Pro Lys Val Glu Gly Ser Thr
   1 5
<210> 282
   <211> 8
   <212> PRT
   <213> artificial
<220>
   <223> L2 fragment
<400> 282
   Ile Pro Lys Val Glu His Asn Thr
   1 5
<210> 283
   <211> 8
   <212> PRT
   <213> artificial
<220>
   <223> L2 fragment
<400> 283
   Ile Pro Lys Val Glu Gln Asn Thr
   1 5
<210> 284
   <211> 8
   <212> PRT
   <213> artificial
<220>
   <223> L2 fragment
<400> 284
   Ile Asn Lys Val Glu His Thr Thr
   1 5
<210> 285
   <211> 8
   <212> PRT
   <213> artificial
<220>
   <223> L2 fragment
<400> 285
   Arg Asn Lys Val Glu Gly Thr Thr
   1 5
<210> 286
   <211> 8
   <212> PRT
   <213> artificial
<220>
   <223> L2 fragment
<400> 286
   Lys Asn Lys Val Glu Ala Asp Thr
   1 5
<210> 287
   <211> 8
   <212> PRT
   <213> artificial
<220>
   <223> L2 fragment
<400> 287
   Lys Asn Lys Val Glu Ala Asn Thr
   1 5
<210> 288
   <211> 8
   <212> PRT
   <213> artificial
<220>
   <223> L2 fragment
<400> 288
   Lys Asn Lys Val Glu Asn Asn Thr
   1               5
<210> 289
   <211> 8
   <212> PRT
   <213> artificial
<220>
   <223> L2 fragment
<400> 289
   Lys Asn Lys Val Glu Gln Thr Thr
   1               5
<210> 290
   <211> 8
   <212> PRT
   <213> artificial
<220>
   <223> L2 fragment
<400> 290
   Lys Asn Lys Phe Glu Asn Ser Thr
   1               5
<210> 291
   <211> 8
   <212> PRT
   <213> artificial
<220>
   <223> L2 fragment
<400> 291
   Lys Asn Lys Phe Glu Asn Ser Thr
   1               5
<210> 292
   <211> 8
   <212> PRT
   <213> artificial
<220>
   <223> L2 fragment
<400> 292
   Gln Asn Lys Ile Glu Gln Thr Thr
   1               5
<210> 293
   <211> 8
   <212> PRT
   <213> artificial
<220>
   <223> L2 fragment
<400> 293
   Ile Lys Arg Tyr Glu Gln Thr Thr
   1 5
<210> 294
   <211> 9
   <212> PRT
   <213> artificial
<220>
   <223> L2 fragment
<400> 294
   Thr Gly Tyr Ile Pro Leu Gln Thr Arg
   1 5
<210> 295
   <211> 9
   <212> PRT
   <213> artificial
<220>
   <223> L2 fragment
<400> 295
   Thr Gly Tyr Val Pro Leu Gly Ser Thr
   1 5
<210> 296
   <211> 9
   <212> PRT
   <213> artificial
<220>
   <223> L2 fragment
<400> 296
   Thr Gly Tyr Val Pro Leu Gly Asn Thr
   1 5
<210> 297
   <211> 9
   <212> PRT
   <213> artificial
<220>
   <223> L2 fragment
<400> 297
   Thr Gly Tyr Val Pro Leu Ser Thr Gly
   1 5
<210> 298
   <211> 9
   <212> PRT
   <213> artificial
<220>
   <223> L2 fragment
<400> 298
   Thr Gly Tyr Ile Pro Leu Gln Ser Thr
   1 5
<210> 299
   <211> 9
   <212> PRT
   <213> artificial
<220>
   <223> L2 fragment
<400> 299
   Thr Gly Tyr Val Pro Val Gly Ser Thr
   1 5
<210> 300
   <211> 9
   <212> PRT
   <213> artificial
<220>
   <223> L2 fragment
<400> 300
   Thr Gly Tyr Val Pro Leu Gln Thr Ser
   1 5
<210> 301
   <211> 9
   <212> PRT
   <213> artificial
<220>
   <223> L2 fragment
<400> 301
   Thr Gly Tyr Val Pro Leu Thr Thr Gly
   1 5
<210> 302
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 302
   Arg Gly Cys Lys Gln Xaa Gly Thr Cys Pro Pro Asp Val Ile Asn Lys
   1               5                   10                  15
   Val Glu Gln
<210> 303
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 303
   Arg Gly Cys Lys Ala Xaa Asn Thr Cys Pro Pro Asp Val Ile Asn Lys
   1               5                   10                  15
   Val Glu Gln
<210> 304
   <211> 19
   <212> PRT
   <213> artificial
<220>
   <223> L2 fragment
<220>
   <221> MISC_FEATURE
   <222> (6)..(6)
   <223> Xaa represent Gly or Ala
<400> 304
   Arg Gly Cys Lys Ala Xaa Gly Thr Cys Pro Pro Asp Val Ile Asn Lys
   1 5 10 15
   Val Glu Gln
<210> 305
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 305
   Gln Ser Cys Lys Ala Xaa Gly Thr Cys Pro Pro Asp Val Leu Asn Lys
   1 5 10 15
   Val Glu Gln
<210> 306
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 306
   Gln Ser Cys Lys Ala Xaa Gly Thr Cys Pro Pro Asp Val Val Asn Lys
   1 5 10 15
   Val Glu Gln
<210> 307
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 307
   Gln Thr Cys Lys Gln Xaa Gly Thr Cys Pro Pro Asp Val Ile Asn Lys
   1               5                   10                  15
   Val Glu Gln
<210> 308
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 308
   Gln Thr Cys Lys Gln Xaa Gly Thr Cys Pro Pro Asp Val Ile Asn Lys
   1               5                   10                  15
   Val Glu Gln
<210> 309
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 309
   Arg Thr Cys Lys Gln Xaa Gly Thr Cys Pro Pro Asp Val Ile Asn Lys
   1               5                   10                  15
   Val Glu Ser
<210> 310
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 310
   Arg Thr Cys Lys Gln Xaa Gly Thr Cys Pro Pro Asp Val Ile Asn Lys
   1               5                   10                  15
   Val Glu Gln
<210> 311
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 311
   Lys Gly Cys Lys Ala Xaa Gly Thr Cys Pro Pro Asp Val Ile Asn Lys
   1               5                   10                  15
   Val Glu Gln
<210> 312
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 312
   Arg Thr Cys Lys Gln Xaa Gly Thr Cys Pro Pro Asp Val Val Pro Lys
   1               5                   10                  15
   Val Glu Gly
<210> 313
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 313
   Arg Thr Cys Lys Gln Xaa Gly Thr Cys Pro Pro Asp Val Ile Pro Lys
   1               5                   10                  15
   Val Glu Gly
<210> 314
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 314
   Arg Thr Cys Lys Val Xaa Gly Thr Cys Pro Ala Asp Val Val Pro Lys
   1               5                   10                  15
   Val Glu Gly
<210> 315
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 315
   Arg Thr Cys Lys Ala Xaa Gly Thr Arg Pro Ala Asp Val Ile Pro Lys
   1 5 10 15
   Val Glu Gly
<210> 316
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 316
   Ser Thr Cys Lys Ala Xaa Gly Thr Cys Pro Pro Asp Val Ile Pro Lys
   1 5 10 15
   Leu Glu Gly
<210> 317
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 317
   Arg Thr Cys Lys Gln Xaa Gly Thr Cys Pro Pro Asp Ile Ile Pro Arg
   1 5 10 15
   Leu Glu Gln
<210> 318
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 318
   Arg Thr Cys Lys Gln Xaa Gly Thr Cys Pro Pro Asp Ile Ile Pro Arg
   1               5                   10                  15
   Val Glu Gln
<210> 319
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 319
   Lys Thr Cys Lys Val Xaa Gly Thr Cys Pro Pro Asp Val Ile Pro Lys
   1               5                   10                  15
   Val Glu Gly
<210> 320
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 320
   Lys Thr Cys Lys Ala Xaa Gly Thr Cys Pro Pro Asp Val Ile Pro Lys
   1               5                   10                  15
   Val Glu Gly
<210> 321
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 321
   Ser Thr Cys Lys Ala Xaa Gly Thr Cys Pro Pro Asp Val Ile Pro Lys
   1               5                   10                  15
   Val Glu Gly
<210> 322
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 322
   Arg Thr Cys Lys Ala Xaa Gly Thr Cys Pro Pro Asp Val Ile Pro Lys
   1               5                   10                  15
   Val Glu Gly
<210> 323
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 323
   Ser Thr Cys Lys Ala Xaa Gly Thr Cys Pro Ala Asp Val Ile Pro Lys
   1               5                   10                  15
   Val Glu Gly
<210> 324
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 324
   Lys Thr Cys Lys Leu Xaa Gly Thr Cys Pro Glu Asp Val Ile Asn Lys
   1               5                   10                  15
   Val Glu Gln
<210> 325
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 325
   Lys Thr Cys Lys Gln Xaa Gly Thr Cys Pro Pro Asp Ile Ile Pro Lys
   1               5                   10                  15
   Ile Glu Gly
<210> 326
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 326
   Lys Thr Cys Lys Gln Xaa Gly Thr Cys Pro Pro Asp Ile Val Pro Lys
   1 5 10 15
   Val Glu Gly
<210> 327
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 327
   Ser Thr Cys Lys Gln Xaa Gly Thr Cys Pro Pro Asp Ile Ile Pro Arg
   1 5 10 15
   Val Glu Gln
<210> 328
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 328
   Lys Thr Cys Lys Gln Xaa Gly Thr Cys Pro Pro Asp Val Ile Pro Lys
   1 5 10 15
   Val Glu Gly
<210> 329
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 329
   Gln Thr Cys Lys Ala Xaa Gly Thr Cys Pro Ser Asp Ile Ile Pro Lys
   1 5 10 15
   Val Glu His
<210> 330
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 330
   Gln Thr Cys Lys Ala Xaa Gly Thr Cys Pro Pro Asp Val Ile Pro Lys
   1 5 10 15
   Val Glu Gln
<210> 331
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 331
   Gln Thr Cys Lys Leu Xaa Gly Thr Cys Pro Pro Asp Val Ile Pro Lys
   1 5 10 15
   Val Glu His
<210> 332
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 332
   Gln Thr Cys Lys Ala Xaa Gly Thr Cys Pro Ser Asp Val Ile Asn Lys
   1               5                   10                  15
   Val Glu His
<210> 333
   <211> 19
   <212> PRT
   <213> artificial
<220>
   <223> L2 fragment
<220>
   <221> MISC_FEATURE
   <222> (6)..(6)
   <223> Xaa represenst Gly or Ala
<400> 333
   Lys Gln Cys Gln Leu Xaa Ala Asp Cys Pro Pro Asp Val Arg Asn Lys
   1               5                   10                  15
   Val Glu Gly
<210> 334
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 334
   Ala Lys Cys Gln Leu Xaa Gly Asn Cys Leu Pro Asp Val Lys Asn Lys
   1               5                   10                  15
   Val Glu Ala
<210> 335
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 335
   Ala Lys Cys Gln Leu Xaa Gly Asp Cys Leu Pro Asp Val Lys Asn Lys
   1               5                   10                  15
   Val Glu Ala
<210> 336
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 336
   Arg His Cys Ala Leu Xaa Gly Thr Cys Pro Asp Asp Val Lys Asn Lys
   1               5                   10                  15
   Val Glu Asn
<210> 337
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 337
   Lys His Cys Ala Gly Xaa Gly Thr Cys Pro Glu Asp Val Lys Asn Lys
   1 5 10 15
   Val Glu Gln
<210> 338
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 338
   Lys Thr Cys Leu Gln Xaa Gly Asp Cys Ile Pro Asp Val Lys Asn Lys
   1 5 10 15
   Phe Glu Asn
<210> 339
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 339
   Arg Ser Cys Leu Gln Xaa Gly Asp Cys Ile Pro Asp Val Gln Asn Lys
   1 5 10 15
   Phe Glu Gly
<210> 340
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 340
   Gln Thr Cys Lys Ala Xaa Gly Thr Cys Pro Pro Asp Val Ile Pro Lys
   1               5                   10                  15
   Val Glu Gly
<210> 341
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 341
   Lys Thr Cys Lys Gln Xaa Gly Thr Cys Pro Pro Asp Val Val Pro Lys
   1               5                   10                  15
   Val Glu Gly
<210> 342
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 342
   Arg Thr Cys Lys Gln Xaa Gly Thr Cys Pro Pro Asp Val Ile Asn Lys
   1               5                   10                  15
   Val Glu Gly
<210> 343
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 343
   Lys Thr Cys Lys Gln Xaa Gly Thr Cys Pro Ser Asp Val Ile Asn Lys
   1               5                   10                  15
   Val Glu Gly
<210> 344
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 344
   Lys Thr Cys Lys Leu Xaa Gly Thr Cys Pro Glu Asp Val Val Asn Lys
   1               5                   10                  15
   Ile Glu Gln
<210> 345
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 345
   Arg Thr Cys Lys Gln Xaa Gly Thr Cys Pro Pro Asp Val Val Asp Lys
   1 5 10 15
   Val Glu Gly
<210> 346
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 346
   Ser Thr Cys Lys Ala Xaa Gly Thr Cys Pro Pro Asp Val Val Asn Lys
   1 5 10 15
   Val Glu Gly
<210> 347
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 347
   Pro Thr Cys Lys Ile Xaa Gly Asn Cys Pro Ala Asp Ile Gln Asn Lys
   1 5 10 15
   Phe Glu Asn
<210> 348
   <211> 19
   <212> PRT
   <213> artificial
<220>
   <223> L2 fragment
<220>
   <221> MISC_FEATURE
   <222> (6)..(6)
   <223> Xaa represents Gly or Ala
<400> 348
   Pro Ala Cys Lys Ile Xaa Asn Thr Cys Pro Pro Asp Ile Ile Asn Lys
   1               5                   10                  15
   Tyr Glu Asn
<210> 349
   <211> 12
   <212> PRT
   <213> artificial
<220>
   <223> L2 fragment
<220>
   <221> MISC_FEATURE
   <222> (6)..(6)
   <223> Xaa represents Gly or Ala
<400> 349
   Arg Gly Cys Lys Gln Xaa Gly Thr Cys Pro Pro Asp
   1               5                   10
<210> 350
   <211> 12
   <212> PRT
   <213> artificial
<220>
   <223> L2 fragment
<220>
   <221> MISC_FEATURE
   <222> (6)..(6)
   <223> Xaa represents Gly or Ala
<400> 350
   Arg Gly Cys Lys Ala Xaa Asn Thr Cys Pro Pro Asp
   1               5                   10
<210> 351
   <211> 12
   <212> PRT
   <213> artificial
<220>
   <223> L2 fragment
<220>
   <221> MISC_FEATURE
   <222> (6)..(6)
   <223> Xaa represent Gly or Ala
<400> 351
   Arg Gly Cys Lys Ala Xaa Gly Thr Cys Pro Pro Asp
   1               5                   10
<210> 352
   <211> 12
   <212> PRT
   <213> artificial
<220>
   <223> L2 fragment
<220>
   <221> MISC_FEATURE
   <222> (6)..(6)
   <223> Xaa represents Gly or Ala
<400> 352
   Gln Ser Cys Lys Ala Xaa Gly Thr Cys Pro Pro Asp
   1               5                   10
<210> 353
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 353
   Gln Thr Cys Lys Gln Xaa Gly Thr Cys Pro Pro Asp
   1               5                   10
<210> 354
   <211> 12
   <212> PRT
   <213> artificial
<220>
   <223> L2 fragment
<220>
   <221> MISC_FEATURE
   <222> (6)..(6)
   <223> Xaa represents Gly or Ala
<400> 354
   Arg Thr Cys Lys Gln Xaa Gly Thr Cys Pro Pro Asp
   1 5 10
<210> 355
   <211> 12
   <212> PRT
   <213> artificial
<220>
   <223> L2 fragment
<220>
   <221> MISC_FEATURE
   <222> (6)..(6)
   <223> Xaa represent Gly or Ala
<400> 355
   Lys Gly Cys Lys Ala Xaa Gly Thr Cys Pro Pro Asp
   1 5 10
<210> 356
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 356
   Pro Thr Cys Lys Ala Xaa Gly Thr Cys Pro Pro Asp
   1 5 10
<210> 357
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 357
   Arg Thr Cys Lys Val Xaa Gly Thr Cys Pro Ala Asp
   1 5 10
<210> 358
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 358
   Arg Thr Cys Lys Ala Xaa Gly Thr Arg Pro Ala Asp
   1               5                   10
<210> 359
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 359
   Lys Thr Cys Lys Val Xaa Gly Thr Cys Pro Pro Asp
   1               5                   10
<210> 360
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 360
   Lys Thr Cys Lys Ala Xaa Gly Thr Cys Pro Pro Asp
   1               5                   10
<210> 361
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 361
   Arg Thr Cys Lys Ala Xaa Gly Thr Cys Pro Pro Asp
   1               5                   10
<210> 362
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 362
   Ser Thr Cys Lys Ala Xaa Gly Thr Arg Pro Pro Asp
   1               5                   10
<210> 363
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 363
   Ser Thr Cys Lys Ala Xaa Gly Thr Cys Pro Ala Asp
   1               5                   10
<210> 364
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 364
   Lys Thr Cys Lys Leu Xaa Gly Thr Cys Pro Glu Asp
   1 5 10
<210> 365
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 365
   Ala Thr Cys Lys Gln Xaa Gly Thr Cys Pro Pro Asp
   1 5 10
<210> 366
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 366
   Ser Thr Cys Lys Gln Xaa Gly Thr Cys Pro Pro Asp
   1 5 10
<210> 367
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 367
   Gln Thr Cys Lys Ala Xaa Gly Thr Cys Pro Ser Asp
   1               5                   10
<210> 368
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 368
   Gln Thr Cys Lys Leu Xaa Gly Thr Cys Pro Pro Asp
   1               5                   10
<210> 369
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 369
   Lys Gln Cys Gln Leu Xaa Ala Asp Cys Pro Pro Asp
   1               5                   10
<210> 370
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 370
   Ala Lys Cys Gln Leu Xaa Gly Asn Cys Leu Pro Asp
   1 5 10
<210> 371
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 371
   Ala Lys Cys Gln Leu Xaa Gly Asp Cys Leu Pro Asp
   1 5 10
<210> 372
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 372
   Arg His Cys Ala Leu Xaa Gly Thr Cys Pro Asp Asp
   1 5 10
<210> 373
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 373
   Lys His Cys Ala Gly Xaa Gly Thr Cys Pro Glu Asp
   1 5 10
<210> 374
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 374
   Lys Thr Cys Leu Gln Xaa Gly Asp Cys Ile Pro Asp
   1               5                   10
<210> 375
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 375
   Arg Ser Cys Leu Gln Xaa Gly Asp Cys Ile Pro Asp
   1               5                   10
<210> 376
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 376
   Gln Thr Cys Lys Ala Xaa Gly Thr Cys Pro Pro Asp
   1               5                   10
<210> 377
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 377
   Lys Thr Cys Lys Gln Xaa Gly Thr Cys Pro Glu Asp
   1               5                   10
<210> 378
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 378
   Lys Thr Cys Lys Gln Xaa Gly Thr Cys Pro Ser Asp
   1               5                   10
<210> 379
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 379
   Lys Thr Cys Lys Gln Xaa Gly Thr Cys Pro Ser Asp
   1               5                   10
<210> 380
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 380
   Pro Thr Cys Lys Ile Xaa Gly Asn Cys Pro Ala Asp
   1               5                   10
<210> 381
   <211> 12
   <212> PRT
   <213> artificial
<220>
   <223> L2 fragment
<220>
   <221> MISC_FEATURE
   <222> (6) .. (6)
   <223> Xaa represents Gly or Ala
<400> 381
   Pro Ala Cys Lys Ile Xaa Asn Thr Cys Pro Pro Asp
   1               5                   10
<210> 382
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 382
   Gly Cys Lys Gln Xaa Gly Thr Cys Pro Pro Asp
   1               5                   10
<210> 383
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 383
   Gly Cys Lys Ala Xaa Asn Thr Cys Pro Pro Asp
   1               5                   10
<210> 384
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 384
   Ala Cys Lys Ala Xaa Gly Thr Cys Pro Pro Asp
   1               5                   10
<210> 385
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 385
   Ser Cys Lys Ala Xaa Gly Thr Cys Pro Pro Asp
   1               5                   10
<210> 386
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 386
   Lys Cys Lys Ala Xaa Gly Thr Cys Ile Pro Asp
   1               5                   10
<210> 387
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 387
   Gly Cys Lys Ala Xaa Gly Thr Cys Pro Pro Asp
   1               5                   10
<210> 388
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 388
   Lys Cys Lys Ala Xaa Gly Thr Cys Pro Pro Asp
   1               5                   10
<210> 389
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 389
   Thr Cys Lys Val Xaa Gly Thr Cys Pro Ala Asp
   1               5                   10
<210> 390
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 390
   Thr Cys Lys Ala Xaa Gly Thr Arg Pro Ala Asp
   1               5                   10
<210> 391
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 391
   Thr Cys Lys Val Xaa Gly Thr Cys Pro Pro Asp
   1               5                   10
<210> 392
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 392
   Ser Cys Lys Leu Xaa Gly Thr Cys Pro Pro Asp
   1               5                   10
<210> 393
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 393
   Ser Cys Lys Gln Xaa Gly Thr Cys Pro Ser Asp
   1               5                   10
<210> 394
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 394
   Thr Cys Lys Ala Xaa Gly Thr Cys Pro Ala Asp
   1               5                   10
<210> 395
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 395
   Thr Cys Lys Leu Xaa Gly Thr Cys Pro Glu Asp
   1               5                   10
<210> 396
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 396
   Thr Cys Lys Ala Xaa Gly Thr Cys Pro Ser Asp
   1 5 10
<210> 397
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 397
   Thr Cys Lys Leu Xaa Gly Thr Cys Pro Pro Asp
   1 5 10
<210> 398
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 398
   Gln Cys Gln Leu Xaa Ala Asp Cys Pro Pro Asp
   1 5 10
<210> 399
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 399
   Lys Cys Gln Leu Xaa Gly Asn Cys Leu Pro Asp
   1 5 10
<210> 400
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 400
   Lys Cys Gln Leu Xaa Gly Asp Cys Leu Pro Asp
   1 5 10
<210> 401
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 401
   His Cys Ala Leu Xaa Gly Thr Cys Pro Asp Asp
   1 5 10
<210> 402
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 402
   His Cys Ala Gly Xaa Gly Thr Cys Pro Glu Asp
   1 5 10
<210> 403
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 403
   Thr Cys Leu Gln Xaa Gly Asp Cys Ile Pro Asp
   1               5                   10
<210> 404
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 404
   Thr Cys Leu Gln Xaa Gly Asp Cys Ile Pro Asp
   1               5                   10
<210> 405
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 405
   Thr Cys Lys Ala Xaa Gly Thr Cys Pro Pro Asp
   1               5                   10
<210> 406
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 406
   Thr Cys Lys Gln Xaa Gly Thr Cys Pro Ser Asp
   1               5                   10
<210> 407
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 407
   Thr Cys Lys Ile Xaa Gly Asn Cys Pro Ala Asp
   1               5                   10
<210> 408
   <211> 11
   <212> PRT
   <213> artificial
<220>
   <223> L2 fragment
<220>
   <221> MISC_FEATURE
   <222> (5)..(5)
   <223> Xaa represents Gly or Ala
<400> 408
   Thr Cys Lys Ile Xaa Gly Asn Cys Pro Ala Asp
   1               5                   10
<210> 409
   <211> 10
   <212> PRT
   <213> artificial
<220>
   <223> L2 fragment
<220>
   <221> MISC_FEATURE
   <222> (4)..(4)
   <223> Xaa represent Gly or Ala
<400> 409
   Cys Lys Gln Xaa Gly Thr Cys Pro Asp Asp
   1 5 10
<210> 410
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 410
   Cys Lys Ala Xaa Asn Thr Cys Pro Pro Asp
   1 5 10
<210> 411
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 411
   Cys Leu Ala Xaa Gly Thr Cys Pro Ala Asp
   1 5 10
<210> 412
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 412
   Cys Leu Ala Xaa Gly Thr Cys Pro Pro Asp
   1 5 10
<210> 413
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 413
   Cys Lys Leu Xaa Gly Thr Cys Pro Ala Asp
   1               5                   10
<210> 414
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 414
   Cys Lys Val Xaa Gly Thr Cys Pro Ala Asp
   1               5                   10
<210> 415
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 415
   Cys Lys Ala Xaa Gly Thr Arg Pro Ala Asp
   1               5                   10
<210> 416
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 416
   Cys Lys Ala Xaa Gly Thr Arg Pro Ala Asp
   1               5                   10
<210> 417
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 417
   Cys Lys Ala Xaa Gly Thr Cys Pro Ala Asp
   1               5                   10
<210> 418
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 418
   Cys Lys Leu Xaa Gly Thr Cys Pro Glu Asp
   1               5                   10
<210> 419
   <211> 10
   <212> PRT
   <213> artificial
<220>
   <223> L2 fragment
<220>
   <221> MISC_FEATURE
   <222> (4)..(4)
   <223> L2 fragment
<400> 419
   Cys Lys Ala Xaa Gly Thr Cys Pro Ser Asp
   1               5                   10
<210> 420
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 420
   Cys Lys Leu Xaa Gly Thr Cys Pro Pro Asp
   1               5                   10
<210> 421
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 421
   Cys Gln Leu Xaa Ala Asp Cys Pro Pro Asp
   1               5                   10
<210> 422
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 422
   Cys Gln Leu Xaa Gly Asn Cys Leu Pro Asp
   1 5 10
<210> 423
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 423
   Cys Gln Leu Xaa Gly Asp Cys Leu Pro Asp
   1 5 10
<210> 424
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 424
   Cys Ala Leu Xaa Gly Thr Cys Pro Asp Asp
   1 5 10
<210> 425
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 425
   Cys Ala Gly Xaa Gly Thr Cys Pro Glu Asp
   1 5 10
<210> 426
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 426
   Cys Leu Gln Xaa Gly Asp Cys Ile Pro Asp
   1 5 10
<210> 427
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 427
   Cys Lys Ala Xaa Gly Thr Cys Pro Pro Asp
   1 5 10
<210> 428
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 428
   Cys Lys Gln Xaa Gly Thr Cys Pro Ser Asp
   1 5 10
<210> 429
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 429
   Cys Lys Ile Xaa Gly Asn Cys Pro Ala Asp
   1               5                   10
<210> 430
   <211> 10
   <212> PRT
   <213> artificial
<220>
   <223> L2 fragment
<220>
   <221> MISC_FEATURE
   <222> (4)..(4)
   <223> Xaa represents Gly or Ala
<400> 430
   Cys Lys Ile Xaa Asn Thr Cys Pro Pro Asp
   1               5                   10
<210> 431
   <211> 10
   <212> PRT
   <213> artificial
<220>
   <223> L2 fragment
<220>
   <221> MISC_FEATURE
   <222> (4)..(4)
   <223> Xaa represents Gly or Ala
<220>
   <221> MISC_FEATURE
   <222> (5)..(5)
   <223> Xaa represents Gly or Ala
<400> 431
   Gly Cys Lys Gln Xaa Gly Thr Cys Pro Pro
   1               5                   10
<210> 432
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 432
   Gly Cys Lys Ala Xaa Asn Thr Cys Pro Pro
   1               5                   10
<210> 433
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 433
   Gly Cys Lys Ala Xaa Gly Thr Cys Pro Pro
   1               5                   10
<210> 434
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 434
   Ser Cys Lys Ala Xaa Gly Thr Cys Pro Pro
   1               5                   10
<210> 435
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 435
   Thr Cys Lys Gln Xaa Gly Thr Cys Pro Pro
   1               5                   10
<210> 436
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 436
   Gly Cys Lys Ala Xaa Gly Thr Cys Pro Pro
   1               5                   10
<210> 437
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 437
   Thr Cys Lys Gln Xaa Gly Thr Cys Pro Pro
   1               5                   10
<210> 438
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 438
   Thr Cys Lys Val Xaa Gly Thr Cys Pro Ala
   1               5                   10
<210> 439
   <211> 10
   <212> PRT
   <213> artificial
<220>
   <223> Xaa represents Gly or Ala
<220>
   <221> misc_feature
   <222> (5)..(5)
   <223> Xaa can be any naturally occurring amino acid
<400> 439
   Thr Cys Lys Ala Xaa Gly Thr Arg Pro Ala
   1               5                   10
<210> 440
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 440
   Thr Cys Lys Val Xaa Gly Thr Cys Pro Pro
   1               5                   10
<210> 441
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 441
   Thr Cys Lys Ala Xaa Gly Thr Cys Pro Pro
   1               5                   10
<210> 442
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 442
   Thr Cys Lys Ala Xaa Gly Thr Cys Pro Pro
   1               5                   10
<210> 443
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 443
   Thr Cys Lys Ala Xaa Gly Thr Cys Pro Ala
   1               5                   10
<210> 444
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 444
   Thr Cys Lys Leu Xaa Gly Thr Cys Pro Glu
   1               5                   10
<210> 445
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 445
   Thr Cys Lys Ala Xaa Gly Thr Cys Pro Ser
   1               5                   10
<210> 446
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 446
   Thr Cys Lys Leu Xaa Gly Thr Cys Pro Pro
   1               5                   10
<210> 447
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 447
   Gln Cys Gln Leu Xaa Ala Asp Cys Pro Pro
   1               5                   10
<210> 448
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 448
   Lys Cys Gln Leu Xaa Gly Asn Cys Leu Pro
   1               5                   10
<210> 449
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 449
   Lys Cys Gln Leu Xaa Gly Asp Cys Leu Pro
   1               5                   10
<210> 450
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 450
   His Cys Ala Leu Xaa Gly Thr Cys Pro Asp
   1               5                   10
<210> 451
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 451
   His Cys Ala Gly Xaa Gly Thr Cys Pro Glu
   1               5                   10
<210> 452
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 452
   Thr Cys Leu Gln Xaa Gly Asp Cys Ile Pro
   1               5                   10
<210> 453
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 453
   Ser Cys Leu Gln Xaa Gly Asp Cys Ile Pro
   1               5                   10
<210> 454
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 454
   Thr Cys Lys Ala Xaa Gly Thr Cys Pro Pro
   1               5                   10
<210> 455
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 455
   Thr Cys Lys Gln Xaa Gly Thr Cys Pro Ser
   1               5                   10
<210> 456
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 456
   Thr Cys Lys Ile Xaa Gly Asn Cys Pro Ala
   1               5                   10
<210> 457
   <211> 10
   <212> PRT
   <213> artificial
<220>
   <223> L2 fragment
<220>
   <221> MISC_FEATURE
   <222> (5)..(5)
   <223> Xaa represents Gly or Ala
<400> 457
   Ala Cys Lys Ile Xaa Asn Thr Cys Pro Pro
   1               5                   10
<210> 458
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 458
   Cys Lys Gln Xaa Gly Thr Cys Pro Pro
   1               5
<210> 459
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 459
   Cys Lys Ala Xaa Asn Thr Cys Pro Pro
   1               5
<210> 460
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 460
   Cys Lys Ala Xaa Gly Thr Cys Pro Pro
   1               5
<210> 461
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 461
   Cys Lys Ala Xaa Gly Thr Cys Pro Pro
   1               5
<210> 462
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 462
   Cys Lys Gln Xaa Gly Thr Cys Pro Pro
   1               5
<210> 463
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 463
   Cys Lys Val Xaa Gly Thr Cys Pro Ala
   1               5
<210> 464
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 464
   Cys Lys Ala Xaa Gly Thr Arg Pro Ala
   1               5
<210> 465
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 465
   Cys Lys Val Xaa Gly Thr Cys Pro Pro
   1               5
<210> 466
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 466
   Cys Lys Ala Xaa Gly Thr Cys Pro Ala
   1               5
<210> 467
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 467
   Cys Lys Leu Xaa Gly Thr Cys Pro Glu
   1 5
<210> 468
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 468
   Cys Lys Ala Xaa Gly Thr Cys Pro Ser
   1 5
<210> 469
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 469
   Cys Lys Leu Xaa Gly Thr Cys Pro Pro
   1 5
<210> 470
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 470
   Cys Gln Leu Xaa Ala Asp Cys Pro Pro
   1 5
<210> 471
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 471
   Cys Gln Leu Xaa Gly Asn Cys Leu Pro
   1 5
<210> 472
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 472
   Cys Gln Leu Xaa Gly Asp Cys Leu Pro
   1 5
<210> 473
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 473
   Cys Ala Leu Xaa Gly Thr Cys Pro Asp
   1 5
<210> 474
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 474
   Cys Ala Gly Xaa Gly Thr Cys Pro Glu
   1 5
<210> 475
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 475
   Cys Leu Gln Xaa Gly Asp Cys Ile Pro
   1 5
<210> 476
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 476
   Cys Lys Ala Xaa Gly Thr Cys Pro Pro
   1 5
<210> 477
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 477
   Cys Lys Gln Xaa Gly Thr Cys Pro Ser
   1 5
<210> 478
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 478
   Cys Lys Ile Xaa Gly Asn Cys Pro Ala
   1 5
<210> 479
   <211> 9
   <212> PRT
   <213> artificial
<220>
   <223> L2 fragment
<220>
   <221> MISC_FEATURE
   <222> (4)..(4)
   <223> Xaa represents Gly or Ala
<400> 479
   Cys Lys Ile Xaa Asn Thr Cys Pro Pro
   1 5
<210> 480
   <211> 6
   <212> PRT
   <213> artificial
<220>
   <223> loop site of FN3
<400> 480
   Pro Ala Val Thr Val Arg
   1 5
<210> 481
   <211> 9
   <212> PRT
   <213> artificial
<220>
   <223> loop site of FN3
<400> 481
   Gly Arg Gly Asp Ser Pro Ala Ser Ser
   1 5
<210> 482
   <211> 7
   <212> PRT
   <213> artificial
<220>
   <223> loop site of bilin-binding protein
<400> 482
   Pro Asn Ser Val Glu Lys Tyr
   1 5
<210> 483
   <211> 5
   <212> PRT
   <213> artificial
<220>
   <223> loop site of bilin-binding protein
<400> 483
   Ile His Gly Lys Glu
   1 5
<210> 484
   <211> 7
   <212> PRT
   <213> artificial
<220>
   <223> loop-site of bilin-binding protein
<400> 484
   Thr Tyr Gly Gly Val Thr Lys
   1 5
<210> 485
   <211> 5
   <212> PRT
   <213> artificial
<220>
   <223> loop-site
<400> 485
   Tyr Lys Gly Gln Pro
   1 5
<210> 486
   <211> 19
   <212> PRT
   <213> Human papillomavirus type 18
<400> 486
   Lys Thr Cys Lys Gln Ser Gly Thr Cys Pro Ser Asp Val Val Asn Lys
   1 5 10 15
   Val Glu Gly
<210> 487
   <211> 19
   <212> PRT
   <213> Human papillomavirus type 31
<400> 487
   Gln Thr Cys Lys Ala Ala Gly Thr Cys Pro Ser Asp Val Ile Pro Lys
   1 5 10 15
   Ile Glu His
<210> 488
   <211> 19
   <212> PRT
   <213> Human papillomavirus type 68
<400> 488
   Lys Thr Cys Lys Gln Ser Gly Thr Cys Pro Pro Asp Val Ile Asp Lys
   1 5 10 15
   Val Glu Gly
<210> 489
   <211> 19
   <212> PRT
   <213> Human Paillomavirus Type 82
<400> 489
   Ser Thr Cys Lys Ala Ala Gly Thr Cys Pro Pro Asp Val Ile Pro Lys
   1 5 10 15
   Val Lys Gly
<210> 490
   <211> 12
   <212> PRT
   <213> Human papillomavirus type 18
<400> 490
   Lys Thr Cys Lys Gln Ser Gly Thr Cys Pro Ser Asp
   1 5 10
<210> 491
   <211> 195
   <212> PRT
   <213> Artificial Sequence
<220>
   <223> (SEQ ID NO:2)x3+ (SEQ ID NO: 487)x3 +(SEQ ID NO:77)x3 with tripeptide (GGP) linkers
<400> 491
   Lys Thr Cys Lys Gln Ala Gly Thr Cys Pro Pro Asp Ile Ile Pro Lys
   1 5 10 15
   Val Glu Gly Gly Gly Pro Lys Thr Cys Lys Gln Ala Gly Thr Cys Pro
   20 25 30
   Pro Asp Ile Ile Pro Lys Val Glu Gly Gly Gly Pro Lys Thr Cys Lys
   35 40 45
   Gln Ala Gly Thr Cys Pro Pro Asp Ile Ile Pro Lys Val Glu Gly Gly
   50 55 60
   Gly Pro Gln Thr Cys Lys Ala Ala Gly Thr Cys Pro Ser Asp Val Ile
   65 70 75 80
   Pro Lys Ile Glu His Gly Gly Pro Gln Thr Cys Lys Ala Ala Gly Thr
   85 90 95
   Cys Pro Ser Asp Val Ile Pro Lys Ile Glu His Gly Gly Pro Gln Thr
   100 105 110
   Cys Lys Ala Ala Gly Thr Cys Pro Ser Asp Val Ile Pro Lys Ile Glu
   115 120 125
   His Gly Gly Pro Ser Thr Cys Lys Ala Ala Gly Thr Cys Pro Pro Asp
   130 135 140
   Val Val Asn Lys Val Glu Gly Gly Gly Pro Ser Thr Cys Lys Ala Ala
   145 150 155 160
   Gly Thr Cys Pro Pro Asp Val Val Asn Lys Val Glu Gly Gly Gly Pro
   165 170 175
   Ser Thr Cys Lys Ala Ala Gly Thr Cys Pro Pro Asp Val Val Asn Lys
   180 185 190
   Val Glu Gly
   195
<210> 492
   <211> 195
   <212> PRT
   <213> Artificial Sequence
<220>
   <223> ((SEQ ID NO:2)+ (SEQ ID NO:487) +(SEQ ID NO:77))x3 with tripeptide (GGP) linkers
<400> 492
   Lys Thr Cys Lys Gln Ala Gly Thr Cys Pro Pro Asp Ile Ile Pro Lys
   1               5                   10                  15
   Val Glu Gly Gly Gly Pro Gln Thr Cys Lys Ala Ala Gly Thr Cys Pro
               20                  25                  30
   Ser Asp Val Ile Pro Lys Ile Glu His Gly Gly Pro Ser Thr Cys Lys
           35                  40                  45
   Ala Ala Gly Thr Cys Pro Pro Asp Val Val Asn Lys Val Glu Gly Gly
       50                  55                  60
   Gly Pro Lys Thr Cys Lys Gln Ala Gly Thr Cys Pro Pro Asp Ile Ile
   65                  70                  75                  80
   Pro Lys Val Glu Gly Gly Gly Pro Gln Thr Cys Lys Ala Ala Gly Thr
                   85                  90                  95
   Cys Pro Ser Asp Val Ile Pro Lys Ile Glu His Gly Gly Pro Ser Thr
               100                 105                 110
   Cys Lys Ala Ala Gly Thr Cys Pro Pro Asp Val Val Asn Lys Val Glu
           115                 120                 125
   Gly Gly Gly Pro Lys Thr Cys Lys Gln Ala Gly Thr Cys Pro Pro Asp
       130                 135                 140
   Ile Ile Pro Lys Val Glu Gly Gly Gly Pro Gln Thr Cys Lys Ala Ala
   145                 150                 155                 160
   Gly Thr Cys Pro Ser Asp Val Ile Pro Lys Ile Glu His Gly Gly Pro
                   165                 170                 175
   Ser Thr Cys Lys Ala Ala Gly Thr Cys Pro Pro Asp Val Val Asn Lys
               180                 185                 190
   Val Glu Gly
           195
<210> 493
   <211> 100
   <212> PRT
   <213> Pyrococcus furiosus
<400> 493
   Met Ile Ile Glu Tyr Asp Gly Glu Ile Asp Phe Thr Lys Gly Arg Val
   1               5                   10                  15
   Val Leu Trp Phe Ser Ile Pro Gly Cys Gly Pro Cys Arg Leu Val Glu
               20                  25                  30
   Arg Phe Met Thr Glu Leu Ser Glu Tyr Phe Glu Asp Ile Gln Ile Val
           35                  40                  45
   His Ile Asn Ala Gly Lys Trp Lys Asn Ile Val Asp Lys Phe Asn Ile
       50                  55                  60
   Leu Asn Val Pro Thr Leu Val Tyr Leu Lys Asp Gly Arg Glu Val Gly
   65                  70                  75                  80
   Arg Gln Asn Leu Ile Arg Ser Lys Glu Glu Ile Leu Lys Lys Leu Lys
                   85                  90                  95
   Glu Leu Gln Glu
               100
<210> 494
   <211> 101
   <212> PRT
   <213> Thermococcus kodakarensis
<400> 494
   Met Ile Val Glu Tyr Asp Glu Asn Val Asp Phe Thr Lys Gly Lys Ala
   1               5                   10                  15
   Val Leu Trp Phe Ser Ile Pro Gly Cys Gly Pro Cys Arg Leu Val Glu
               20                  25                  30
   Ala Phe Met Lys Glu Leu Ser Glu Glu Phe Gly Glu Ile Ala Ile Val
           35                  40                  45
   His Val Asn Ala Glu Lys Trp Ser Gly Leu Val Glu Gly Phe Arg Ile
       50                  55                  60
   Leu Asn Val Pro Thr Leu Val Tyr Leu Lys Asp Gly Lys Glu Val Ala
   65                  70                  75                  80
   Arg Gln Asn Leu Ile Arg Gly Lys Gly Glu Val Leu Ile Lys Phe Glu
                   85                  90                  95
   Glu Pro Arg Glu Leu
               100
<210> 495
   <211> 105
   <212> PRT
   <213> Thermococcus onnurineus
<400> 495
   Met Ile Arg Glu Phe Asp Gly Asp Phe Gly Lys Val Glu Arg Ala Lys
   1               5                   10                  15
   Tyr Ala Leu Leu Trp Phe Ser Ser Pro Gly Cys Gly Pro Cys Arg Met
               20                  25                  30
   Ile Glu Pro Phe Met His Glu Leu Ser Glu Glu Tyr Lys Glu Val Glu
           35                  40                  45
   Phe Trp Glu Val Asp Val Glu Lys His Leu Pro Leu Ala Glu Lys Phe
       50                  55                  60
   Asp Val Met Asn Val Pro Thr Leu Ile Tyr Leu Lys Glu Gly Asn Glu
   65                  70                  75                  80
   Ile Ala Arg Gln Asn Leu Val Arg Lys Lys Glu Glu Val Glu Glu Lys
                   85                  90                  95
   Leu Met Met Leu Leu Gly Ser Asp Ser
               100                 105
<210> 496
   <211> 100
   <212> PRT
   <213> Thermococcus sibiricus
<400> 496
   Met Ile His Glu Tyr Asp Gly Lys Ile Asp Phe Asn Arg Gly Lys Val
   1               5                   10                  15
   Val Leu Trp Phe Ser Ile Gln Gly Cys Gly Pro Cys Arg Leu Val Glu
               20                  25                  30
   Ser Phe Met Glu Glu Val Ser Glu Glu Phe Ser Glu Ile Arg Phe Ile
           35                  40                  45
   His Val Gly Ala Glu Lys Trp Ser Asn Ile Val Lys Arg Phe Glu Val
       50                  55                  60
   Leu Asn Val Pro Thr Leu Val Tyr Leu Lys Asp Gly Lys Glu Val Ala
   65                  70                  75                  80
   Arg Gln Asn Leu Ile Arg Ser Lys Glu Glu Val Leu Ala Lys Ile Glu
                   85                  90                  95
   Glu Leu His Glu
               100
<210> 497
   <211> 253
   <212> PRT
   <213> Artificial Sequence
<220>
   <223> Dimer of Escherichia coli thioredoxin variants
<400> 497
   Met Ser Asp Lys Ile Ile His Leu Thr Asp Asp Ser Phe Asp Thr Asp
   1               5                   10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Gly Gly
               100                 105                 110
   Gly Ser Glu Gly Gly Gly Ser Glu Gly Gly Gly Ser Glu Gly Gly Gly
           115                 120                 125
   Ser Glu Gly Gly Gly Ser Glu Gly Gly Gly Ser Glu Gly Gly Gly Met
       130                 135                 140
   Ser Asp Lys Ile Ile His Leu Thr Asp Asp Ser Phe Asp Thr Asp Val
   145                 150                 155                 160
   Leu Lys Ala Asp Gly Ala Ile Leu Val Asp Phe Trp Ala Glu Trp Cys
                   165                 170                 175
   Gly Pro Gly Cys Lys Met Ile Ala Pro Ile Leu Asp Glu Ile Ala Asp
               180                 185                 190
   Glu Tyr Gln Gly Lys Leu Thr Val Ala Lys Leu Asn Ile Asp Gln Asn
           195                 200                 205
   Pro Gly Thr Ala Pro Lys Tyr Gly Ile Arg Gly Ile Pro Thr Leu Leu
       210                 215                 220
   Leu Phe Lys Asn Gly Glu Val Ala Ala Thr Lys Val Gly Ala Leu Ser
   225                 230                 235                 240
   Lys Gly Gln Leu Lys Glu Phe Leu Asp Ala Asn Leu Ala
                   245                 250
<210> 498
   <211> 396
   <212> PRT
   <213> Artificial
<220>
   <223> Trimer of Escherichia coli thioredoxin variants
<400> 498
   Met Ser Asp Lys Ile Ile His Leu Thr Asp Asp Ser Phe Asp Thr Asp
   1               5                   10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Gly Gly
               100                 105                 110
   Gly Ser Glu Gly Gly Gly Ser Glu Gly Gly Gly Ser Glu Gly Gly Gly
           115                 120                 125
   Ser Glu Gly Gly Gly Ser Glu Gly Gly Gly Ser Glu Gly Gly Gly Met
       130                 135                 140
   Ser Asp Lys Ile Ile His Leu Thr Asp Asp Ser Phe Asp Thr Asp Val
   145                 150                 155                 160
   Leu Lys Ala Asp Gly Ala Ile Leu Val Asp Phe Trp Ala Glu Trp Cys
                   165                 170                 175
   Gly Pro Gly Cys Lys Met Ile Ala Pro Ile Leu Asp Glu Ile Ala Asp
               180                 185                 190
   Glu Tyr Gln Gly Lys Leu Thr Val Ala Lys Leu Asn Ile Asp Gln Asn
           195                 200                 205
   Pro Gly Thr Ala Pro Lys Tyr Gly Ile Arg Gly Ile Pro Thr Leu Leu
       210                 215                 220
   Leu Phe Lys Asn Gly Glu Val Ala Ala Thr Lys Val Gly Ala Leu Ser
   225                 230                 235                 240
   Lys Gly Gln Leu Lys Glu Phe Leu Asp Ala Asn Leu Ala Gly Gly Gly
                   245                 250                 255
   Gly Ser Glu Gly Gly Gly Ser Glu Gly Gly Gly Ser Glu Gly Gly Gly
               260                 265                 270
   Ser Glu Gly Gly Gly Ser Glu Gly Gly Gly Ser Glu Gly Gly Gly Met
           275                 280                 285
   Ser Asp Lys Ile Ile His Leu Thr Asp Asp Ser Phe Asp Thr Asp Val
       290                 295                 300
   Leu Lys Ala Asp Gly Ala Ile Leu Val Asp Phe Trp Ala Glu Trp Cys
   305                 310                 315                 320
   Leu Ser Cys Lys Met Ile Ala Pro Ile Leu Asp Glu Ile Ala Asp Glu
                   325                 330                 335
   Tyr Gln Gly Lys Leu Thr Val Ala Lys Leu Asn Ile Asp Gln Asn Pro
               340                 345                 350
   Gly Thr Ala Pro Lys Tyr Gly Ile Arg Gly Ile Pro Thr Leu Leu Leu
           355                 360                 365
   Phe Lys Asn Gly Glu Val Ala Ala Thr Lys Val Gly Ala Leu Ser Lys
       370                 375                 380
   Gly Gln Leu Lys Glu Phe Leu Asp Ala Asn Leu Ala
   385                 390                 395

## Description

The present disclosure relates to an immunogenic polypeptide comprising a) a scaffold polypeptide, and b) a L2 polypeptide or a fragment of said L2 polypeptide, wherein said scaffold polypeptide constrains the structure of said L2 polypeptide, or of the fragment of said L2 polypeptide. Moreover, the present disclosure relates to a vaccine comprising said immunogenic polypeptide. The present disclosure is also concerned with a method for producing an antibody against human papillomavirus. Also encompassed by the present disclosure is an antibody obtained by carrying out the said method.

Cervical cancer is women's second most frequent cancer worldwide. Clinical and molecular studies have shown that certain types of human papillomavirus (HPV), referred to as high-risk types, are the etiological agents of this disease. Two anti-HPV vaccines for the prophylaxis of cervical cancer have been licensed recently by Merck (Gardasil^{™}) and GlaxoSmithKline (Cervarix^{™}) (Schmiedeskamp et al, (2006) Human papillomavirus vaccines. Ann Pharmacother, 40, 1344-1352). Both vaccines rely on the major capsid protein L1 in the form of virus-like particles (VLPs) as antigen (Roden et al., (2006) How will HPV vaccines affect cervical cancer? Nat Rev Cancer, 6, 753-763); they protect against the HPV types from which the L1-VLPs were derived, yet are largely ineffective against all but the most closely related HPV types. The two most prominent high-risk HPV types, 16 and 18, are the major targets of both vaccines, although there is evidence for partial cross-protection against HPV types 31 and 45 (reviewed by Muller and Gissmann, (2007) A long way: history of the prophylactic papillomavirus vaccine. Dis Markers, 23, 331-336; Huh and Roden, (2008) The future of vaccines for cervical cancer. Gynecol Oncol, 109, S48-56). The limited cross-protective capacity of L1-based vaccines, which is the main reason for the continuing effort toward the development of improved vaccination strategies, likely reflects the HPV type specificity of L1 neutralizing epitopes (Giroglou et al., (2001) Immunological analyses of human papillomavirus capsids. Vaccine, 19, 1783-1793).

Antibodies against the minor capsid protein L2 also neutralize HPV infection and are often capable to cross-neutralize various non-cognate virions, although with varying efficiencies (Kondo et al. 2007, Neutralization of HPV16, 18, 31, and 58 pseudovirions with antisera induced by immunizing rabbits with synthetic peptides representing segments of the HPV16 minor capsid protein L2 surface region. Virology, 358, 266-272; Gambhira, R., (2007) A protective and broadly cross-neutralizing epitope of human papillomavirus L2. J Virol, 81, 13927-13931). The N-terminal region of L2 interacts with an as yet unidentified secondary receptor on the surface of target cells (Yang et al. (2003) Cell surface-binding motifs of L2 that facilitate papillomavirus infection. J Virol, 77, 3531-3541) and this interaction can be blocked by anti-L2 antibodies. The precise identity of the L2 region involved in HPV-cell surface interaction is still a matter of debate. This was initially proposed as the region comprised of amino acids (aa) 108-120, and antibodies targeting this particular L2 region were indeed shown to block viral infection *in vitro* albeit at low titers (Kawana et al. (2001) Nasal immunization of mice with peptide having a cross-neutralization epitope on minor capsid protein L2 of human papillomavirus type 16 elicit systemic and mucosal antibodies. Vaccine, 19, 1496-1502; Kawana et al. (2001b) Human papillomavirus type 16 minor capsid protein L2 N-terminal region containing a common neutralization epitope binds to the cell surface and enters the cytoplasm. J Virol, 75, 2331-2336). Subsequent experiments identified additional neutralizing epitopes in the aa 1-88 region (Pastrana et al. (2005) Cross-neutralization of cutaneous and mucosal Papillomavirus types with anti-sera to the amino terminus of L2. Virology, 337, 365-372) as well as in more extended N-terminal regions comprised of aa 11-200 and aa 18-144 (Kondo loc. cit). Perhaps the most prominent of these N-terminal epitopes is the one located between aa 17-36. This was identified as the target of an HPV16 neutralizing and protective monoclonal antibody (RG-1) as well as the major determinant of the neutralizing activity found in sera from rabbits and humans immunized with extended versions of L2 (aa 1-88, 11-200 or the full-length protein) (Gambhira, 2007, loc cit. ). Since it had been found that mutation of L2 amino acids 18 and 19 or of amino acids 20 and 21 disrupted both L2 binding to the cell surface and viral infection (Yang, R., et al. (2003). Cell surface-binding motifs of L2 that facilitate papillomavirus infection. J. Virol. 77:3531-3541), it was concluded that the epitope recognized by the RG-1 antibody overlaps the surface-binding motif of HPV16 L2.

Besides the lack of precise knowledge on the most relevant (cross) neutralizing epitope(s), a major problem with the use of L2 as a tool for HPV prophylaxis is the poor immunogenicity of the L2 protein and peptides thereof, as compared to L1-VLPs. A substantial increase in immunogenicity has been reported lately via chemical coupling of the HPV16 L2 peptide (17-36) to a broadly recognized T helper epitope and to the Toll-like receptor ligand dipalmitoyl S-glyceryl cysteine (Alphs et al. (2008) Protection against heterologous human papillomavirus challenge by a synthetic lipopeptide vaccine containing a broadly cross-neutralizing epitope of L2. Proc Natl Acad Sci USA, 105, 5850-5855). Alternatively, L2 peptides have been fused to Adenovirus surface proteins (WO 2008/140474) or to other HPV proteins to increase immunogenicity (WO 2002/070004, de Jong et al. (2002), Enhancement of human papillomavirus (HPV) type 16 E6 and E7-specific T-cell immunity in healthy volunteers through vaccination with TA-CIN, an HPV16 L2E7E6 fusion protein vaccine, Vaccine, 20(29-30):3456-3464).

A recently developed alternative strategy for increasing peptide immunogenicity relies on the use of thioredoxin (Trx) as a scaffold protein with the ability to constrain the structure of single-copy as well as multimeric (tandemly repeated) peptide epitopes inserted within its surface-exposed active site loop (Moretto et al. (2007) Conformation-sensitive antibodies against Alzheimer amyloid-beta by immunization with a thioredoxin-constrained B-cell epitope peptide. J Biol Chem, 282, 11436-11445).

Also, the authors of Mambetisaeva et al. (1997), Protein Expression and Purification 11(1):26, used expression of connexin 32 domains as thioredoxin fusion proteins for immunization. Moreover, WO2008/140474 used recombinant adenovirus constructs comprising a HPV L2 peptide as an immunogen in mice. WO 02/070004 and De Jong (2002), Vaccine 20(29-30):3456 disclosed a vaccine comprising HPV L2, E6, and E7 as a single fusion protein inducing an antibody response in man. Furthermore, the idea of using scaffolds in immunization was summarized in Skerra et al. (2007), Curr Opinion Biotechnol 18(4):295.

Thus, the L1 polypeptide is highly immunogenic and antibodies against it show only a limited cross-protective capacity, whereas antibodies against the L2 polypeptide are capable of cross-neutralizing various HPV genotypes. The L2 polypeptide, however has only limited immunogenicity.

Therefore, immunogenic polypeptides that are highly immunogenic and allow for a cross-neutralization of various HPV genotypes without the drawbacks as referred to above are highly required.

The technical problem underlying the present invention can be seen as the provision of means and methods for complying with the aforementioned needs.

The technical problem is solved by the embodiments characterized in the claims and herein below.

Accordingly, the present disclosure relates to an immunogenic polypeptide comprising
a) a scaffold polypeptide, and
b) a L2 polypeptide having an amino acid sequence as shown in SEQ ID NO:1, or a fragment of said L2 polypeptide,
wherein said scaffold polypeptide constrains the structure of said L2 polypeptide, or of the fragment of said L2 polypeptide.

The term "polypeptide" as used herein relates to a polymer comprising amino acids linked together by peptide bonds. The term "immunogenic polypeptide" is understood by the skilled person. Immunogenic polypeptides, preferably, elicit protective immune response in a host, preferably, in a human. The immunogenic polypeptide in the context of the present invention, preferably, shall allow for establishing or improving immunity to infection with various HPV genotypes. Preferably, the immunogenic polypeptide according to the present invention allows for establishing or improving immunity to infection with human papillomavirus genotypes 16, 18, 31, 45 and 58. Preferably, the said polypeptide also allows for establishing or improving immunity to infection with human papillomavirus genotypes 6, 52, 2, 27, 57 and/or 11. Immunogenic polypeptides are preferred reagents for vaccine compositions.

The term "L2 polypeptide", preferably, refers to the N-terminal region of the full-length L2 polypeptide of HPV16 (human papillomavirus 16). The full-length L2 is one of the two capsid proteins of HPV16 and is frequently also referred to as minor capsid protein. Together with the major capsid protein, L1, the full-length L2 polypeptide forms viral capsids. The L2 polypeptide in the context of the present invention, preferably, comprises the N-terminal amino acids 1 to 120 of the HPV16 L2 polypeptide as shown in SEQ ID NO:1.

The term "fragment" as used herein, preferably, refers to a sub-polypeptide of the L2 polypeptide (as shown in SEQ ID NO:1). Said fragment comprises at least 7, preferably, at least 10, at least 12, at least 15, or at least 20 consecutive amino acid residues of said L2 polypeptide. Preferred fragments of the L2 polypeptide have an amino acid sequence as shown in SEQ ID NO:2 (KTCKQAGTCPPDIIPKVEG), as shown in SEQ ID NO:3 (KTCKQAGTCPPD), as shown in SEQ ID NO:4 (TCKQAGTCPPD), as shown in SEQ ID NO:5 (CKQAGTCPPD), as shown in SEQ ID NO:6 (TCKQAGTCPP), as shown in SEQ ID NO:7 (CKQAGTCPP), as shown in SEQ ID NO:8 (DIIPKVEGKT), as shown in SEQ ID NO:9 (TGYIPLGTR).

The most preferred fragments in the context of the present invention are fragments having a sequence as shown in SEQ ID NO:2 (KTCKQAGTCPPDIIPKVEG, amino acids 20 to 38 of the L2 polypeptide as shown in SEQ ID NO:1)), or as shown in SEQ ID NO:5 (CKQAGTCPPD, amino acids 22 to 31 of the L2 polypeptide as shown in SEQ ID NO:1).

Preferably, the terms "polypeptide" "L2 polypeptide" and "fragment of the L2 polypeptide", respectively, shall also encompass variants of said polypeptide, L2 polypeptide or variants of said fragment of said L2 polypeptide, respectively. Such variants have essentially the same immunological properties as the specific polypeptides, respectively. In particular, they share the same immunological properties if they are detectable by the same specific assays referred to in this specification, e.g., by ELISA assays using polyclonal or monoclonal antibodies specifically recognizing the said polypeptides, respectively. Moreover, it is to be understood that a variant as referred to in accordance with the present invention shall have an amino acid sequence which differs due to at least one amino acid substitution, deletion and/or addition wherein the amino acid sequence of the variant is still at least 65%, preferably at least 70%, 75%, 80%, 85%, 90%, 92%, 95%, 97%, 98%, or 99%, identical with the amino sequence of the specific polypeptide. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Further included are variants which differ due to posttranslational modifications such as phosphorylation or myristoylation.

As mentioned above, in a preferred embodiment of the present invention the fragment of the L2 polypeptide comprised by the scaffold polypeptide of the immunogenic polypeptide has a sequence as shown in SEQ ID NO:2 (KTCKQAGTCPPDIIPKVEG), or as shown in SEQ ID NO:3 (KTCKQAGTCPPD), or as shown in SEQ ID NO:4 (TCKQAGTCPPD), or as shown in SEQ ID NO:5 (CKQAGTCPPD), or as shown in SEQ ID NO:6 (TCKQAGTCPP), or a sequence as shown in SEQ ID NO:7 (CKQAGTCPP), or a sequence as shown in SEQ ID NO:31 (IIPKVEGKT), or a sequence as shown in SEQ ID NO:32 (IPKVEGKT). Since it has been shown in the context of the present invention that the Alanine (A) residue comprised by the aforementioned fragments can be replaced with other amino acid residues (particularly, with a Glycine (G) residue) without significantly affecting the immunogenicity of the polypeptide according to the invention as well as the neutralizing capacity of the antibodies against the said immunogenic polypeptide (see Examples), variants of the aforementioned fragments preferably have the amino acid sequence as shown in SEQ ID NO:10 (KTCKQXGTCPPDIIPKVEG), or as shown in SEQ ID NO:11 (KTCKQXGTCPPD), or as shown in SEQ ID NO:12 (TCKQXGTCPPD), or as shown in SEQ ID NO:13 (CKQXGTCPPD), or as shown in SEQ ID NO:14 (TCKQXGTCPP), or a sequence as shown in SEQ ID NO: 15 (CKQXGTCPP). Preferably, X represents a Glycine (G) or an Alanine (A) residue. Moreover, experiments with the aforementioned fragments of the L2 polypeptide have shown that the most crucial amino acid residues for immunogenicity and for the generation of cross-neutralizing antibodies were amino acid residues 22 to 24 (CKQ) and 26 to 31 (GTCPPD) of the L2 polypeptide as shown in SEQ ID NO:1 (see Examples). Accordingly, the most preferred variant of a fragment of the L2 polypeptide has a sequence as shown in SEQ ID NO: 13), CKQXGTCPPD).

In one preferred embodiment of the present invention, the immunogenic polypeptide comprises a multimer of the L2 polypeptide or a fragment thereof (or a variant of said L2 polypeptide or a variant of any fragment thereof). Thus, the immunogenic polypeptide shall comprise more than one L2 polypeptide or more than one fragment of the L2 polypeptide. It is particularly envisaged that the immunogenic polypeptide comprises more than one fragment of the L2 polypeptide (or variants thereof). Preferably, the immunogenic polypeptide comprises multimers of 2 to 15 fragments of the L2 polypeptide, and more preferably multimers of 3 to 9 (and, thus, of 3, 4, 5 , 6, 7, 8 or 9) fragments of the L2 polypeptide. Most preferably, said immunogenic polypeptide comprises multimers of three or four fragments of the L2 polypeptide. Preferably, said fragments are directly linked together. More preferably, said fragments are linked via a linker peptide (for an explanation of the term "linker peptide", see herein below). Preferably, if the immunogenic polypeptide comprises more than one fragment of the L2 polypeptide, the fragments shall have the same or essentially the same sequence. However, it is also contemplated that the multimer comprises various fragments (or variants thereof) of the L2 polypeptide.

Other preferred L2 fragments (or variants thereof) are selected from the group consisting of SEQ ID NO: 33 to SEQ ID NO:479. SEQ ID NO:33 to SEQ ID NO:479 are also shown in Table 1. The sequences as shown in SEQ ID NO:33 to SEQ ID NO:79 and in SEQ ID NO:486 to SEQ ID NO:489 are variants of the L2 fragment KTCKQAGTCPPDIIPKVEG as shown in SEQ ID NO:2; the sequences as shown in SEQ ID NO:80 to SEQ ID NO:112 and in SEQ ID NO: 490 are variants of the L2 fragment KTCKQAGTCPPD as shown in SEQ ID NO:3; the sequences as shown in SEQ ID NO:113 to SEQ ID NO:139 are variants of the L2 fragment TCKQAGTCPPD as shown in SEQ ID NO:4; the sequences as shown in SEQ ID NO:140 to SEQ ID NO:161 are variants of the L2 fragment CKQAGTCPPD as shown in SEQ ID NO:5; the sequences as shown in SEQ ID NO:162 to SEQ ID NO:188 are variants of the L2 fragment TCKQAGTCPP as shown in SEQ ID NO:6; the sequences as shown in SEQ ID NO:189 to SEQ ID NO:210 are variants of the L2 fragment CKQAGTCPP as shown in SEQ ID NO:7; the sequences as shown in SEQ ID NO:211 to SEQ ID NO:238 are variants of the L2 fragment DIIPKVEGKT as shown in SEQ ID NO:8; the sequences as shown in SEQ ID NO:239 to SEQ ID NO:266 are variants of the L2 fragment IIPKVEGKT as shown in SEQ ID NO:31; the sequences as shown in SEQ ID NO:267 to SEQ ID NO:293 are variants of the L2 fragment IPKVEGKT as shown in SEQ ID NO:32; the sequences as shown in SEQ ID NO:294 to SEQ ID NO:301 are variants of the L2 fragment TGYIPLGTR as shown in SEQ ID NO:9; the sequences as shown in SEQ ID NO:302 to SEQ ID NO:348 are variants of the L2 fragment KTCKQXGTCPPDIIPKVEG as shown in SEQ ID NO:10; the sequences as shown in SEQ ID NO:349 to SEQ ID NO:381 are variants of the L2 fragment KTCKQXGTCPPD as shown in SEQ ID NO:11; the sequences as shown in SEQ ID NO: 382 to SEQ ID NO: 408 are variants of the L2 fragment TCKQXGTCPPD as shown in SEQ ID NO:12; the sequences as shown in SEQ ID NO: 409 to SEQ ID NO: 430 are variants of the L2 fragment CKQXGTCPPD as shown in SEQ ID NO:13; the sequences as shown in SEQ ID NO: 431 to SEQ ID NO: 457 are variants of the L2 fragment TCKQXGTCPP as shown in SEQ ID NO:14; the sequences as shown in SEQ ID NO:458 to SEQ ID NO:479 are variants of the L2 fragment CKQXGTCPP as shown in SEQ ID NO:15.

As mentioned above, the immunogenic polypeptide shall also comprise a linker peptide or more than one linker peptide. Said linker peptide, preferably, shall prevent the formation of junctional epitopes. Preferably, the linker peptide is positioned at the C- and/or N-Terminus of the L2 polypeptide, or of the fragment (or of the variant thereof). If the immunogenic polypeptide comprises more than one fragment of the L2 polypeptide (or more than one variant of said fragment), it is particularly contemplated that the immunogenic polypeptide comprises a linker peptide between the various fragments (or variants thereof). For example, SEQ ID NO:21 shows a multimer of L2 fragments with a GGP-linker (SEQ ID NO:16) inserted between any one of the L2 fragments.

Preferably, said linker has a length of 1 to 5 amino acids. The person skilled in the art knows how to select suitable linker peptides. Preferably, said 1 to 5 amino acids comprised by said linker peptide are selected from the group consisting of Glycine (G), Proline (P) or Serine (S). A particularly preferred linker peptide comprises the amino acid sequence GGP (SEQ ID NO: 16). However, also other linkers can be used such as GPGP (SEQ ID NO: 17), GPGPG (SEQ ID NO: 18), or SGSG (SEQ ID NO: 19). Preferably, said linker peptide is positioned at the junction of the scaffold polypeptide and the fragment of the L2 polypeptide and/or at the junction of two L2 fragments (or variants thereof). Thus, said linker peptide can be positioned either N-terminally or C-terminally from the L2 fragment (or variant thereof) or both.

A preferred multimer of a fragment of the L2 polypeptide comprised by the immunogenic polypeptide according to the invention has an amino acid sequence such as the one shown in SEQ ID NO: 20, or in SEQ ID NO: 21, or a sequence as shown in SEQ ID NO: 22.

Other preferred multimers are multimers comprising combinations of different homooligomers of fragments of the L2 polypeptide (e.g. a trimer of SEQ ID NO:2 linked to a trimer of SEQ ID NO:487 linked to a trimer of SEQ ID NO:487). More preferably, the L2 polypeptides comprised in said multimers are separated by linker sequences, see e.g. SEQ ID NO: 491. Also preferred are repeats of heterooligomers of fragments of the L2 polypeptide. A heterooligomer comprises e.g. SEQ ID NO:2 linked to SEQ ID NO:487 linked to SEQ ID NO:77, the corresponding multimer comprising e.g. said heterooligomer repeated three times. More preferably, the L2 polypeptides comprised in said multimers are separated by linker sequences, see e.g. SEQ ID NO:492.

The L2 polypeptide, or fragment thereof (or the variant of said L2 polypeptide or of the fragment thereof, or the corresponding multimers, see elsewhere herein) shall be comprised by a scaffold polypeptide which constrains the structure of the L2 polypeptide, or the fragment thereof (or the respective variants).

The term "constraining" as used herein, preferably, means that the L2 polypeptide, or the fragment thereof (or the respective variants) that is comprised by the scaffold protein is present in a conformation that mimics its natural conformation. Preferably, said L2 polypeptide, or the fragment thereof (or the respective variant) is kept by the scaffold polypeptide in a fixed conformation, when constrained.

Any scaffold polypeptide being capable of constraining the structure of said L2 polypeptide, or of the fragment of said L2 polypeptide, preferably, can be used for the production of the immunogenic polypeptide according to the present disclosure.

According to the present disclosure, the scaffold polypeptide is selected from the group consisting of thioredoxin, capsid polypeptides of adeno-associated viruses (e.g. AAV2, GenBank Accession No., NC_001401.2, GI:110645916; AAV8 GenBank Accession No., NC_006261.1, GI:51949963; AAV7 GenBank Accession No., NC_006260.1, GI:51949960), the tenth type III module of fibronectin (FN3, GenBank Accession No. 1TTF_A; GI:157834026, with insertion of the L2 polypeptide, fragment or variant thereof within the exposed PAVTVR (SEQ ID NO: 480) or GRGDSPASS (SEQ ID NO: 481) loop sites), lipocalins (particularly, the bilin-binding protein from Pieris brassicae, GenBank Accession No. CAA54063.1, GI:434995, with insertion of the L2 polypeptide, fragment or variant thereof within the PNSVEKY (SEQ ID NO: 482), IHGKE (SEQ ID NO: 483), TYGGVTK (SEQ ID NO: 484) and/or YDEDKKGH loop sites), a catalytically inactive version of Staphylococcus nuclease (e.g., GenBank Accession No. 2SNS_A, 2SNS_A, GI:157836360, with peptide insertion within the YKGQP (SEQ ID NO: 485) loop site); an alpha-amylase inhibitor, preferably tendamistat (GenBank Accession No. CAA00655.1, GI:413044, with peptide insertion within the EDD and/or IGSHG loop sites); or stefin A (GenBank Accession No. P01040.1, GI:118177, with insertion of the L2 polypeptide, fragment or variant thereof within the KSL loop site).

According to the present invention, the scaffold protein is a thioredoxin polypeptide.

Thioredoxin polypeptides are the major cellular disulfide redox components and serve as electron donors for enzymes such as ribonucleotide reductases, thioredoxin peroxidases and methionine sulfoxide reductases. Thioredoxins have an alpha/beta structure with two disulfide bondable cysteine residues. Thioredoxins are ubiquitous polypeptides and were shown to be present in most organisms (for a review see Arnér and Holmgren, Physiological functions of thioredoxin and thioredoxin reductase, European Journal of Biochemistry, Volume 267 Issue 20, Pages 6102 - 6109). The thioredoxin polypeptide in the context of the present invention may be derived from any organism. Preferably, the thioredoxin polypeptide comprises the so called thioredoxin display site CGPC (SEQ ID NO: 23). The thioredoxin display site, also known as thioredoxin motif or as dithiol/disulfide active site, is a highly conserved motif amongst thioredoxin polypeptides. Preferably, said thioredoxin polypeptide is selected from the group consisting of prokaryotic and eukaryotic thioredoxin polypeptides, or any other thioredoxin or thioredoxin-like protein, or proteins harbouring a thioredoxin (TRX) Pfam domain, bearing the conserved CGPC (SEQ ID NO: 23), or a CGXC, or a CXXC sequence motif (e.g., gi|40253454; gi|77456671; gi|31543902). More preferably, said thioredoxin polypeptide is selected from the group consisting of bacterial, animal and plant thioredoxin polypeptides Even more preferably, the thioredoxin polypeptide is a Escherichia coli thioredoxin as shown in SEQ ID NO: 24 (which shows 100% identity with the thioredoxin polypeptide of Salmonella typhi), or the homologous thioredoxin polypeptides from Salmonella enterica (SEQ ID NO: 25), mouse (SEQ ID NO: 26), rabbit (SEQ ID NO: 27), human (SEQ ID NO: 28), or any other thioredoxin or thioredoxin-like protein as shown in SEQ ID NO: 17. Also included are oligomers of said thioredoxin polypeptides, i.e. fusion polypeptides comprising at least two copies of thioredoxin polypeptides, e.g. dimers or trimers, wherein the C-terminus of one copy of a thioredoxin polypeptide is linked to the N-terminus of the following copy of a thioredoxin polypeptide. Preferably, at least one of the thioredoxin polypeptides comprises at least one L2 peptide inserted within the display site. More preferably, in said oligomers the thioredoxin polypeptides are separated by linker peptides, see e.g. SEQ ID NO:497 and SEQ ID NO: 498.

Preferably, the L2 polypeptide, or the fragment of said L2 polypeptide (or multimer or fragment thereof) is positioned within the so called "display site" of thioredoxin. Thus, the said L2 polypeptide or fragment thereof, preferably is positioned between the C and the G, or between the G and the P, or between the P and the C residues of the display site sequence CGPC (SEQ ID NO: 23) of the thioredoxin polypeptide. Also contemplated by the present invention is positioning the L2 polypeptide or fragment thereof adjacent to the display site, preferably, between any pair of amino acid residues located up to 20, up to 10, or up to 5 amino acid residues upstream or downstream from the display site.

In a preferred embodiment the thioredoxin polypeptide is selected from the group consisting of
a) a polypeptide having a sequence as shown in SEQ ID No: 24, SEQ ID No: 25, SEQ ID No: 26, SEQ ID No: 27, or SEQ ID No: 28 (or any other thioredoxin polypeptide as recited herein); and
b) a variant polypeptide having a sequence at least 70% identical to the sequence shown in SEQ ID No: 24, SEQ ID No: 25, SEQ ID No: 26, SEQ ID No: 27, or SEQ ID No: 28 (or any other thioredoxin polypeptide as recited herein),
wherein said polypeptide constrains the structure of the L2 polypeptide, or which constrains the structure of a fragment of said L2 polypeptide (or of a variant thereof).

As set forth above the thioredoxin polypeptide in the context of the present invention, preferably, shall comprise the thioredoxin display site.

In another preferred embodiment the thioredoxin polypeptide is derived from a thermophile bacterium. The use of a thioredoxin polypeptide from a thermophile bacterium allows for storage of the immunogenic polypeptides, e.g., at room temperature (instead of storing said polypeptide, e.g., at 4°C or at even lower temperatures). Storing the immunogenic polypeptide, e.g., at 20°C is, particularly, advantageous if said polypeptide is used as a vaccine since it allows the distribution of the polypeptide even in regions where cooling systems are not available.

Thermophile bacteria are known to grow at elevated temperatures (> 50°C), particularly in and/or around geothermal vents in marine or aquatic environments. A variety of termophile bacteria is known in the art. Preferred thermophile bacteria in the context of the present invention are Archaebacteria, particularly Methanosaeta thermophila, Archaeoglobus fulgidus, Metallosphaera sedula, Sulfolobus solfataricus, Sulfolobus tokodaii, Sulfolobus acidocaldarius, Metallosphaera sedula, Thermofilum pendens, Picrophilus torridus, Caldivirga maquilingensis. The amino acid sequence of thioredoxin polypeptides of a variety of thermophile bacteria is well known in the art. Preferred thiorexodin polypeptides derived from thermophile bacteria have an amino acid sequence as shown in GenBank-Accession Numbers: Methanosaeta thermophila (gi|116754023, YP_843141; gi|116754438, YP_843556); Archaeoglobus fulgidus (gi|11498883, NP_070112; gi|11499727, NP_070969); Metallosphaera sedula (gi|146304377, YP_001191693; gi|146303559, YP_001190875); Sulfolobus solfataricus (gi|15897303, NP_341908; gi|15899007, NP_343612); Sulfolobus tokodaii (gi|15922449, NP_378118; gi|15921676, NP_377345); Sulfolobus acidocaldarius (gi|70605894, YP_254764.1; gi|70607552, YP_256422.1; gi|70607229, YP_256099); Thermofilum pendens (gi|119720035, YP_920530); Picrophilus torridus (gi|48477193, YP_022899); Caldivirga maquilingensis (gi|159040636, YP_001539888). Also included are thioredoxin polypeptides from Pyrococcus furiosus (SEQ ID NO: 493), Thermococcus kodakarensis (SEQ ID NO: 494), Thermococcus onnurineus (SEQ ID NO: 495), and Thermococcus sibiricus (SEQ ID NO: 496).

In a preferred embodiment the immunogenic polypeptide further comprises a polypeptide that further stimulates (enhances) immunogenicity of said immunogenic polypeptides. Such polypeptides stimulating immunogenicity are well known in the art. Preferred stimulating polypeptides are C4bp (Complement component 4 binding protein) and MDC/CCL22 (Macrophage-Derived Chemokine_CC motif_ligand 22. It is to be understood that the immunogenic polypeptide and the stimulating polypeptide are fused in frame. Preferably, the stimulating polypeptide is fused to the N- or C-terminus of to the immunogenic polypeptide

Preferably, the immunogenic polypeptide according to the invention is a polypeptide having an amino acid sequence as shown in SEQ ID NO: 29, or SEQ ID NO: 30.

The immunogenic polypeptide as shown in SEQ ID NO:29 comprises a multimer of 3 of the L2 fragment having a sequence as shown in SEQ ID NO:2, said fragments being connected by a linker peptide having a sequence as shown in SEQ ID NO: 16.

The immunogenic polypeptide as shown in SEQ ID NO:30 comprises a multimer of 9 of the L2 fragment having a sequence as shown in SEQ ID NO:2, said fragments being connected by a linker peptide having a sequence as shown in SEQ ID NO:16.

The sequences as shown in SEQ ID NO: 29, and SEQ ID NO: 30 comprise two hexahistidine-tags for purification of said polypeptides. It is to be understood that these tag do not contribute to the immunogenicity of said polypeptide and, thus, can be omitted.

Advantageously, it was shown in the studies underlying the present disclosure that an immunogenic polypeptide comprising a scaffold polypeptide and a L2 polypeptide or a fragment thereof, wherein said scaffold protein constrains the structure of said polypeptide or of said fragment, confers strong immunogenicity and induces strong neutralizing responses against HPV16 as well as strong cross-neutralizing responses against other HPV genotypes such as HPV18, HPV31, HPV45 and HPV58. Particularly, it was shown that a thioredoxin polypeptide that comprises within its display site the L2 polypeptide or a fragment has a strong immunogenicity and allows for strong neutralizing as well as cross-neutralizing responses (see Examples). The immunogenicity and (cross-)neutralizing response was further enhanced when multimers of the L2 polypeptides or fragments thereof were inserted within the display site of the thioredoxin polypeptide (see Examples).

The immunogenic polypeptide according to the present invention is of advantage over prior art polypeptides, since the polypeptides as disclosed in prior art have a low immunogenicity or only induce strong but not cross-neutralizing responses. For example, L2 based peptides that are disclosed in the art are poorly immunogenic whereas L1 based peptides have a limited cross-protective capacity. Thus, the immunogenic polypeptide according to the present invention allows for the production of vaccines against a broad range of HPV genotypes, particularly high-risk HPV genotypes.

Moreover, the present invention relates to a polynucleotide encoding the immunogenic polypeptide according to the present invention.

The polynucleotides of the present invention may contain further nucleic acid sequences as well. Specifically, the polynucleotides of the present invention may encode fusion proteins wherein one partner of the fusion protein is a polypeptide being encoded by a nucleic acid sequence recited above. Such fusion proteins may comprise as additional part peptide sequences for monitoring expression (e.g., green, yellow, blue or red fluorescent proteins, alkaline phosphatase and the like) or so called "tags" which may serve as a detectable marker or as an auxiliary measure for purification purposes. Tags for the different purposes are well known in the art and comprise FLAG-tags, 6-histidine-tags, MYC-tags and the like.

The term "polynucleotide" as used herein refers to a linear or circular nucleic acid molecule. It encompasses DNA as well as RNA molecules. The polynucleotide of the present invention shall be provided, preferably, either as an isolated polynucleotide (i.e. isolated from its natural context) or in genetically modified form. The term encompasses single as well as double stranded polynucleotides. Moreover, comprised are also chemically modified polynucleotides including naturally occurring modified polynucleotides such as glycosylated or methylated polynucleotides or artificially modified derivatives such as biotinylated polynucleotides. The polynucleotide of the present invention is characterized in that it shall encode a polypeptide as referred to above. The polynucleotide, preferably, has a specific nucleotide sequence as mentioned above. Moreover, due to the degeneracy of the genetic code, polynucleotides are encompassed which encode a specific amino acid sequence as recited above.

Moreover, the term "polynucleotide" as used in accordance with the present invention further encompasses variants of the aforementioned specific polynucleotides. Said variants may represent orthologs, paralogs or other homologs of the polynucleotide of the present invention. The polynucleotide variants, preferably, comprise a nucleic acid sequence characterized in that the sequence can be derived from the aforementioned specific nucleic acid sequences by at least one nucleotide substitution, addition and/or deletion whereby the variant nucleic acid sequence shall still encode a polypeptide having a structure as specified in the claims. Variants also
encompass polynucleotides comprising a nucleic acid sequence which is capable of hybridizing to the aforementioned specific nucleic acid sequences, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6 × sodium chloride/sodium citrate (= SSC) at approximately 45°C, followed by one or more wash steps in 0.2 × SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5 × SSC (pH 7.2). If an organic solvent is present in the above mentioned buffer, for example 50% formamide, the temperature under standard conditions is approximately 42°C. The hybridization conditions for DNA:DNA hybrids are preferably for example 0.1 × SSC and 20°C to 45°C, preferably between 30°C and 45°C. The hybridization conditions for DNA:RNA hybrids are preferably, for example, 0.1 × SSC and 30°C to 55°C, preferably between 45°C and 55°C. The above mentioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. Alternatively, polynucleotide variants are obtainable by PCR-based techniques such as mixed oligonucleotide primer- based amplification of DNA, i.e. using degenerated primers against conserved domains of the polypeptides of the present invention. Conserved domains of the polypeptide of the present invention may be identified by a sequence comparison of the nucleic acid sequence of the polynucleotide or the amino acid sequence of the polypeptide of the present invention with sequences of other members of the enzyme families referred to in accordance with this invention.

Oligonucleotides suitable as PCR primers as well as suitable PCR conditions are described in the accompanying Examples. As a template, DNA or cDNA from bacteria, fungi, plants or animals may be used. Further, variants include polynucleotides comprising nucleic acid sequences which are at least 70%, at least 75%, at least 80%, at least 85%, at least 90%, at least 95%, at least 98% or at least 99% identical to the specific nucleic acid sequences. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the specific amino acid sequences referred to herein. The percent identity values are, preferably, calculated over the entire amino acid or nucleotide sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Higgins 1989, CABIOS, 5 1989: 151-153) or the programs Gap and BestFit (Needleman 1970, J. Mol. Biol. 48; 443-453 and Smith 198, Adv. Appl. Math. 2: 482-489), which are part of the GCG software packet from Genetics Computer Group, 575 Science Drive, Madison, Wisconsin, USA 53711, version 1991, are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

Moreover, the present invention relates to a vaccine comprising the immunogenic polypeptide according to the invention.

The term "vaccine" as used herein, preferably, relates to a composition which -when administered to an animal, preferably a human- elicits an immune response against various HPV genotypes. Thus, administering said vaccine would stimulate the immune system and establish or improve immunity to infection with various HPV genotypes. Preferably, the vaccine according to the present invention allows for establishing or improving immunity to infection with human papillomavirus genotypes 16, 18, 31, 45 and 58. Preferably, the vaccine according to the present invention also allows for establishing or improving immunity to infection with human papillomavirus genotypes 6, 52, 2, 27, 57 and/or 11. It is to be understood that the vaccine according to the present invention may comprise further components.

A preferred further component is an adjuvant. Adjuvants are compounds which may not elicit an immune response when administered to the host alone but which may further enhance the immune response of the host when administered together with the immunogenic polypeptides. It is known in the art that adjuvants may act as surfactants which promote concentration of immunogenic polypeptides over a large surface area, or may have immunostimulatory properties.

Preferred adjuvants in the context of the present invention are muramyl dipeptide, saponins such as QS21 and Quil A, monophosphoryl lipid A, mineral oil/surfactant mixtures (e.g., Montanide), aluminum hydroxide, aluminum phosphate, hydroxyapatite, complete and/or incomplete Freund's adjuvant, or cytokines such as interleukins, macrophage derived chemokines, complement binding proteins and tumor necrosis factor (either free or fused to the scaffold protein), and human use-approved live microbial carriers such as the live attenuated *Salmonella enterica* serovar Typhimurium strain.

Moreover the present disclosure relates to the use of the immunogenic polypeptide according the invention for the preparation of a vaccine for immunization of a subject against infection with HPV.

Preferably, said subject is an animal, more preferably, said subject is a vertebrate, even more preferably, said subject is a mammal and, most preferably, said subject is a human. Preferably, the immunization of said subject, establishes or improves immunity of said subject to various HPV genotypes as referred to elsewhere herein. It is to be understood that the immunogenic polypeptide according to the invention or vaccine according to the invention has to be administered to said subject for immunization. Said administration can be done by any method deemed appropriate such as oral or parenteral administration.

Moreover, the present disclosure relates to a method for producing an antibody against the immunogenic polypeptide according to the invention, comprising the following steps:
a) providing the immunogenic polypeptide according to the invention;
b) immunizing a host with said immunogenic polypeptide, and
c) harvesting the antibody against said immunogenic polypeptide.

Preferably, the host will be sacrificed after the method has been carried out. It is to be understood that such a method is not deemed to be a method of treatment of the human or animal body.

The "host" in the context may be any host deemed appropriate. Preferably, the host is a non-human host. Preferred host for the production of monoclonal antibodies is a mouse or a rabbit. A host for the production of polyclonal antibodies is preferably selected from the group consisting of rabbits, mice, chickens, goats, guinea pigs, hamsters, horses, rats, and sheep.

Antibodies against the immunogenic polypeptide according to the present invention can be prepared by well known methods using said immunogenic polypeptide as an antigen. Preferably, the produced antibody is a polyclonal antibody. More preferably, said antibody is a monoclonal antibody.

In a further disclosure, said monoclonal antibody is produced by the hybridoma cell line which has been deposited with the German Collection of Microorganisms and Cell Cultures (DSMZ), Braunschweig, Germany on November 27, 2008 under deposit number DSM ACC2983 according to the Budapest Treaty, or a fragment thereof (preferably, F(ab)2, F(ab')2, Fab, F (ab'), Dab, Fv, sFv,scFv, or Fc fragments), or said monoclonal antibody produced by the hybridoma cell line which has been deposited with the German Collection of Microorganisms and Cell Cultures (DSMZ), Braunschweig, Germany on November 27, 2008 under deposit number DSM ACC2984 according to the Budapest Treaty, or a fragment thereof (preferably, F(ab)2, F(ab')2, Fab, F (ab'), Dab, Fv, sFv,scFv, or Fc fragments). The monoclonal antibody produced by the hybridoma cell line which has been deposited with the German Collection of Microorganisms and Cell Cultures (DSMZ), Braunschweig, Germany on November 27, 2008 under deposit number DSM ACC2983 is herein also referred to as K4L2(20-38)4.1B. The monoclonal antibody produced by the hybridoma cell line which has been deposited with the German Collection of Microorganisms and Cell Cultures (DSMZ), Braunschweig, Germany on November 27, 2008 under deposit number DSM ACC2984 is herein also referred to as K18L2(20-38)XIII.5G.

It is also contemplated by the present disclosure that the antibody is a single chain antibody, a recombinant, human or humanized antibody or primatized, chimerized or a fragment of the antibody according to the present invention.

Also comprised by the aforementioned method of the present disclosure is the production of a synthetic antibody, an antibody fragment, such as F(ab)2, F(ab')2, Fab, F (ab'), Dab, Fv, sFv, scFv, or Fc fragments etc., or a chemically modified derivative of any of these. The antibody may belong to any immunoglobulin class, including IgM, IgG, IgD, IgE, IgA, or subclasses of IgG (such as IgG1, IgG2, IgG2, IgG2a, IgG2b, IgG3 or IgGM).

How to produce and harvest the aforementioned antibodies and fragments is well known in the art. Antibodies or fragments thereof can be obtained by using methods which are described, e.g., in Harlow and Lane "Antibodies, A Laboratory Manual", CSH Press, Cold Spring Harbor, 1988. Monoclonal antibodies can be prepared by the techniques originally described in Köhler and Milstein, Nature 256 (1975), 495, and Galfré, Meth. Enzymol. 73 (1981), 3, which comprise the fusion of mouse myeloma cells to spleen cells derived from immunized mammals. It is also contemplated that monoclonal antibodies are produced by fusing myeloma cells with the B-cells from rabbits that have been immunized with the desired antigen.

It is to be understood that the antibody produced by the aforementioned method shall specifically bind the immunogenic polypeptide according to the invention. Specific binding can be tested by various well known techniques. Preferably, the antibody produced by the aforementioned method shall specifically bind the L2 polypeptide or fragment thereof. More preferably, said antibody shall specifically bind the L2 polypeptide or fragment thereof, when comprised by the immunogenic polypeptide according to the present invention (linked to the scaffold polypeptide), and thus when being present in a constrained structure. Thus, the antibody according to the present disclosure shall not specifically bind the parts of the immunogenic polypeptide that are derived from the scaffold polypeptide.

The aforementioned method disclosed herein, preferably, allows for the production of an antibody against human papillomavirus. Preferably, said antibody binds the L2 polypeptide or fragments thereof of various HPV genotypes. Preferably, said antibody binds the L2 polypeptide or fragment thereof of HPV genotypes 16, 18, 31, 45 and 58. Preferably, the said antibody also binds the L2 polypeptide or fragments thereof of HPV genotypes 52, 2, 27, 57 and/or 11.

The present disclosure relates also to an antibody obtainable/produced by the aforementioned method of the present invention.

Said antibody of the present disclosure, preferably is a polyclonal antibody and, more preferably, a monoclonal antibody.

Most preferably, the antibody according to the present disclosure is the monoclonal antibody K4L2(20-38)4.1B (see Examples) produced by the hybridoma cell line which has been deposited with the German Collection of Microorganisms and Cell Cultures (DSMZ), Braunschweig, Germany on November 27, 2008 under deposit number DSM ACC2983 according to the Budapest Treaty, or a fragment thereof (preferably, F(ab)2, F(ab')2, Fab, F (ab'), Dab, Fv, sFv,scFv, or Fc fragments), or the antibody according to the present invention is the monoclonal antibody K18L2(20-38)XIII.5G (see Examples), which has been deposited with the German Collection of Microorganisms and Cell Cultures (DSMZ), Braunschweig, Germany on November 27, 2008 under deposit number DSM ACC2984 according to the Budapest Treaty, or a fragment thereof (preferably, F(ab)2, F(ab')2, Fab, F (ab'), Dab, Fv, sFv,scFv, or Fc fragments).

The antibodies according to the present disclosure can be used, for example, for the immunoprecipitation and immunolocalization of the immunogenic polypeptides of the present invention as well as for monitoring the presence of said variant polypeptides; for example, for the diagnosis of HPV infection, particularly for diagnosing infection with HPV genotypes 16, 18, 31, 45 and/or 58. Preferably, said diagnosis is done by determining the amount (or presence) of the L2 polypeptide in a biological sample from a subject suspected to be infected with HPV genotype 16, 18, 31, 45 and/or 58 (e.g. in a Pap smear). The presence of the L2 polypeptide (or increased amounts of the L2 polypeptide compared with a reference amount, e.g. the amount of said polypeptide in a sample from a subject not infected with HPV) indicates infection with HPV, whereas the absence of the L2 polypeptide (or decreased amounts of the L2 polypeptide compared with a reference amount, e.g. the amount of said polypeptide in a sample of a subject not infected with HPV) indicates that said subject is not infected with HPV.

Moreover, the antibodies according to the present disclosure can be used for the preparation of a pharmaceutical composition for passive immunization against various HPV genotypes, particularly against HPV genotypes 16, 18, 31, 45 and/or 58. For passive immunization, the antibody according to the present disclosure is administered to a subject in order to protect said subject against infection with various HPV genotypes and/or to treat an existing HPV infection, particularly infection with HPV genotypes 16, 18, 31, 45 or 58.
Also, the antibody of the present disclosure can be used for the production of anti-idiotypic antibodies. An "anti-idiotypic antibody" in the context of the present disclosure is an antibody that specifically binds to the idiotypic region of the antibody according to the present disclosure, or a fragment thereof. The idiotypic region of the antibody according to the present disclosure (or a fragment thereof) is, preferably, the unique part of its variable region that specifically binds to the immunogenic polypeptide according to the present disclosure. Preferably, the anti-idiotypic antibody is a monoclonal antibody.

Anti-idiotypic antibodies as well as methods for their production are well known in the art, see, e.g., US20080127359, or US Patent 5792455; Dalgleish: An anti-idiotype vaccine for AIDS based on the HIV receptor. Ann Ist Super Sanita. 1991;27(1):27-31, or Attanasio, Int Rev Immunol. 1990;7(1):109-19.

Preferably, said anti-idiotypic antibodies are produced by a) providing an antibody according to the present disclosure (preferably, a monoclonal antibody according to the invention, more preferably, K4L2(20-38)4.1B, or a fragment thereof, or K18L2(20-38)XIII.5G, or a fragment thereof), b) immunizing a host with said antibody, and c) harvesting the resulting anti-idiotypic antibody.

Accordingly, the present disclosure also relates to a method for producing anti-idiotypic antibodies by carrying out the aforementioned steps a) and b).

Moreover, the present disclosure relates to the use of the hybridoma cell line which has been deposited with the German Collection of Microorganisms and Cell Cultures (DSMZ), Braunschweig, Germany on November 27, 2008 under deposit number DSM ACC2983, and to the use of the hybridoma cell line which has been deposited with the German Collection of Microorganisms and Cell Cultures (DSMZ), Braunschweig, Germany on November 27, 2008 under deposit number DSM ACC2984 for the production of a monoclonal antibody that specifically binds the L2 peptide (as described herein).

Finally, the present disclosure also relates to the hybridoma cell line which has been deposited with the German Collection of Microorganisms and Cell Cultures (DSMZ), Braunschweig, Germany on November 27, 2008 under deposit number DSM ACC2983 or under deposit number DSM ACC2984 according to the Budapest Treaty.

The Figures show:

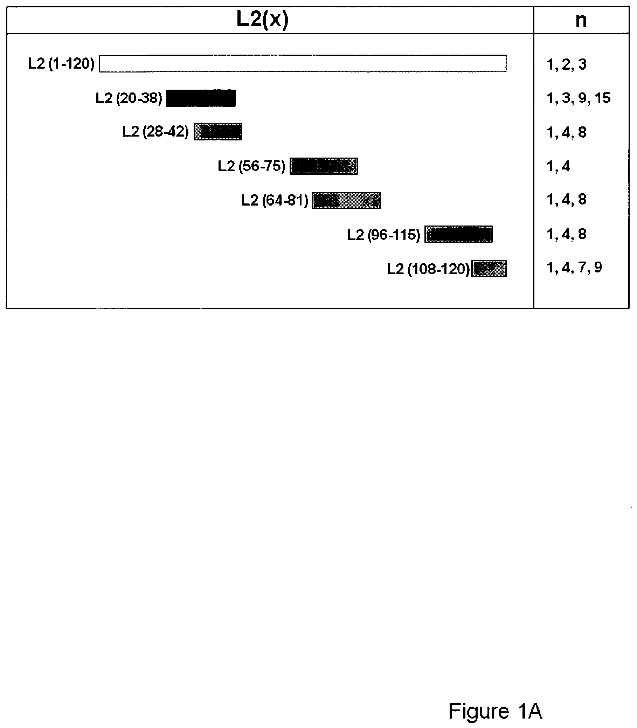

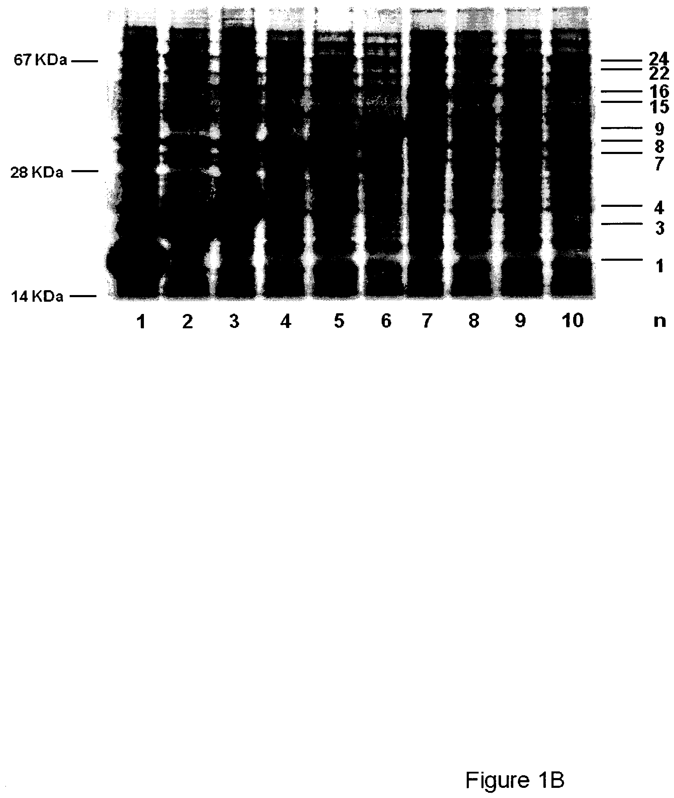

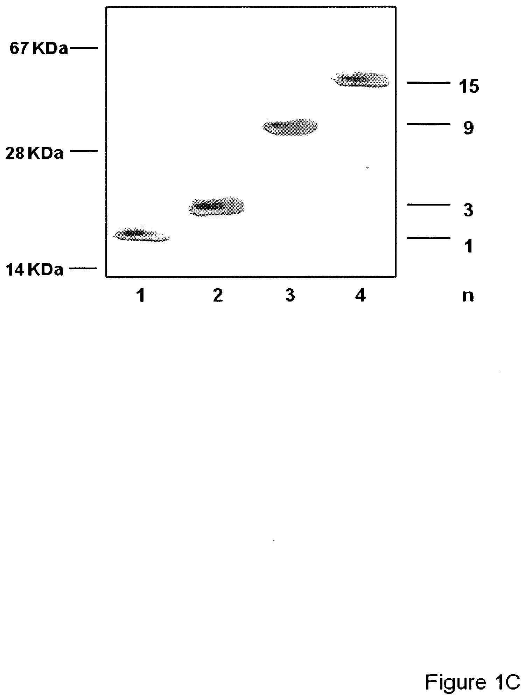

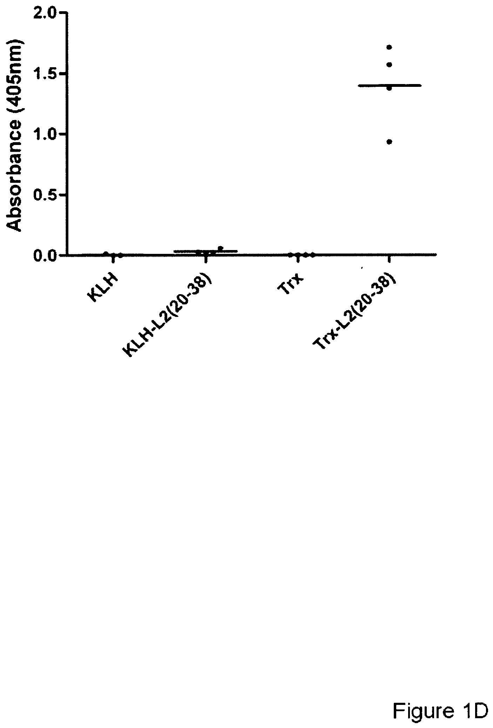

**Figure 1****. Trx-L2 peptides.** (A) Schematic representation of the HPV16 L2 peptides examined in this study. L2 aa 1-120 1x (1x SEQ ID NO:1 + 1x SEQ ID NO:24, thus the L2 polypeptide having a sequence as shown in SEQ ID NO1 inserted within the display site of the thioredoxin polypeptide having a sequence as shown in SEQ ID NO:24), 2x (SEQ ID NO:1 (2x) + SEQ ID NO:16(1x) + SEQ ID NO:24, thus two fragments of the L2 polypeptide, said fragments having a sequence as shown in SEQ ID NO:2, said fragments linked via one linker peptide having a sequence as shown in SEQ ID NO:16, inserted within the display site of the thioredoxin polypeptide having a sequence as shown in SEQ ID NO:24), 3x (SEQ ID NO:1 (3x)+ SEQ ID NO:16(2x) + SEQ ID NO:24); L2 aa 20-38 1x (SEQ IS NO:2 + SEQ ID NO:24), 3x(SEQ ID NO:29), 9x(SEQ ID NO:30), 15X (SEQ IS NO:2 (15x) + SEQ ID NO:16 (14x)+ SEQ ID NO:24); L2 aa 28-42 1x, 4x, 8x; L2 aa 56-75 1x, 4x; L2 aa 64-81 1x, 4x, 8x; L2 aa 95-115 1x, 4x, 8x. (B) Representative example of the expression analysis of pTrx-L2(20-38)n constructs with varying L2 peptide insert multiplicity (n). SDS-PAGE of total bacterial lysates from different clones ordered according to the multiplicity of their L2(20-38) peptide inserts; the peptide insert multiplicity (n) of the various fusion proteins and the migration positions of molecular mass markers are indicated on the right hand side and on the left hand side, respectively. (C) Representative examples of purified Trx-L2(20-38)n fusion proteins used for mice immunization (1) L2 aa 20-38 1x (SEQ IS NO:2 + SEQ ID NO:24), 3x(SEQ ID NO:29), 9x(SEQ ID NO:30), 15X (SEQ IS NO:2 (15x) + SEQ ID NO:16 (14x)+ SEQ ID NO:24). (D) Comparison of the immune responses elicited by the HPV16 L2(20-38) peptide chemically conjugated to KLH and by the same peptide grafted to Trx, both administered (100 µg/dose) with the same CFA/IFA immunization protocol described in 'Materials and methods'. ELISA data, obtained using GST-L2 as target antigen and expressed as A405 values, are presented as dot plots. The L2 binding activity of individual sera as well as the mean binding activity of each group (horizontal bars) are shown; KLH and Trx indicate the unconjugated carrier proteins utilized as negative controls. Please note that the numbers correspond to the amino acid positions of the L2 polypeptide as shown in SEQ ID NO:1. E.g., "L2(20-38)" stands for a fragment of the L2 polypeptide as shown in SEQ ID NO:1 comprising amino acids 20 to 28 of said polypeptide.

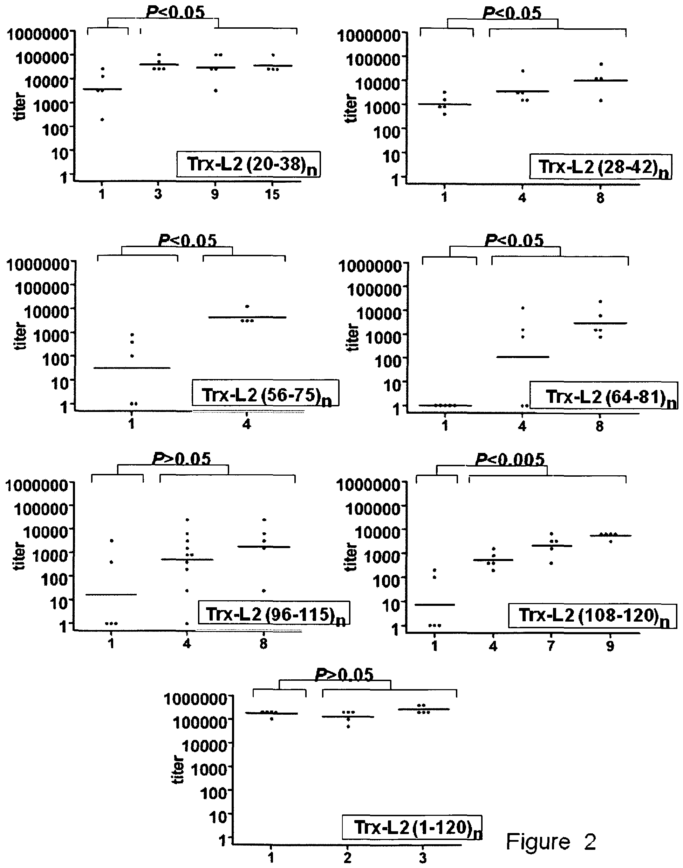

**Figure 2****. Antibody titers of mice vaccinated with the Trx-L2 peptide fusions.** GST-L2 ELISA was used to determine the antibody titers of sera from mice immunized three times with the indicated Trx-L2(peptide)n fusions, for group L2 aa 20-38 1x (SEQ IS NO:2 + SEQ ID NO:24), 3x(SEQ ID NO:29), 9x(SEQ ID NO:30), 15X (SEQ IS NO:2 (15x) + SEQ ID NO:16 (14x)+ SEQ ID NO:24) and group Trx-L2 L2 aa 1-120 1x (1x SEQ ID NO:1 +1x SEQ ID NO:24), 2x (SEQ ID NO:1 (2x)+ SEQ ID NO:16(1x)+ SEQ ID NO:24), 3x (SEQ ID NO:1 (3x)+ SEQ ID NO:16(2x)+ SEQ ID NO:24); L2 aa 28-42 1x, 4x, 8x; L2 aa 56-75 1x, 4x; L2 aa 64-81 1x, 4x, 8x; L2 aa 95-115 1x, 4x, 8x (100 µg each, corresponding to 1.7-4.3 nmol of protein, depending on the size of the peptide insert; n values are shown on the x-axis) administered with the CFA/IFA immunization protocol described in 'Materials and methods'. Sera from mice immunized with the Trx protein scaffold only (not shown) were used as negative controls and were assayed in parallel in each set of ELISAs. Binding titers are given as the reciprocal of the maximum antisera dilutions that yielded A405 values higher than the mean absorbance plus four standard deviations of sera from mice immunized with the Trx scaffold only. Data are presented as log10 dot-plots of the titers; horizontal bars represent the geometric mean of the titers for each of the indicated subgroups of Trx-L2 antisera. The P values in each panel indicate the statistical significance of the differences between the immune responses induced by monopeptide and multipeptide Trx-L2 fusions in each group.

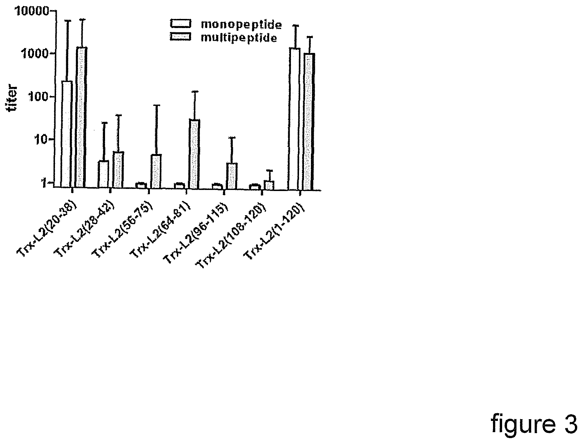

**Figure 3****. Neutralization of HPV 16 infection by Trx-L2 peptide antisera.** Serial dilutions of antisera raised against the monopeptide and multipeptide Trx-L2 fusions shown in Fig. 2 were analyzed for their capacity to block infection of 293TT cells by HPV16 pseudovirions using secreted alkaline phosphatase (SEAP) activity as readout. Neutralization efficiency was determined relative to that of mock-treated (0% neutralization) and HPV16 L1-specific mAb-treated (100% neutralization) controls, which were run in parallel in each assay (see 'Materials and methods' for details). Data for the monopeptide and the aggregated multipeptide forms of each Trx-L2 immunogen L2 aa 20-38 1x (SEQ IS NO:2 + SEQ ID NO:24), 3x(SEQ ID NO:29), 9x(SEQ ID NO:30), 15X (SEQ IS NO:2 (15x) + SEQ ID NO:16 (14x)+ SEQ ID NO:24); L2 aa 28-42 1x, 4x, 8x; L2 aa 56-75 1x, 4x; L2 aa 64-81 1x, 4x, 8x and L2 aa 95-115 1x, 4x, 8x are expressed as the reciprocal of the maximum dilution causing ≥70% neutralization. The geometric means of the titers and the 95% confidence intervals for each group of anti-Trx-L2 peptide antisera plus the anti-Trx-L2(1-120) 1x (1x SEQ ID NO:1 +1x SEQ ID NO:24), 2x (SEQ ID NO:1 (2x)+ SEQ ID NO:16(1x)+ SEQ ID NO:24), 3x (SEQ ID NO:1 (3x)+ SEQ ID NO:16(2x)+ SEQ ID NO:24) reference are represented on a log scale.

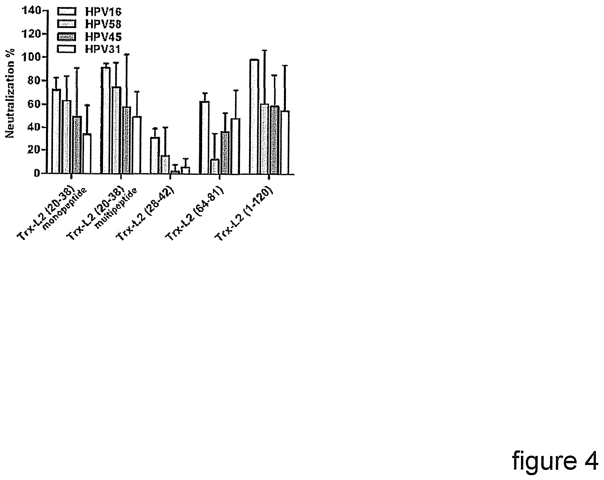

**Figure 4****. Cross-neutralization of HPV 31, 45, and 58 pseudovirions.** The crossneutralization activities of the indicated subset of Trx-L2 peptide antisera were assayed at a fixed 1:200 dilution against three heterologous pseudovirions (HPV 31, 45 and 58) plus the cognate HPV16 type. Mock-treated 293TT cells and cells treated with type-specific neutralizing antibodies served as negative and positive controls, respectively (see Fig. 3) legend and 'Materials and methods' for details). Cumulative monopeptide and multipeptide data are presented for each immunogen except Trx-L2 aa 20-38 1x (SEQ IS NO:2 + SEQ ID NO:24), 3x(SEQ ID NO:29), 9x(SEQ ID NO:30), 15X (SEQ IS NO:2 (15x) + SEQ ID NO:16 (14x)+ SEQ ID NO:24), the only one for which a trend toward a peptide multiplicity-dependent increase in cross-neutralization activity was observed; they are represented as the mean plus SD of the neutralization values for the various Trx-L2 peptide antisera relative to those obtained with HPV type-specific antibodies.

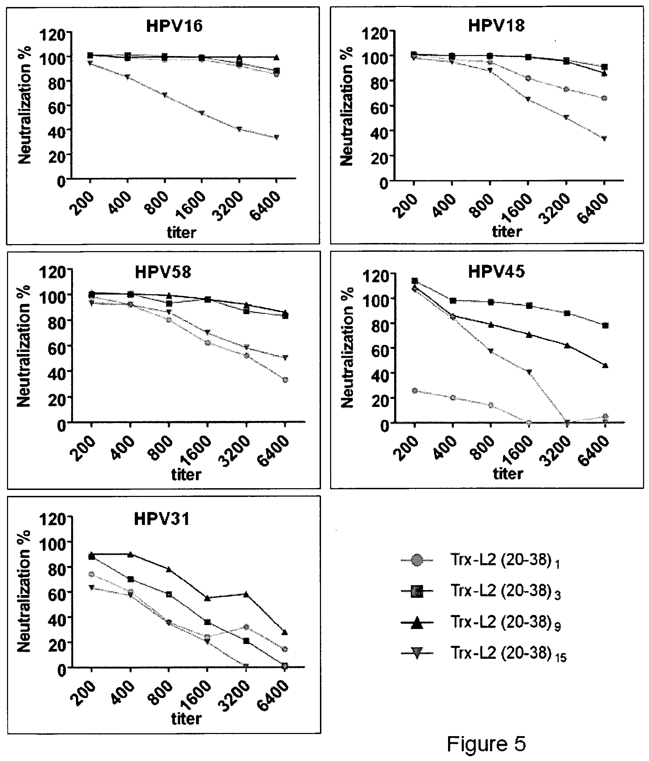

**Figure 5****. Neutralization of homologous and heterologous pseudovirions by Trx-L2(20-38)n antisera.** The strongest HPV 16 neutralizing antisera from each group of Trx-L2(20-38)n antigens (n = 1, 3, 9, 15) 1x (SEQ IS NO:2 + SEQ ID NO:24), 3x(SEQ ID NO:29), 9x(SEQ ID NO:30), 15X (SEQ IS NO:2 (15x) + SEQ ID NO:16 (14x)+ SEQ ID NO:24) were titrated against homologous (HPV16) and heterologous (HPV18, 31, 45, 58) pseudovirions (see 'Materials and methods' and Fig. 3 legend for details).

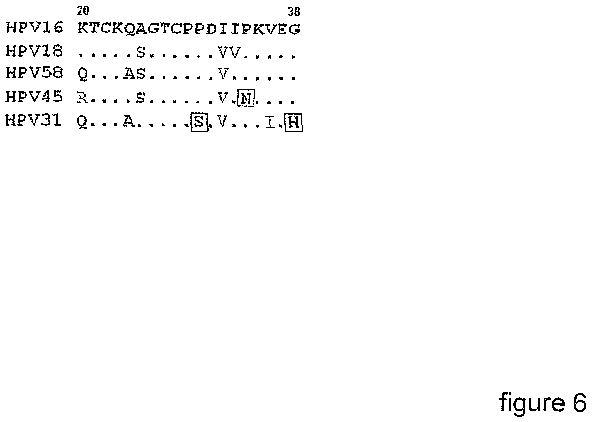

**Figure 6****. Sequence comparison of the L2(20-38) region of the examined HPV types.** Multiple sequence alignment was performed with CLUSTAL W [30]; amino acids identical to those of the cognate HPV16 type are indicated with dots. Conservative and non-conservative substitutions are shown in standard and in bold characters, respectively; non-conservative substitutions occurring in only one of the five examined HPV types are boxed.

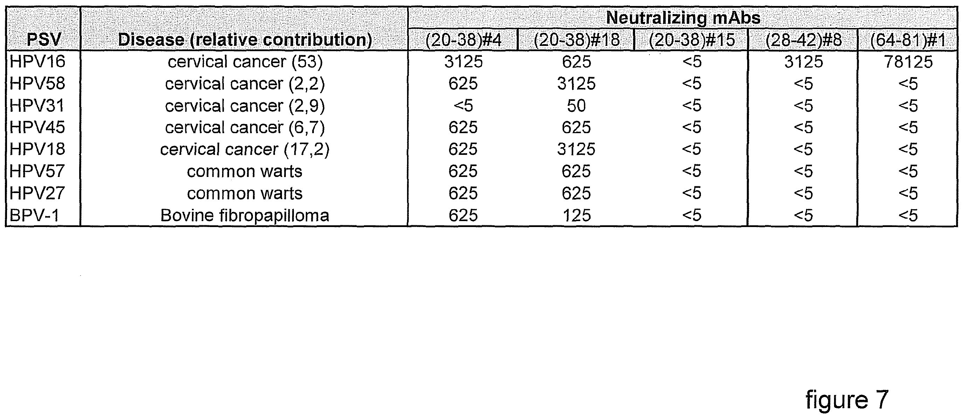

**Figure 7****. Neutralizing titers of supernatants of monoclonal antibodies against the aa 20-31 from HPV16 L2 protein**
   IgG concentration in supernatants was adjusted to 0,6 µg/ml, titer was defined as the last dilution that can protect 70% of pseudovirions infection. There are not big differences in the neutralization capacity of antibodies #4 (K4L2(20-38)4.1B) and #18 (K18L2(20-38)XIII.5G) except for the neutralization of HPV31. Antibody #18 can neutralize the infection although with low titer, but, antibody #4 is unable to neutralize the infection even at low dilution factor. Antibody #8 and #1 can neutralize only HPV16. Antibody #1 can neutralize HPV16 with high titer.

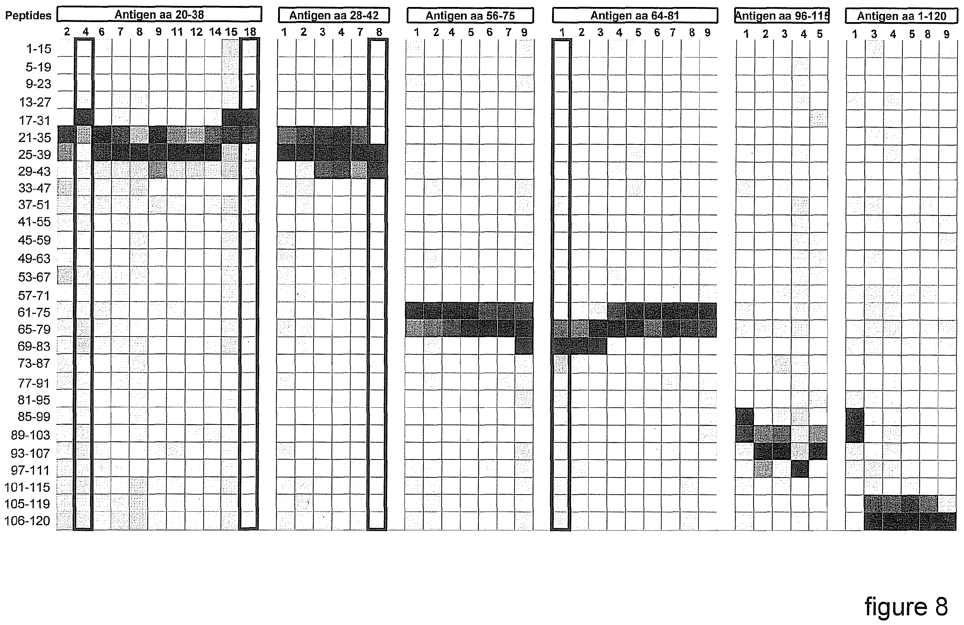

**Figure 8****. Identification of epitopes recognized by neutralizing (boxed) and non-neutralizing antibodies**
   The different monoclonal antibodies raised against different regions of the HPV 16 N-terminus were tested for their reactivity with a set of overlapping peptides (amino acids 1-15, 5-19, 106-120) in ELISA. All four neutralizing antibodies show a distinct pattern in binding the peptides, different to the pattern of the non-neutralizing antibodies. The two cross-neutralizing antibodies (K4L2(20-38)4.1B) and #18 (K18L2(20-38)XIII.5G) are directed against region 20-38. Antibody #15, which shows a similar binding pattern compared to #18, has an about 30 fold lower affinity to its target which explains its failure to neutralize HPV pseudovirions.

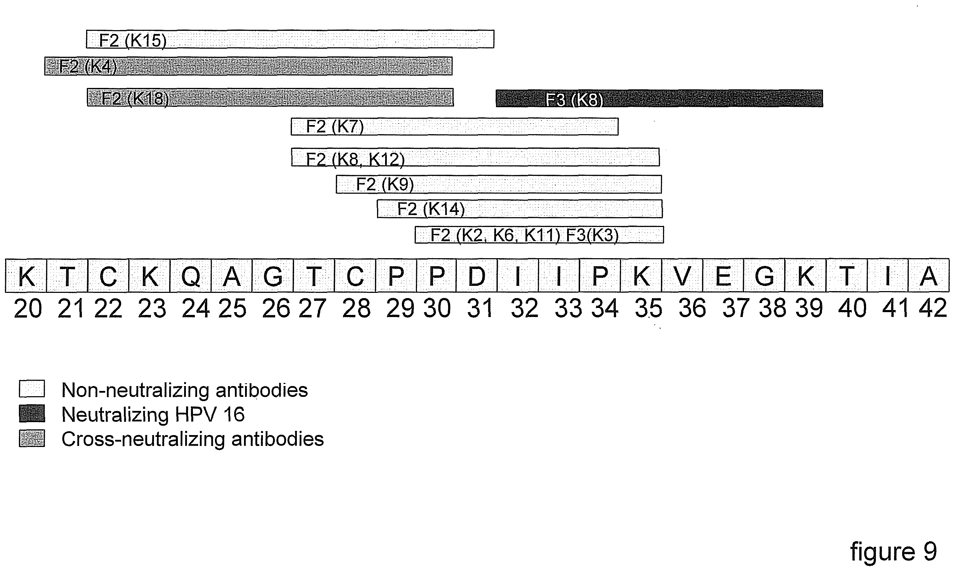

**Figure 9****. Epitopes for non-neutralizing, neutralizing and cross-neutralizing antibodies (K4L2(20-38)4.1B and K18L2(20-38)XIII.5G) within region 20-42 of HPV 16 L2.** Scheme with the recognition patron of all mAbs isolated against the region 20-42. Cross-neutralizing antibodies Mab K4L2(20-38)4.1B recognize the sequence aa 21-30 SEQ ID NO:4 and K18L2(20-38)XIII.5G recognize the sequence aa 22-30 SEQ ID NO:5. Neutralizing antibody anti HPV16 K8L2(28-42)12.4B recognize the sequence aa 32-39 SEQ ID NO:31.

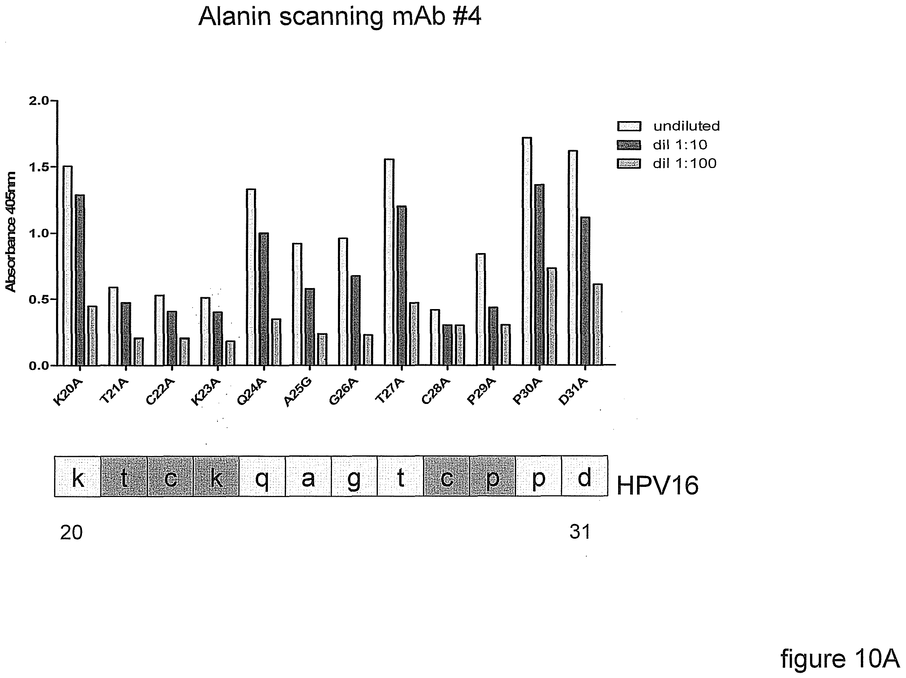

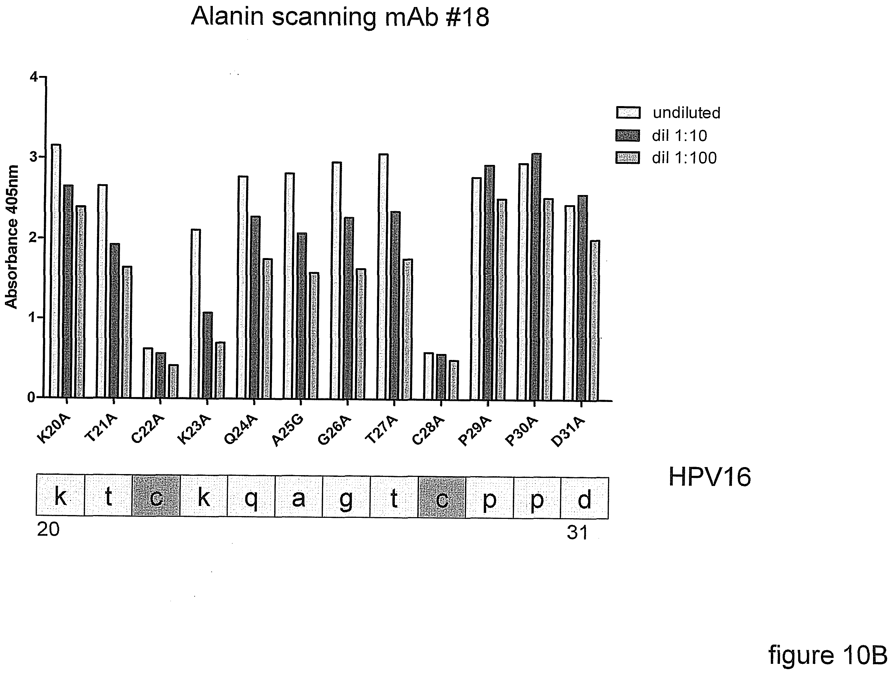

**Figure 10****. Epitope mapping for the two neutralizing antibodies (K4L2(20-38)4.1B and #18 (K18L2(20-38)XIII.5G).** To determine the amino acids required for binding of the two antibodies K4 (A9 and K18 (B) an peptide-alanine scan was performed. Antibody #4 the five amino acids xTCKxxxxCPxx are essential for binding while for K8 only the two cysteine residues are crucial for binding, although the remainder residues might contribute to the binding.

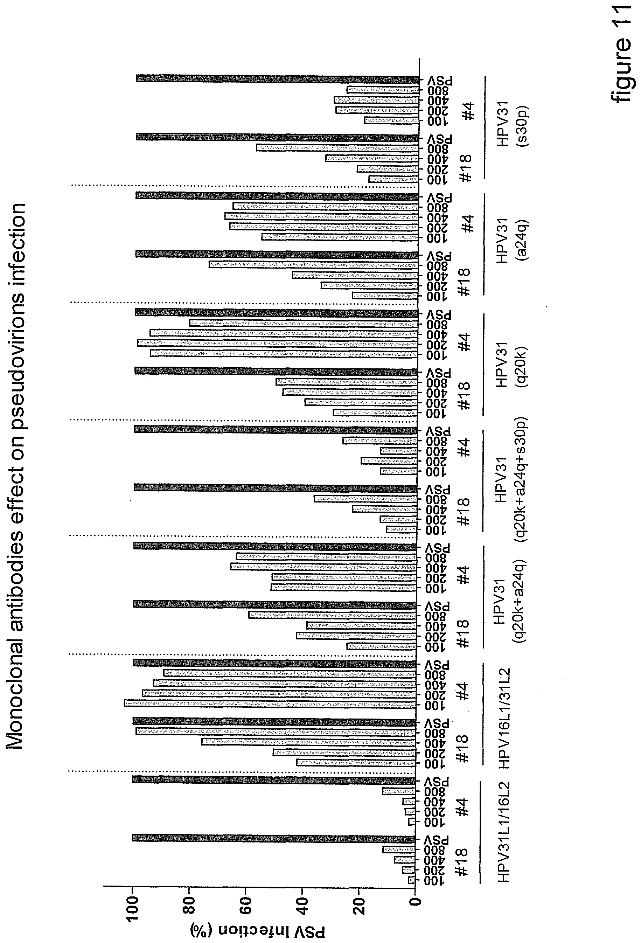

**Figure 11****. Neutralization assay of HPV 16 and HPV 31 pseudovirions with modified L2 proteins.** To determine why antibodies #4 (K4L2(20-38)4.1B) and #18 (K18L2(20-38)XIII.5G) have different abilities to neutralize HPV 31 we tested hybrid particles composed of HPV 16 L1 HPV 31 L2 and vice versa. In addition, the corresponding epitope in HPV 31 recognized by K4 and K18 was modified. Results indicate that the ability of (K4L2(20-38)4.1B) and #18 (K18L2(20-38)XIII.5G) antibodies to neutralize depends on the epitope sequence as HPV 31 L1/16L2 pseudovirions can be neutralized by both antibodies. Altering serine at position 30 into proline restores the ability to neutralize HPV 31 pseudovirions indicating that this residue is important in binding the antibodies.

### Examples

### Example 1

Monopeptide (1 x SEQ ID NO:2) and multipeptide ((SEQ ID NO:2 (3x)+ SEQ ID NO:16(2x)) or 3x (SEQ ID NO:2 (3x)+ SEQ ID NO:16(2x)) immunogenic peptides are inserted within the display site of the thioredoxin polypeptide having a sequence as shown in SEQ ID NO: 493 to SEQ ID NO: 496. Fusion proteins are produced in *E. coli* cells, purified from cell extracts and used for immunization.

**Table 1 List of L2 peptide immunogens (and variants thereof)_**

| | |
|---|---|
| **SEQ ID NO: 33** | RGCKQAGTCPPDVINKVEQ |
| SEQ ID NO: 34 | RGCKASNTCPPDVINKVEQ |
| SEQ ID NO: 35 | RGCKAAGTCPPDVINKVEQ |
| SEQ ID NO: 36 | QSCKAAGTCPPDVLNKVEQ |
| SEQ ID NO: 37 | QSCKAAGTCPPDVVNKVEQ |
| SEQ ID NO: 38 | QTCKQAGTCPPDVINKVEQ |
| SEQ ID NO: 39 | QTCKQAGTCPPDVVNKVEQ |
| SEQ ID NO: 40 | RTCKQAGTCPPDVINKVES |
| SEQ ID NO: 41 | RTCKQAGTCPPDVINKVEQ |
| SEQ ID NO: 42 | KGCKASGTCPPDVINKVEQ |
| SEQ ID NO: 43 | RTCKQSGTCPPDVVPKVEG |
| SEQ ID NO: 44 | RTCKQAGTCPPDVIPKVEG |
| SEQ ID NO: 45 | RTCKVTGTCPADVVPKVEG |
| SEQ ID NO: 46 | RTCKATGTRPADVIPKVEG |
| SEQ ID NO: 47 | RTCKQSGTCPPDIIPRVEQ |
| SEQ ID NO: 48 | RTCKQAGTCPPDIIPRLEQ |
| SEQ ID NO: 49 | RTCKQAGTCPPDIIPRVEQ |
| SEQ ID NO: 50 | KTCKVAGTCPPDVIPKVEG |
| SEQ ID NO: 51 | KTCKAAGTCPPDVIPKVEG |
| SEQ ID NO: 52 | RTCKAAGTCPPDVIPKVEG |
| SEQ ID NO: 53 | RTCKASGTCPPDVIPKVEG |
| SEQ ID NO: 54 | STCKAAGTCPADVIPKVEG |
| SEQ ID NO: 55 | KTCKLSGTCPEDVINKVEQ |
| SEQ ID NO: 56 | KTCKQSGTCPPDIIPKVEG |
| SEQ ID NO: 57 | KTCKQAGTCPPDIVPKVEG |
| SEQ ID NO: 58 | QTCKASGTCPPDVIPKVEG |
| SEQ ID NO: 59 | KTCKQAGTCPPDVIPKVEG |
| SEQ ID NO: 60 | QTCKAAGTCPSDIIPKVEH |
| SEQ ID NO: 61 | QTCKASGTCPPDVIPKVEQ |
| SEQ ID NO: 62 | QTCKLTGTCPPDVIPKVEH |
| SEQ ID NO: 63 | QTCKAAGTCPSDVINKVEH |
| SEQ ID NO: 64 | KQCQLGADCPPDVRNKVEG |
| SEQ ID NO: 65 | AKCQLSGNCLPDVKNKVEA |
| SEQ ID NO: 66 | AKCQLSGDCLPDVKNKVEA |
| SEQ ID NO: 67 | RHCALSGTCPDDVKNKVEN |
| SEQ ID NO: 68 | KHCAGSGTCPEDVKNKVEQ |
| SEQ ID NO: 69 | KTCLQGGDCIPDVKNKFEN |
| SEQ ID NO: 70 | RSCLQGGDCIPDVQNKFEG |
| SEQ ID NO: 71 | QTCKATGTCPPDVIPKVEG |
| SEQ ID NO: 72 | KTCKQSGTCPPDVVPKVEG |
| SEQ ID NO: 73 | RTCKQSGTCPPDVINKVEG |
| SEQ ID NO: 74 | KTCKQAGTCPSDVINKVEG |
| SEQ ID NO: 75 | KTCKLSGTCPEDVVNKIEQ |
| SEQ ID NO: 76 | RTCKQSGTCPPDVVDKVEG |
| SEQ ID NO: 77 | STCKAAGTCPPDVVNKVEG |
| SEQ ID NO: 78 | PTCKIAGNCPADIQNKFEN |
| SEQ ID NO: 79 | PACKISNTCPPDIINKYEN |
| | |
| **SEQ ID NO: 80** | RGCKQAGTCPPD |
| SEQ ID NO: 81 | RGCKASNTCPPD |
| SEQ ID NO: 82 | RGCKAAGTCPPD |
| SEQ ID NO: 83 | QSCKAAGTCPPD |
| SEQ ID NO: 84 | QTCKQAGTCPPD |
| SEQ ID NO: 85 | RTCKQAGTCPPD |
| SEQ ID NO: 86 | KGCKASGTCPPD |
| SEQ ID NO: 87 | RTCKQSGTCPPD |
| SEQ ID NO: 88 | RTCKVTGTCPAD |
| SEQ ID NO: 89 | RTCKATGTRPAD |
| SEQ ID NO: 90 | KTCKVAGTCPPD |
| SEQ ID NO: 91 | KTCKAAGTCPPD |
| SEQ ID NO: 92 | RTCKAAGTCPPD |
| SEQ ID NO: 93 | RTCKASGTCPPD |
| SEQ ID NO: 94 | STCKAAGTCPAD |
| SEQ ID NO: 95 | KTCKLSGTCPED |
| SEQ ID NO: 96 | KTCKQAGTCPED |
| SEQ ID NO: 97 | QTCKASGTCPPD |
| SEQ ID NO: 98 | QTCKAAGTCPSD |
| SEQ ID NO: 99 | QTCKLTGTCPPD |
| SEQ ID NO: 100 | KQCQLGADCPPD |
| SEQ ID NO: 101 | AKCQLSGNCLPD |
| SEQ ID NO: 102 | AKCQLSGDCLPD |
| SEQ ID NO: 103 | RHCALSGTCPDD |
| SEQ ID NO: 104 | KHCAGSGTCPED |
| SEQ ID NO: 105 | KTCLQGGDCIPD |
| SEQ ID NO: 106 | RSCLQGGDCIPD |
| SEQ ID NO: 107 | QTCKATGTCPPD |
| SEQ ID NO: 108 | KTCKQSGTCPPD |
| SEQ ID NO: 109 | KTCKQAGTCPSD |
| SEQ ID NO: 110 | STCKAAGTCPPD |
| SEQ ID NO: 111 | PTCKIAGNCPAD |
| SEQ ID NO: 112 | PACKISNTCPPD |
| | |
| **SEQ ID NO: 113** | GCKQAGTCPPD |
| SEQ ID NO: 114 | GCKASNTCPPD |
| SEQ ID NO: 115 | GCKAAGTCPPD |
| SEQ ID NO: 116 | SCKAAGTCPPD |
| SEQ ID NO: 117 | TCKQSGTCPSD |
| SEQ ID NO: 118 | GCKASGTCPPD |
| SEQ ID NO: 119 | TCKQSGTCPPD |
| SEQ ID NO: 120 | TCKVTGTCPAD |
| SEQ ID NO: 121 | TCKATGTRPAD |
| SEQ ID NO: 122 | TCKVAGTCPPD |
| SEQ ID NO: 123 | TCKAAGTCPPD |
| SEQ ID NO: 124 | TCKASGTCPPD |
| SEQ ID NO: 125 | TCKAAGTCPAD |
| SEQ ID NO: 126 | TCKLSGTCPED |
| SEQ ID NO: 127 | TCKAAGTCPSD |
| SEQ ID NO: 128 | TCKLTGTCPPD |
| SEQ ID NO: 129 | QCQLGADCPPD |
| SEQ ID NO: 130 | KCQLSGNCLPD |
| SEQ ID NO: 131 | KCQLSGDCLPD |
| SEQ ID NO: 132 | HCALSGTCPDD |
| SEQ ID NO: 133 | HCAGSGTCPED |
| SEQ ID NO: 134 | TCLQGGDCIPD |
| SEQ ID NO: 135 | SCLQGGDCIPD |
| SEQ ID NO: 136 | TCKATGTCPPD |
| SEQ ID NO: 137 | TCKQAGTCPSD |
| SEQ ID NO: 138 | TCKIAGNCPAD |
| SEQ ID NO: 139 | ACKISNTCPPD |
| | |
| **SEQ ID NO: 140** | CKQSGTCPDD |
| SEQ ID NO: 141 | CKASNTCPPD |
| SEQ ID NO: 142 | CKAAGTCPPD |
| SEQ ID NO: 143 | CKASGTCPPD |
| SEQ ID NO: 144 | CKQSGTCPPD |
| SEQ ID NO: 145 | CKVTGTCPAD |
| SEQ ID NO: 146 | CKATGTRPAD |
| SEQ ID NO: 147 | CKVAGTCPPD |
| SEQ ID NO: 148 | CKAAGTCPAD |
| SEQ ID NO: 149 | CKLSGTCPED |
| SEQ ID NO: 150 | CKAAGTCPSD |
| SEQ ID NO: 151 | CKLTGTCPPD |
| SEQ ID NO: 152 | CQLGADCPPD |
| SEQ ID NO: 153 | CQLSGNCLPD |
| SEQ ID NO: 154 | CQLSGDCLPD |
| SEQ ID NO: 155 | CALSGTCPDD |
| SEQ ID NO: 156 | CAGSGTCPED |
| SEQ ID NO: 157 | CLQGGDCIPD |
| SEQ ID NO: 158 | CKATGTCPPD |
| SEQ ID NO: 159 | CKQAGTCPSD |
| SEQ ID NO: 160 | CKIAGNCPAD |
| SEQ ID NO: 161 | CKISNTCPPD |
| | |
| **SEQ ID NO: 162** | GCKQAGTCPP |
| SEQ ID NO: 163 | GCKASNTCPP |
| SEQ ID NO: 164 | GCKAAGTCPP |
| SEQ ID NO: 165 | SCKAAGTCPP |
| SEQ ID NO: 166 | TCKLAGTCPP |
| SEQ ID NO: 167 | GCKASGTCPP |
| SEQ ID NO: 168 | TCKQSGTCPP |
| SEQ ID NO: 169 | TCKVTGTCPA |
| SEQ ID NO: 170 | TCKATGTRPA |
| SEQ ID NO: 171 | TCKVAGTCPP |
| SEQ ID NO: 172 | TCKAAGTCPP |
| SEQ ID NO: 173 | TCKASGTCPP |
| SEQ ID NO: 174 | TCKAAGTCPA |
| SEQ ID NO: 175 | TCKLSGTCPE |
| SEQ ID NO: 176 | TCKAAGTCPS |
| SEQ ID NO: 177 | TCKLTGTCPP |
| SEQ ID NO: 178 | QCQLGADCPP |
| SEQ ID NO: 179 | KCQLSGNCLP |
| SEQ ID NO: 180 | KCQLSGDCLP |
| SEQ ID NO: 181 | HCALSGTCPD |
| SEQ ID NO: 182 | HCAGSGTCPE |
| SEQ ID NO: 183 | TCLQGGDCIP |
| SEQ ID NO: 184 | SCLQGGDCIP |
| SEQ ID NO: 185 | TCKATGTCPP |
| SEQ ID NO: 186 | TCKQAGTCPS |
| SEQ ID NO: 187 | TCKIAGNCPA |
| SEQ ID NO: 188 | ACKISNTCPP |
| | |
| **SEQ ID NO: 189** | CKLAGTCPP |
| SEQ ID NO: 190 | CKASNTCPP |
| SEQ ID NO: 191 | CKAAGTCPP |
| SEQ ID NO: 192 | CKASGTCPP |
| SEQ ID NO: 193 | CKQSGTCPP |
| SEQ ID NO: 194 | CKVTGTCPA |
| SEQ ID NO: 195 | CKATGTRPA |
| SEQ ID NO: 196 | CKVAGTCPP |
| SEQ ID NO: 197 | CKAAGTCPA |
| SEQ ID NO: 198 | CKLSGTCPE |
| SEQ ID NO: 199 | CKAAGTCPS |
| SEQ ID NO: 200 | CKLTGTCPP |
| SEQ ID NO: 201 | CQLGADCPP |
| SEQ ID NO: 202 | CQLSGNCLP |
| SEQ ID NO: 203 | CQLSGDCLP |
| SEQ ID NO: 204 | CALSGTCPD |
| SEQ ID NO: 205 | CAGSGTCPE |
| SEQ ID NO: 206 | CLQGGDCIP |
| SEQ ID NO: 207 | CKATGTCPP |
| SEQ ID NO: 208 | CKQAGTCPS |
| SEQ ID NO: 209 | CKIAGNCPA |
| SEQ ID NO: 210 | CKISNTCPP |
| | |
| **SEQ ID NO: 211** | DVINKVEQTT |
| SEQ ID NO: 212 | DVINKVEQST |
| SEQ ID NO: 213 | DVINKVEQKT |
| SEQ ID NO: 214 | DVLNKVEQTT |
| SEQ ID NO: 215 | DVVNKVEQTT |
| SEQ ID NO: 216 | DVINKVESTT |
| SEQ ID NO: 217 | DVINKVEQNT |
| SEQ ID NO: 218 | DVVPKVEGDT |
| SEQ ID NO: 219 | DVIPKVEGDT |
| SEQ ID NO: 220 | DIIPRVEQNT |
| SEQ ID NO: 221 | DIIPRLEQNT |
| SEQ ID NO: 222 | DIIPRVEQDT |
| SEQ ID NO: 223 | DVIPKVEGTT |
| SEQ ID NO: 224 | DIIPKVEQKT |
| SEQ ID NO: 225 | DVIPKVEGST |
| SEQ ID NO: 226 | DIIPKVEHNT |
| SEQ ID NO: 227 | DVIPKVEQNT |
| SEQ ID NO: 228 | DVIPKVEHNT |
| SEQ ID NO: 229 | DVINKVEHTT |
| SEQ ID NO: 230 | DVRNKVEGTT |
| SEQ ID NO: 231 | DVKNKVEADT |
| SEQ ID NO: 232 | DVKNKVEANT |
| SEQ ID NO: 233 | DVKNKVENNT |
| SEQ ID NO: 234 | DVKNKVEQTT |
| SEQ ID NO: 235 | DVKNKFENST |
| SEQ ID NO: 236 | DVQNKFEGNT |
| SEQ ID NO: 237 | DIQNKIEQTT |
| SEQ ID NO: 238 | DVIKRYEQTT |
| | |
| **SEQ ID NO: 239** | VINKVEQTT |
| SEQ ID NO: 240 | VINKVEQST |
| SEQ ID NO: 241 | VINKVEQKT |
| SEQ ID NO: 242 | VLNKVEQTT |
| SEQ ID NO: 243 | VVNKVEQTT |
| SEQ ID NO: 244 | VINKVESTT |
| SEQ ID NO: 245 | VINKVEQNT |
| SEQ ID NO: 246 | VVPKVEGDT |
| SEQ ID NO: 247 | VIPKVEGDT |
| SEQ ID NO: 248 | IIPRVEQNT |
| SEQ ID NO: 249 | IIPRLEQNT |
| SEQ ID NO: 250 | IIPRVEQDT |
| SEQ ID NO: 251 | VIPKVEGTT |
| SEQ ID NO: 252 | IIPKVEQKT |
| SEQ ID NO: 253 | VIPKVEGST |
| SEQ ID NO: 254 | IIPKVEHNT |
| SEQ ID NO: 255 | VIPKVEQNT |
| SEQ ID NO: 256 | VIPKVEHNT |
| SEQ ID NO: 257 | VINKVEHTT |
| SEQ ID NO: 258 | VRNKVEGTT |
| SEQ ID NO: 259 | VKNKVEADT |
| SEQ ID NO: 260 | VKNKVEANT |
| SEQ ID NO: 261 | VKNKVENNT |
| SEQ ID NO: 262 | VKNKVEQTT |
| SEQ ID NO: 263 | VKNKFENST |
| SEQ ID NO: 264 | VQNKFEGNT |
| SEQ ID NO: 265 | IQNKIEQTT |
| SEQ ID NO: 266 | VIKRYEQTT |
| | |
| **SEQ ID NO: 267** | INKVEQTT |
| SEQ ID NO: 268 | INKVEQST |
| SEQ ID NO: 269 | INKVEQKT |
| SEQ ID NO: 270 | LNKVEQTT |
| SEQ ID NO: 271 | VNKVEQTT |
| SEQ ID NO: 272 | INKVESTT |
| SEQ ID NO: 273 | INKVEQNT |
| SEQ ID NO: 274 | VPKVEGDT |
| SEQ ID NO: 275 | IPKVEGDT |
| SEQ ID NO: 276 | IPRVEQNT |
| SEQ ID NO: 277 | IPRLEQNT |
| SEQ ID NO: 278 | IPRVEQDT |
| SEQ ID NO: 279 | IPKVEGTT |
| SEQ ID NO: 280 | IPKVEHKT |
| SEQ ID NO: 281 | IPKVEGST |
| SEQ ID NO: 282 | IPKVEHNT |
| SEQ ID NO: 283 | IPKVEQNT |
| SEQ ID NO: 284 | INKVEHTT |
| SEQ ID NO: 285 | RNKVEGTT |
| SEQ ID NO: 286 | KNKVEADT |
| SEQ ID NO: 287 | KNKVEANT |
| SEQ ID NO: 288 | KNKVENNT |
| SEQ ID NO: 289 | KNKVEQTT |
| SEQ ID NO: 290 | KNKFENST |
| SEQ ID NO: 291 | QNKFEGNT |
| SEQ ID NO: 292 | QNKIEQTT |
| SEQ ID NO: 293 | IKRYEQTT |
| | |
| **SEQ ID NO: 294** | TGYIPLQTR |
| SEQ ID NO: 295 | TGYVPLGST |
| SEQ ID NO: 296 | TGYVPLGNT |
| SEQ ID NO: 297 | TGYVPLSTG |
| SEQ ID NO: 298 | TGYIPLQST |
| SEQ ID NO: 299 | TGYVPVGST |
| SEQ ID NO: 300 | TGYVPLQTS |
| SEQ ID NO: 301 | TGYVPLTTG |
| | |
| **SEQ ID NO: 302** | RGCKQXGTCPPDVINKVEQ |
| SEQ ID NO: 303 | RGCKAXNTCPPDVINKVEQ |
| SEQ ID NO: 304 | RGCKAXGTCPPDVINKVEQ |
| SEQ ID NO: 305 | QSCKAXGTCPPDVLNKVEQ |
| SEQ ID NO: 306 | QSCKAXGTCPPDVVNKVEQ |
| SEQ ID NO: 307 | QTCKQXGTCPPDVINKVEQ |
| SEQ ID NO: 308 | QTCKQXGTCPPDVVNKVEQ |
| SEQ ID NO: 309 | RTCKQXGTCPPDVINKVES |
| SEQ ID NO: 310 | RTCKQXGTCPPDVINKVEQ |
| SEQ ID NO: 311 | KGCKAXGTCPPDVINKVEQ |
| SEQ ID NO: 312 | RTCKQXGTCPPDVVPKVEG |
| SEQ ID NO: 313 | RTCKQXGTCPPDVIPKVEG |
| SEQ ID NO: 314 | RTCKVXGTCPADVVPKVEG |
| SEQ ID NO: 315 | RTCKAXGTRPADVIPKVEG |
| SEQ ID NO: 316 | STCKAXGTCPPDVIPKLEG |
| SEQ ID NO: 317 | RTCKQXGTCPPDIIPRLEQ |
| SEQ ID NO: 318 | RTCKQXGTCPPDIIPRVEQ |
| SEQ ID NO: 319 | KTCKVXGTCPPDVIPKVEG |
| SEQ ID NO: 320 | KTCKAXGTCPPDVIPKVEG |
| SEQ ID NO: 321 | STCKAXGTCPPDVIPKVEG |
| SEQ ID NO: 322 | RTCKAXGTCPPDVIPKVEG |
| SEQ ID NO: 323 | STCKAXGTCPADVIPKVEG |
| SEQ ID NO: 324 | KTCKLXGTCPEDVINKVEQ |
| SEQ ID NO: 325 | KTCKQXGTCPPDIIPKIEG |
| SEQ ID NO: 326 | KTCKQXGTCPPDIVPKVEG |
| SEQ ID NO: 327 | STCKQXGTCPPDIIPRVEQ |
| SEQ ID NO: 328 | KTCKQXGTCPPDVIPKVEG |
| SEQ ID NO: 329 | QTCKAXGTCPSDIIPKVEH |
| SEQ ID NO: 330 | QTCKAXGTCPPDVIPKVEQ |
| SEQ ID NO: 331 | QTCKLXGTCPPDVIPKVEH |
| SEQ ID NO: 332 | QTCKAXGTCPSDVINKVEH |
| SEQ ID NO: 333 | KQCQLXADCPPDVRNKVEG |
| SEQ ID NO: 334 | AKCQLXGNCLPDVKNKVEA |
| SEQ ID NO: 335 | AKCQLXGDCLPDVKNKVEA |
| SEQ ID NO: 336 | RHCALXGTCPDDVKNKVEN |
| SEQ ID NO: 337 | KHCAGXGTCPEDVKNKVEQ |
| SEQ ID NO: 338 | KTCLQXGDCIPDVKNKFEN |
| SEQ ID NO: 339 | RSCLQXGDCIPDVQNKFEG |
| SEQ ID NO: 340 | QTCKAXGTCPPDVIPKVEG |
| SEQ ID NO: 341 | KTCKQXGTCPPDVVPKVEG |
| SEQ ID NO: 342 | RTCKQXGTCPPDVINKVEG |
| SEQ ID NO: 343 | KTCKQXGTCPSDVINKVEG |
| SEQ ID NO: 344 | KTCKLXGTCPEDVVNKIEQ |
| SEQ ID NO: 345 | RTCKQXGTCPPDVVDKVEG |
| SEQ ID NO: 346 | STCKAXGTCPPDVVNKVEG |
| SEQ ID NO: 347 | PTCKIXGNCPADIQNKFEN |
| SEQ ID NO: 348 | PACKIXNTCPPDIINKYEN |
| | |
| ***X = Gly (G) or Ala (A) | |
| | |
| **SEQ ID NO: 349** | RGCKQXGTCPPD |
| SEQ ID NO: 350 | RGCKAXNTCPPD |
| SEQ ID NO: 351 | RGCKAXGTCPPD |
| SEQ ID NO: 352 | QSCKAXGTCPPD |
| SEQ ID NO: 353 | QTCKQXGTCPPD |
| SEQ ID NO: 354 | RTCKQXGTCPPD |
| SEQ ID NO: 355 | KGCKAXGTCPPD |
| SEQ ID NO: 356 | PTCKAXGTCPPD |
| SEQ ID NO: 357 | RTCKVXGTCPAD |
| SEQ ID NO: 358 | RTCKAXGTRPAD |
| SEQ ID NO: 359 | KTCKVXGTCPPD |
| SEQ ID NO: 360 | KTCKAXGTCPPD |
| SEQ ID NO: 361 | RTCKAXGTCPPD |
| SEQ ID NO: 362 | STCKAXGTRPPD |
| SEQ ID NO: 363 | STCKAXGTCPAD |
| SEQ ID NO: 364 | KTCKLXGTCPED |
| SEQ ID NO: 365 | ATCKQXGTCPPD |
| SEQ ID NO: 366 | STCKQXGTCPPD |
| SEQ ID NO: 367 | QTCKAXGTCPSD |
| SEQ ID NO: 368 | QTCKLXGTCPPD |
| SEQ ID NO: 369 | KQCQLXADCPPD |
| SEQ ID NO: 370 | AKCQLXGNCLPD |
| SEQ ID NO: 371 | AKCQLXGDCLPD |
| SEQ ID NO: 372 | RHCALXGTCPDD |
| SEQ ID NO: 373 | KHCAGXGTCPED |
| SEQ ID NO: 374 | KTCLQXGDCIPD |
| SEQ ID NO: 375 | RSCLQXGDCIPD |
| SEQ ID NO: 376 | QTCKAXGTCPPD |
| SEQ ID NO: 377 | KTCKQXGTCPED |
| SEQ ID NO: 378 | KTCKQXGTCPSD |
| SEQ ID NO: 379 | STCKAXGTCPPD |
| SEQ ID NO: 380 | PTCKIXGNCPAD |
| SEQ ID NO: 381 | PACKIXNTCPPD |
| | |
| ***X = Gly (G) or Ala (A) | |
| | |
| **SEQ ID NO: 382** | GCKQXGTCPPD |
| SEQ ID NO: 383 | GCKAXNTCPPD |
| SEQ ID NO: 384 | ACKAXGTCPPD |
| SEQ ID NO: 385 | SCKAXGTCPPD |
| SEQ ID NO: 386 | KCKAXGTCIPD |
| SEQ ID NO: 387 | GCKAXGTCPPD |
| SEQ ID NO: 388 | KCKAXGTCPPD |
| SEQ ID NO: 389 | TCKVXGTCPAD |
| SEQ ID NO: 390 | TCKAXGTRPAD |
| SEQ ID NO: 391 | TCKVXGTCPPD |
| SEQ ID NO: 392 | SCKLXGTCPPD |
| SEQ ID NO: 393 | SCKQXGTCPSD |
| SEQ ID NO: 394 | TCKAXGTCPAD |
| SEQ ID NO: 395 | TCKLXGTCPED |
| SEQ ID NO: 396 | TCKAXGTCPSD |
| SEQ ID NO: 397 | TCKLXGTCPPD |
| SEQ ID NO: 398 | QCQLXADCPPD |
| SEQ ID NO: 399 | KCQLXGNCLPD |
| SEQ ID NO: 400 | KCQLXGDCLPD |
| SEQ ID NO: 401 | HCALXGTCPDD |
| SEQ ID NO: 402 | HCAGXGTCPED |
| SEQ ID NO: 403 | TCLQXGDCIPD |
| SEQ ID NO: 404 | SCLQXGDCIPD |
| SEQ ID NO: 405 | TCKAXGTCPPD |
| SEQ ID NO: 406 | TCKQXGTCPSD |
| SEQ ID NO: 407 | TCKIXGNCPAD |
| SEQ ID NO: 408 | ACKIXNTCPPD |
| | |
| ***X = Gly (G) or Ala (A) | |
| | |
| **SEQ ID NO: 409** | CKQXGTCPDD |
| SEQ ID NO: 410 | CKAXNTCPPD |
| SEQ ID NO: 411 | CLAXGTCPAD |
| SEQ ID NO: 412 | CLAXGTCPPD |
| SEQ ID NO: 413 | CKLXGTCPAD |
| SEQ ID NO: 414 | CKVXGTCPAD |
| SEQ ID NO: 415 | CKAXGTRPAD |
| SEQ ID NO: 416 | CKVXGTCPPD |
| SEQ ID NO: 417 | CKAXGTCPAD |
| SEQ ID NO: 418 | CKLXGTCPED |
| SEQ ID NO: 419 | CKAXGTCPSD |
| SEQ ID NO: 420 | CKLXGTCPPD |
| SEQ ID NO: 421 | CQLXADCPPD |
| SEQ ID NO: 422 | CQLXGNCLPD |
| SEQ ID NO: 423 | CQLXGDCLPD |
| SEQ ID NO: 424 | CALXGTCPDD |
| SEQ ID NO: 425 | CAGXGTCPED |
| SEQ ID NO: 426 | CLQXGDCIPD |
| SEQ ID NO: 427 | CKAXGTCPPD |
| SEQ ID NO: 428 | CKQXGTCPSD |
| SEQ ID NO: 429 | CKIXGNCPAD |
| SEQ ID NO: 430 | CKIXNTCPPD |
| | |
| ***X = Gly (G) or Ala (A) | |
| | |
| SEQ ID NO: 486 | KTCKQSGTCPSDVVNKVEG |
| SEQ ID NO: 487 | QTCKAAGTCPSDVIPKIEH |
| SEQ ID NO: 488 | KTCKQSGTCPPDVIDKVEG |
| SEQ ID NO: 489 | STCKAAGTCPPDVIPKVKG |
| SEQ ID NO: 490 | KTCKQSGTCPSD |
| | |
| SEQ ID NO: 491 ((SEQ ID NO:2)x3+ (SEQ ID NO: 487)x3 +(SEQ ID NO:77)x3 with a tripeptide (GGP) linker): | |
| | |
| | |
| SEQ ID NO: 492 ((SEQ ID NO:2)+ (SEQ ID NO:487) +(SEQ ID NO:77))x3 with a tripeptide (GGP) linker | |
| | |

**Table 2: List of thioredoxin variants**

| |
|---|
| SEQ ID NO: 493 (variant thiorexodin polypeptide from hyperthermophile archaebacterium *Pyrococcus furiosus)* |
| |
| SEQ ID NO: 494 (variant thiorexodin polypeptide from hyperthermophile archaebacterium *Thermococcus kodakarensis)* |
| |
| |
| SEQ ID NO: 495 (variant thiorexodin polypeptide from hyperthermophile archaebacterium *Thermococcus onnurineus)* |
| |
| |
| SEQ ID NO: 496 (variant of thiorexodin polypeptide from hyperthermophile archaebacterium *Thermococcus sibiricus)* |
| |
| |
| SEQ ID NO: 497 (Dimer of Escherichia coli thioredoxin variants) |
| |
| |
| SEQ ID NO: 498 (Trimer of Escherichia coli thioredoxin variants) |
| |

## Claims

1. An immunogenic polypeptide comprising
a) a multimer (i) of an L2 polypeptide comprising an amino acid sequence as shown in SEQ ID NO:1 or a variant thereof, or (ii) of a fragment or fragments of said L2 polypeptide or variants thereof,
wherein the amino acid sequence of said variants is at least 65% identical with said L2 polypeptide or fragment of said L2 polypeptide, and
wherein said fragments comprise at least 7 consecutive amino acid residues of said L2 polypeptide;
and
b) a scaffold polypeptide,
wherein said scaffold polypeptide constrains the structure of said L2 polypeptides or variants thereof, or of said fragments of said L2 polypeptide or variants thereof, and
wherein said scaffold polypeptide is selected from the group consisting of thioredoxin polypeptides and thioredoxin polypeptides derived from thermophile bacteria.

2. The immunogenic polypeptide of claim 1, wherein the scaffold polypeptide is a thioredoxin polypeptide selected from the group consisting of
a) a polypeptide having a sequence as shown in SEQ ID No: 24, SEQ ID No: 25, SEQ ID No: 26, SEQ ID No: 27, or SEQ ID No: 28, and
b) a variant polypeptide having a sequence at least 70% identical to the sequence shown in SEQ ID No: 24, SEQ ID No: 25, SEQ ID No: 26, SEQ ID No: 27, or SEQ ID No: 28,
wherein said polypeptide constrains the structure of the HPV L2 polypeptide, or which constrains the structure of a fragment of said HPV L2 polypeptide.

3. The immunogenic polypeptide according to claim 1 or 2, wherein the fragment or variant of the HPV L2 polypeptide is selected from the group consisting of SEQ ID NO: 2 to 15, 20 to 22, and 31 to 479.

4. The immunogenic polypeptide according to anyone of claims 1 to 3, wherein the fragment of the HPV L2 polypeptide is selected from the group consisting of SEQ ID NO: 2, 3, 4, 5, 6, 7, 8, 9, 10, 11, 12, 13, 14, 15, 31 and 32.

5. The immunogenic polypeptide according to any one of claims 1 to 5, wherein the fragment of the HPV L2 polypeptide has a sequence as shown in SEQ ID NO:2.

6. The immunogenic polypeptide according to any one of claims 1 to 5, comprising a multimer of 2 to 15 fragments of said L2 polypeptide or variants thereof.

7. The immunogenic polypeptide according to any one of claims 1 to 6, wherein the immunogenic polypeptide has a sequence as shown in SEQ ID NO: 29 or 30.

8. A polynucleotide encoding the immunogenic polypeptide according to anyone of claims 1 to 7.

9. A vaccine comprising the immunogenic polypeptide according to any one of claims 1 to 7.

10. Use of the immunogenic polypeptide according to any one of claims 1 to 7 for the preparation of a vaccine for immunization of a subject against infection with HPV.

11. A method for producing an antibody against the immunogenic polypeptide of any one of claims 1 to 7 comprising the steps:
a) providing said immunogenic polypeptide,
b) immunizing a non-human host with said immunogenic polypeptide, and
c) harvesting the antibody against said immunogenic
polypeptide.

12. The method of claim 11, wherein the antibody is against HPV genotypes 52, 2, 27, 57 and/or 11.

13. The method of claim 10 or 11, wherein the antibody is polyclonal or monoclonal.

14. An immunogenic polypeptide comprising
a) a scaffold polypeptide, and
b) an L2 polypeptide comprising an amino acid sequence as shown in SEQ ID NO: 1, or a fragment of said L2 polypeptide,
wherein said scaffold polypeptide constrains the structure of said L2 polypeptide, or of said fragment of said L2 polypeptide,
wherein said scaffold polypeptide is a thioredoxin polypeptide derived from thermophile bacteria, and
wherein said fragment comprises at least 7 consecutive amino acid residues of said L2 polypeptide.

## Patentansprüche

1. Immunogenes Polypeptid, umfassend
a) ein Multimer (i) eines L2-Polypeptids, das eine Aminosäuresequenz gemäß SEQ ID NO:1 umfasst, oder einer Variante davon oder (ii) eines Fragments bzw. von Fragmenten des L2-Polypeptids oder von Varianten davon,
wobei die Aminosäuresequenz der Varianten zu wenigstens 65% mit dem L2-Polypeptid bzw. Fragment des L2-Polypeptids identisch ist und wobei die Fragmente wenigstens 7 aufeinander folgende Aminosäurereste des L2-Polypeptids umfassen;
und
b) ein Gerüstpolypeptid,
wobei das Gerüstpolypeptid die Struktur der L2-Polypeptide oder Varianten davon bzw. der Fragmente des L2-Polypeptids oder Varianten davon Beschränkungen unterwirft und
wobei das Gerüstpolypeptid aus der aus Thioredoxin-Polypeptiden und aus thermophilen Bakterien stammenden Thioredoxin-Polypeptiden bestehenden Gruppe ausgewählt ist.

2. Immunogenes Polypeptid nach Anspruch 1, wobei es sich bei dem Gerüstpolypeptid um ein Thioredoxin-Polypeptid handelt, das aus der aus
a) einem Polypeptid mit einer Sequenz gemäß SEQ ID Nr. 24, SEQ ID Nr. 25, SEQ ID Nr. 26, SEQ ID Nr. 27 oder SEQ ID Nr. 28 und
b) einer Polypeptidvariante mit einer Sequenz, die zu wenigstens 70% mit der Sequenz gemäß SEQ ID Nr. 24, SEQ ID Nr. 25, SEQ ID Nr. 26, SEQ ID Nr. 27 bzw. SEQ ID Nr. 28 identisch ist,
bestehenden Gruppe ausgewählt ist, wobei das Polypeptid die Struktur des HPV-L2-Polypeptids Beschränkungen unterwirft oder die Struktur eines Fragments des HPV-L2-Polypeptids Beschränkungen unterwirft.

3. Immunogenes Polypeptid gemäß Anspruch 1 oder 2, wobei das Fragment bzw. die Variante des HPV-L2-Polypeptids aus der aus SEQ ID NO: 2 bis 15, 20 bis 22 und 31 bis 479 bestehenden Gruppe ausgewählt ist.

4. Immunogenes Polypeptid gemäß einem der Ansprüche 1 bis 3, wobei das Fragment des HPV-L2-Polypeptids aus der aus SEQ ID NO: 2, 3, 4, 5, 6, 7, 8, 9, 10, 11, 12, 13, 14, 15, 31 und 32 bestehenden Gruppe ausgewählt ist.

5. Immunogenes Polypeptid gemäß einem der Ansprüche 1 bis 5, wobei das Fragment des HPV-L2-Polypeptids eine Sequenz gemäß SEQ ID NO: 2 aufweist.

6. Immunogenes Polypeptid gemäß einem der Ansprüche 1 bis 5, umfassend ein Multimer von 2 bis 15 Fragmenten des L2-Polypeptids oder Varianten davon.

7. Immunogenes Polypeptid gemäß einem der Ansprüche 1 bis 6, wobei das immunogene Polypeptid eine Sequenz gemäß SEQ ID NO: 29 oder 30 aufweist.

8. Polynukleotid, codierend das immunogene Polypeptid gemäß einem der Ansprüche 1 bis 7.

9. Impfstoff, umfassend das immunogene Polypeptid gemäß einem der Ansprüche 1 bis 7.

10. Verwendung des immunogenen Polypeptids gemäß einem der Ansprüche 1 bis 7 zur Herstellung eines Impfstoffs für die Immunisierung eines Individuums gegen eine Infektion mit HPV.

11. Verfahren zur Erzeugung eines Antikörpers gegen das immunogene Polypeptid nach einem der Ansprüche 1 bis 7, umfassend die Schritte:
a) Bereitstellen des immunogenen Polypeptids,
b) Immunisieren eines nichtmenschlichen Wirts mit dem immunogenen Polypeptid und
c) Ernten des Antikörpers gegen das immunogene Polypeptid.

12. Verfahren nach Anspruch 11, wobei es sich bei dem Antikörper um einen Antikörper gegen HPV-Genotypen 52, 2, 27, 57 und/oder 11 handelt.

13. Verfahren nach Anspruch 10 oder 11, wobei der Antikörper polyklonal oder monoklonal ist.

14. Immunogenes Polypeptid, umfassend
a) ein Gerüstpolypeptid und
b) ein L2-Polypeptid, das eine Aminosäuresequenz gemäß SEQ ID NO:1 umfasst, oder ein Fragment des L2-Polypeptids,
wobei das Gerüstpolypeptid die Struktur des L2-Polypeptids bzw. des Fragments des L2-Polypeptids Beschränkungen unterwirft,
wobei es sich bei dem Gerüstpolypeptid um ein aus thermophilen Bakterien stammendes Thioredoxin-Polypeptid handelt und
wobei das Fragment wenigstens 7 aufeinander folgende Aminosäurereste des L2-Polypeptids umfasst.

## Revendications

1. Polypeptide immunogène comprenant
a) un multimère (i) d'un polypeptide L2 comprenant une séquence d'acides aminés telle que présentée dans la SEQ ID n° : 1 ou un variant de celle-ci, ou (ii) d'un fragment ou de fragments dudit polypeptide L2 ou de variants de celui/ceux-ci,
dans lequel la séquence d'acides aminés desdits variants est identique, au moins à 65%, au dit polypeptide L2 ou à un fragment dudit polypeptide L2, et
dans lequel lesdits fragments comprennent au moins 7 résidus d'acides aminés consécutifs dudit polypeptide L2 ;
et
b) un polypeptide d'échafaudage,
dans lequel ledit polypeptide d'échafaudage contraint la structure desdits polypeptides L2 ou de variants de ceux-ci, ou desdits fragments dudit polypeptide L2 ou de variants de ceux-ci, et
dans lequel ledit polypeptide d'échafaudage est choisi dans le groupe constitué par des polypeptides de thiorédoxine et polypeptides de thiorédoxine dérivés d'une bactérie thermophile.

2. Polypeptide immunogène selon la revendication 1, dans lequel le polypeptide d'échafaudage est un polypeptide de thiorédoxine choisi dans le groupe constitué par
a) un polypeptide ayant une séquence telle que présentée dans la SEQ ID n° : 24, la SEQ ID n° : 25, la SEQ ID n° : 26, la SEQ ID n° : 27 ou la SEQ ID n° : 28, et
b) un polypeptide variant ayant une séquence étant identique, au moins à 70%, à la séquence présentée dans la SEQ ID n° : 24, la SEQ ID n° : 25, la SEQ ID n° : 26, la SEQ ID n° : 27 ou la SEQ ID n° : 28,
dans lequel ledit polypeptide contraint la structure du polypeptide L2 du HPV ou contraint la structure d'un fragment dudit polypeptide L2 du HPV.

3. Polypeptide immunogène selon la revendication 1 ou 2, dans lequel le fragment ou un variant du polypeptide L2 du HPV est choisi dans le groupe constitué par les SEQ ID n° : 2 à 15, 20 à 22 et 31 à 479.

4. Polypeptide immunogène selon l'une quelconque des revendications 1 à 3, dans lequel le fragment du polypeptide L2 du HPV est choisi dans le groupe constitué par les SEQ ID n° : 2, 3, 4, 5, 6, 7, 8, 9, 10, 11, 12, 13, 14, 15, 31 et 32.

5. Polypeptide immunogène selon l'une quelconque des revendications 1 à 5, dans lequel le fragment du polypeptide L2 du HPV a une séquence telle que présentée dans la SEQ ID n° : 2.

6. Polypeptide immunogène, selon l'une quelconque des revendications 1 à 5, comprenant un multimère de 2 à 15 fragments dudit polypeptide L2 ou de variants de celui-ci.

7. Polypeptide immunogène selon l'une quelconque des revendications 1 à 6, le polypeptide immunogène ayant une séquence telle que présentée dans la SEQ ID n° : 29 ou 30.

8. Polynucléotide codant pour le polypeptide immunogène selon l'une quelconque des revendications 1 à 7.

9. Vaccin comprenant le polypeptide immunogène selon l'une quelconque des revendications 1 à 7.

10. Utilisation du polypeptide immunogène, selon l'une quelconque des revendications 1 à 7, pour la préparation d'un vaccin destiné à l'immunisation d'un sujet contre une infection par HPV.

11. Procédé de production d'un anticorps contre le polypeptide immunogène, selon l'une quelconque des revendications 1 à 7, comprenant les étapes consistant à :
a) fournir ledit polypeptide immunogène,
b) immuniser un hôte non humain avec ledit polypeptide immunogène, et
c) recueillir l'anticorps contre ledit polypeptide immunogène.

12. Procédé selon la revendication 11, dans lequel l'anticorps est contre les génotypes 52, 2, 27, 57 et/ou 11 du HPV.

13. Procédé selon la revendication 10 ou 11, dans lequel l'anticorps est polyclonal ou monoclonal.

14. Polypeptide immunogène comprenant
a) un polypeptide d'échafaudage, et
b) un polypeptide L2 comprenant une séquence d'acides aminés telle que présentée dans la SEQ ID n° : 1, ou un fragment dudit polypeptide L2,
dans lequel ledit polypeptide d'échafaudage contraint la structure dudit polypeptide L2 ou dudit fragment dudit polypeptide L2,
dans lequel ledit polypeptide d'échafaudage est un polypeptide de thiorédoxine dérivé d'une bactérie thermophile, et
dans lequel ledit fragment comprend au moins 7 résidus d'acides aminés consécutifs dudit polypeptide L2.
